# EUROPEAN PATENT SPECIFICATION

(11) **EP 4 781 195 B1**
(45) Date of publication and mention of the grant of the patent: **07.10.2026**
(21) Application number: 24737514.0
(22) Date of filing: 17.06.2024
(51) Int. Cl.: G01N 33/50

(54) **METHODS FOR DETERMINING WHEN A NATURAL THERAPEUTIC OR BENEFICIAL PRODUCT EXERTS ITS THERAPEUTIC OR BENEFICIAL EFFECT THROUGH A PHYSIOLOGICAL MODE OF ACTION**
VERFAHREN ZUR BESTIMMUNG, WANN EIN NATÜRLICHES THERAPEUTISCHES ODER NÜTZLICHES PRODUKT SEINE THERAPEUTISCHE ODER VORTEILHAFTE WIRKUNG DURCH EINEN PHYSIOLOGISCHEN WIRKUNGSMODUS AUSZEICHNET
PROCÉDÉS POUR DÉTERMINER QUAND UN PRODUIT THÉRAPEUTIQUE OU BÉNÉFIQUE NATUREL EXERCE SON EFFET THÉRAPEUTIQUE OU BÉNÉFIQUE PAR L'INTERMÉDIAIRE D'UN MODE D'ACTION PHYSIOLOGIQUE

(30) Priority: 11.01.2024 WO PCT/IB2024/050280; 09.05.2024 WO PCT/IB2024/054526
(43) Date of publication of application: 29.07.2026
(73) Proprietor: Bios-Therapy, Physiological Systems for Health S.p.A., 52037 Sansepolcro (AR) (IT)
(72) Inventor: MERCATI, Valentino, 52037 Sansepolcro (AR) (IT); LUCCI, Jacopo, 52037 Sansepolcro (AR) (IT)
(74) Representative: Predazzi, Valentina
(86) International application number: PCT/IB2024/055892
(87) International publication number: WO 2025/149788

(56) References cited:
- US-A1- 2023 321 173
- GIBBONS S: "An overview of plant extracts as potential therapeutics", EXPERT OPINION ON THERAPEUTIC PATENTS, TAYLOR & FRANCIS, GB, vol. 13, no. 3, 1 April 2003 (2003-04-01), pages 489 - 197, XP009096653, ISSN: 1354-3776, DOI: 10.1517/13543776.13.4.489
- WANG JINGHUI ET AL: "Systematic investigation of the mechanism of herbal medicines for the treatment of prostate cancer", AGING, 10 January 2023 (2023-01-10), XP093236706, ISSN: 1945-4589, DOI: 10.18632/aging.204516
- EUROPEAN MEDICINES AGENCY: "Public statement on the use of herbal medicinal products 1 containing estragole Final", 12 May 2023 (2023-05-12), pages 1 - 22, XP093236704, Retrieved from the Internet <URL:https://www.ema.europa.eu/en/documents/other/public-statement-use-herbal-medicinal-products-containing-estragole-revision-1_en.pdf>
- EUROPEAN MEDICINES AGENCY: "Guideline on specifications: test procedures and acceptance criteria for herbal substances, herbal preparations and herbal medicinal products/traditional herbal medicinal products", 18 January 2022 (2022-01-18), pages 1 - 32, XP093236697, Retrieved from the Internet <URL:https://www.ema.europa.eu/en/documents/scientific-guideline/guideline-specifications-test-procedures-and-acceptance-criteria-herbal-substances-herbal-preparations-and-herbal-medicinal-productstraditional-herbal-medicinal-products-revision-3_en.pdf>

## Description

The present invention relates to new methods enabling the evolution of the medical arts passing from the use of artificial substances that are chemically or biologically defined to self-assembling natural entities, obtained from natural raw materials with industrial processes that preserve the endogenous properties thereof, thereby preserving their capability to interact with the networks of the living kingdom (including humans); wherein said natural entities cannot be defined with the classical quali-quantitative composition schemes. The invention, therefore, provides new methods for determining when a therapeutic or beneficial product exerts its therapeutic or beneficial effect through a physiological mode of action. This method provides a necessary tool for the skilled person to assess the mechanism of action of a therapeutic or beneficial product which, with the new developments in the regulatory framework for medical devices and food supplements has become a relevant feature to assess and for which no methods are available in the art.

### STATE OF THE ART

### The scientific evolution in medicine in the last centuries:

In the evolution that our species has undertaken over the last 5 centuries, dating back to the beginning of the so-called Anthropocene where with Paracelsus the alchemical practices which brought the artificiality of therapeutic products into medicine were introduced, we are now aware of the increasingly marked rupture between the reductionist (artificial) technological evolution and that of the different path of the biological entities of the living system that are hyperconnected to each other: both the organic ones including our species, as well as the inorganic ones, which have followed the directions of native intelligence and programmatic design.

In the scientific evolution of recent decades, new conceptual parameters are being applied which will have to find application areas aimed to limit the iatrogenic effects of pharmaceutical APIs both in humans and in the environment.

It is known that synthetic APIs can enter ecosystems through various routes, mainly through the discharge of pharmaceutical waste from manufacturing facilities and the improper disposal of unused or expired drugs. This can lead to the bioaccumulation of artificial and poorly biodegradable substances in aquatic and terrestrial organisms, with potential negative effects on food chains and threats to biodiversity.

Some studies have highlighted adverse effects on aquatic organisms, such as alterations in behaviour, reproduction and even mortality, following exposure to synthetic APIs (Boxall, A. B. et al (2012). Pharmaceuticals and personal care products in the environment: what are the big questions?. Environmental health perspectives, 120(9), 1221-1229); Fick, J., & Lindberg, R. H. (2015). Tysklind, M. and Larsson, D. G. J. (2015). Predicted critical environmental concentrations for 500 pharmaceuticals. Regulatory Toxicology and Pharmacology, 73(1), 607-616.)

It is well known that many synthetic APIs are designed to be biologically active and particularly stable, which, as a result, can hinder their natural degradation processes. Consequently, these molecules persist in the environment for extended periods, potentially accumulating in soils and waters. This reduced biodegradability raises concerns about long-term environmental impacts and the potential for bioaccumulation in organisms [Kasprzyk-Hordern, B., et al (2008). The removal of pharmaceuticals, personal care products, endocrine disruptors and illicit drugs during wastewater treatment and its impact on the quality of receiving waters. Water research, 43(2), 363-380; Verlicchi P., et al (2012). Occurrence of pharmaceutical compounds in urban wastewater: Removal, mass load and environmental risk after a secondary treatment-A review. Science of the total environment, 429, 123-155].

Furthermore, there is growing concern about the potential effects of synthetic APIs on human and animal immune systems. Some pharmaceuticals products have been found to interfere with immune function, either directly or indirectly, leading to altered immune responses or increased susceptibility to infections. This can have significant implications for both individual health and population-level immunity [Vos T. et al (2016). Global, regional, and national incidence, prevalence, and years lived with disability for 310 diseases and injuries, 1990-2015: a systematic analysis for the Global Burden of Disease Study 2015. The Lancet, 388(10053), 1545-1602; Calabrese, E. J., & Baldwin, L. A. (2003). Toxicology rethinks its central belief. Nature, 421(6924), 691-692].

In conclusion, while synthetic APIs have undoubtedly contributed to advancements in healthcare, it is now becoming clear that their environmental and health impacts should be carefully considered. Efforts to develop greener pharmaceuticals, improve waste management, and monitor environmental contamination are crucial steps towards mitigating these concerns.

Furthermore, synthetic molecules (intended as molecules obtained through a production carried out by man through chemical synthesis laboratory/industrial processes) are designed to provide the desired interaction with a specific given target molecule, said design not taking into account all the interactions that the said molecule has within a natural matrix, and will have with the environment and with the whole receiving network of the organism in which they will be used.

### Natural matrices and matrix effect

Natural matrices, such as plant matrices, are complex systems characterized by many molecular components belonging to different phytochemical classes that interact with each other already in the plant to determine the plant's biology. This interaction continues also in the processing phases and different processing techniques affect the post-processing interactions of said components. These compounds can interact at the functional and structural level. Supramolecular aggregates as well as their chemical-physical and structural characteristics that result in both structural and functional networks are dynamic interactions and can be modulated by environmental conditions and, as one can expect, these interactions affect the reactivity of the individual components and, through the so called "matrix effect", result in properties typical of the distinct entity represented by the matrix and are different from the sum of the properties of its single molecular components. Such properties are defined as "emerging properties". This phenomenon has been described and attributed specifically to living matter, which has a drive to self-assemble and self-organize to form supramolecular complex entities [Jean-Marie Lehn Toward complex matter: Supramolecular chemistry and self-organization. PNAS, 2002, 99 (8) 4763-4768 https://doi.org/10.1073/pnas.072065599]. This inherent complexity leads to the fact that individual molecules within a natural matrix cannot be considered as contained in isolated and fixed packages, as mutual non-covalent and dynamic interactions continuously occur between them. Such interactions are intra- and intermolecular and occur both among molecules of the same type as well as among molecules belonging to different chemical classes.

This introduces the need to consider that the ability of a natural matrix to exert a therapeutic action on the human body depends not only on the quali-quantitative composition of the matrix, which is by its own nature prone to be variable per se, but also on the presence of such interactions between same and different molecules, including small molecules as well as more complex ones such as proteins, polysaccharides, lipids, RNA, etc.

It is known that knowledge of the identity and amount of each and every molecule in a natural matrix is not sufficient to predict the dynamic and kinetic properties, as well as the therapeutic effectiveness, of the matrix itself. The opposite happens when selected single molecules, such as APIs, are considered, whereby the structure-activity relationship (SAR), thereby the pharmacodynamic and pharmacokinetic properties are intrinsically related to the chemical identity of the active principle, and to the pharmacodynamic inertia of the excipients. The networks established among all components of the matrix yielding "the matrix effect" makes it impossible to identify a single marker as representative of the network, or to define the activity exerted by a natural matrix-based therapeutic product on the basis of singled out APIs, because no single component is capable of conveying alone all of the properties specific to the matrix, since no single component reflects the interaction between the matrix and the target living organism.

The matrix effect confers the specific and unique properties of the matrix itself or of a mixture of matrices that result in a new different matrix, called emergent properties, which cannot be reconducted to the properties of any of the components taken in isolation. This perfectly reflects the impossibility to correctly study such properties through deterministic chemical methods, commonly used in classical pharmacological chemistry, which, as said above, are well adequate only for single active principles and excipients in pharma settings. The appropriate tools for correctly study such properties are to be found following the approaches within the systems theory and the concepts of the "networks over a network" interaction described herein.

Rather, the dynamic and kinetic behaviour of the matrix is the result of the dynamic networks of interactions taking place within the matrix, showing:
- the presence of a great number of components,
- the inability to reconduct the properties of the matrix to the sum of the properties of the single substances
- the impossibility to describe the interaction between the matrix and the receiving organism according to the key-lock paradigm (model), which is the foundation of SAR.

### Interconnections between living organisms and epigenetic environmental endogenous and exogenous influxes

Native biosynthesised molecules, being produced in natural, non-artificial settings, will intrinsically carry all the essential features to exist and exert their functions in an epigenetically determined context whose description is inaccessible when a conventional deterministic chemical approach is used.

Products obtained from natural sources have been used for thousands of years to prevent and cure human diseases. In this context, many studies have been limited to characterizing their chemical composition at the monomolecular level and the monomolecular activities, while the spontaneous assemblies, interactions, and supramolecular organisation of all the components in said natural products have not been fully investigated and thus understood. Since the development of modern chemistry, the reductionist approach focusing on the isolation of single molecules from natural products and the subsequent artificial synthesis of molecules of therapeutic interest, the aim has been to develop selected active principles that act on a given target following the key-lock paradigm.

This had led to the conviction that research in the field of life sciences was to be aimed at substances that can be chemically validated, with data such as quantities of the individual substances at a molecular level, generating very powerful and effective artificial products, with linear dynamics. It is now becoming evident that this direction is also generating harmful impacts on biodiversity and native immune systems.

As discussed above, the results of the scientific evolution in the last decades are bringing to the awareness that the iatrogenic effects on man and environment, of synthetic APIs must be somehow limited. Therefrom the general trend toward sciences that are more harmonised with life itself aiming at the defense and preservation of the native and inherent balances of intraspecies and interspecies interconnection in the animal, vegetal and mineral kingdoms. Indeed, in the therapeutic field, man has intervened developing synthetic pharmaceutical products, for some centuries, due to a non-awareness of the effects of said products on all species and on the environment that resulted, in time, in contrast with nature, and due to the lack of technology allowing standardisation of sources of naturally assembled matter compliant with therapeutic purposes requirements. The result of this, summarised also in the term "Anthropocene" which defines the present era, comprises the modification of the individual processes and functions of the metabolic framework of every living species. In the present era, the approaching of breaking point of the coexistence between the earth endogenous system that has been in place for billions of years and all the processes, methods and artificial substances produced by man which are not compatible/assimilable with life in the long term, is evident.

In the last decades, but in particular after the Covid19 pandemic, the leading social feeling is of anxiety, also due to the increasing number of orphan, oncological and chronic degenerative diseases, and to the advent of dramatic issues such as antibiotic resistance and the increasingly marked need for assisted births.

Among the fields that cause the most alarm due to the irreversibility of the related pollution caused, is the pharmaceutical field, due to the powerful effects and the non-degradability of each synthetic molecule internalised and eventually released in the environment by the animals or plant organisms treated with synthetic APIs.

It is hence necessary to take note that the main threats to our species such as climate change, the rapid decrease in biodiversity and the other negative connotations that define our era as the "Anthropocene" are strongly linked to the billions of tons of artificial non-biodegradable substances annually introduced into the environment. A non-marginal role, due to their potency, in this framework is represented by drugs and their metabolites excreted by the treated organisms.

The historical concept under which patents are granted for the benefit of the public, particularly in matters of health and safety, has roots that date back centuries. The underlying principle is that, while patents provide inventors with a temporary monopoly on their creations, the aim is to serve the greater good of society.

In contemporary times, this historical concept is reflected in various legal provisions and policies that govern patents. It underscores the understanding that while inventors deserve recognition and protection for their contributions, society, as a whole, should ultimately benefit from these innovations, particularly in areas critical to public health and safety.

In other words, the humanitarian basis of the patent system lies in its goal to strike a balance between fostering innovation and ensuring that the benefits of that innovation are shared for the betterment of society as a whole. In particular, the patent system should ensure a knowledge sharing and the promotion of progress for the scope indicated above.

In particular, the patent system can play a crucial role in addressing humanitarian and global challenges. For instance, it can incentivize the development of sustainable medicines, environmentally sustainable technologies, and solutions for pressing issues like clean energy and water scarcity.

### From reductionism/illuminism for treating symptoms to the rebalancing of states and processes by decreasing anthropic variations:

From the beginning of the 16th century until today, in particular in the field of medical and beneficial products, has been possible to standardise, and hence to validate as active principles suitable for therapy only artificial substances produced with chemically definable alchemical processes or purified/isolated substances.

This path, which although very reductionist has proven to be of great value, allowing many diseases to be eradicated in the past 5 centuries, is now encountering its limits, which derive from the extraneous nature of chemical substances to vital processes. Concerning the development of new sustainable medicaments or beneficial compositions (the latter intended as compositions exerting a homeostasis adjuvating effect), it is now also ascertained that artificial (in particular, chemically synthesised) therapeutical products are generating harmful impacts on biodiversity and native immune systems.

In addition, it has to be noted that, while being chemically analogous to their synthetic counterparts if taken in isolation, natural molecules within a natural matrix (see definition below) are likely to possess distinct fingerprints with respect to their synthetic analogues, due to the totally different synthetic pathway in terms of primary metabolites, reactants, reaction temperatures, energy sources, catalysts etc., potentially influencing their physicochemical behaviour and reactivity, therefore their biological activity.

According to the conventional paradigm, from a chemical-structural viewpoint, the identity of a molecule is embedded in its atomic composition and the geometric arrangement thereof.

By way of example, estragole (1-allyl-4-methoxybenzene), which in nature is prominently identified in essential oils such as those derived from *Ocimum basilicum* and *Artemisia dracunculus* is known in the art for its potential aromatic and medicinal application. The molecular constitution and associated energy states of estragole, contingent upon its origin, have been a subject of robust scientific deliberation. While traditional perspectives postulate uniform molecular attributes, a more rigorous scrutiny suggests nuanced differences.

Given this premise, estragole, whether procured from botanical sources via distillation or synthesized in laboratory confines, should ideally be congruent in its inherent physicochemical attributes.

However, it's paramount to distinguish between the pathways of production. In the native botanical matrices, biosynthesis of estragole is orchestrated by a series of enzymatic reactions, commencing with primary metabolites, and culminating in this specific secondary metabolite. It is known in the art that each of these enzymatic transformations operates within a distinct energy landscape, at temperatures and pressures compatible with the living organism producing estragole, potentially conferring to the molecule a unique energy state.

Conversely, the laboratory synthesis of estragole hinges on chemical reactions steered by different precursors and conditions (such as temperatures and pressures not compatible with the life of a plant). The energy dynamics of such synthetic routes, governed by the thermodynamics and kinetics intrinsic to the reactions, are highly likely to deviate from the plant-mediated enzymatic pathways.

In addition, it is evident that also the isotopic abundances resulting from the two different pathways (natural and synthetic) are unlikely to be the same. Isotopic abundances, even if subtly varied, are known to exert tangible influences on vibrational frequencies, bond strengths, and consequentially, the energy states of the molecule itself [Bigeleisen, J. (1996). Nuclear spin conversion in polyatomic molecules. Journal of Chemical Physics, 105(18), 8121-8129]. Given the likely isotopic disparities between botanical sources and synthetic reagents, the resultant estragole molecules are likely to harbour differential energy imprints and biological activities. In the light of the above, while being chemically analogous, molecules from natural and synthetic origins are reasonably likely to possess distinct energetic fingerprints, potentially influencing their physicochemical properties, reactivity and therefore their biological activity. Indeed, the difference between the activity of a synthetic estragole and natural estragole embedded in a natural matrix (basil extract) has been reported in the art (Suzanne M.F. et al. "Basil extract inhibits the sulfotransferase mediated formation of DNA adducts of the procarcinogen 1'-hydroxyestragole by rat and human liver S9 homogenates and in HepG2 human hepatoma cells" Food and Chemical Toxicology, 2008, 46 (6) 2296-2302, https://doi.org/10.1016/j.fct.2008.03.010.).

### Mode of action

Therapeutical/beneficial products can exert their activity, by modifying one or more specific (defined) pathological or altered activities or by modifying a whole pathological process or state (or altered physiological state in the case of beneficial products).

The first activity is exerted by therapeutic or beneficial products based on the pharmacological relationship between structure and activity (SAR) which is the most relevant relationship in classical pharmacological activities between an active pharmaceutical ingredient (API) and the receptor targeted by said API, which is considered at the level of single molecules. On the other hand, it is likely that products comprising or consisting of natural matrices, (where the networks established among all components of the matrix yielding "the matrix effect" makes it impossible to identify a single marker as representative of the networks because no single component is capable of conveying alone all of the properties specific to the matrix, since no single component reflects the interaction between the matrix and the target living organism), thanks to the networks to network interaction characterising their activity, may exert their therapeutic action by modifying a whole pathological process or state (or altered physiological state in the case or beneficial products) rather than a restricted number of biological functions, however, at present, no simple methods are provided to assess whether a therapeutic or beneficial product provides the modification of a whole pathological/altered state. "Mode of action" is defined by FDA as "the means by which a product achieves its intended therapeutic effect or action", where the "intended effect or action" includes any effect or action intended to reach the medical/beneficial purpose claimed. Since the pharmacological mode of action is distinctive of the medicinal product, it seems necessary to interpret these definitions in line with the requirements of Directive 2001/83 on medicinal products and relative guidelines, as well as in line with the scientific literature on the subject.

Thus, it is possible to identify the following distinctive features of a pharmacological mode of action:
a) There must be at least one well-identified active principle,
b) There must be at least one well-identified cellular target,
c) The action of each active principle is intimately related to its three-dimensional chemical structure, and seemingly minor alterations to the molecule results in major changes in the pharmacological properties. An obvious relationship between ligand structure and activity (SAR) is identifiable,
d) Each active principle must interact with its target and induce a well-identified and scientifically proven interaction coherent with the expected relationship between structure and activity (SAR),
e) The reproducibility of the activity is factually recapitulated by the reproducibility of the structure, especially at the singular molecular level.

Coherence between the claimed effects of its interaction with a biological system and knowledge of the relationship between structure and activity of the ligand is the pivotal aspect of the pharmacological mode of action because on it dwell the characterizing points above.

The mode of action of natural matrices, given their complexity (which results in the above-described matrix effect) clearly cannot satisfy the requirements above. A possible deciphering of natural matrices and biosynthesised molecules may be provided applying quantum biology, however, at present, it is not possible to decipher the complexity of natural matrices, nor their interaction with the receiving organism based on the schemes and tools commonly used to assess the interaction between synthetic or isolated molecules and the receiving organism.

In fact, the interaction natural matrices/receiving organism, being a networks-over-network interaction, evades the logics of structure and activity (SAR) and may investigated only with instruments that can detect their "networks mechanism of action".

Although a matrix mode of action may also comprise mechanical effects (such as e.g. barrier effect) it comprises biological activities (matrix networks acting on receiving organism network) that are ruled by material and immaterial characteristics (e.g. the logics behind the message delivered by a nucleic acid sequence), and its interaction with the body can therefore be approached only through probabilistic canons of the systems theory and cannot at present be validate. As said, knowledge of the identity and amount of each and every molecule in a natural matrix cannot suffice to predict the dynamic and kinetic properties. The opposite happens when selected single molecules, such as APIs, are considered, whereby the SAR as well as the ensuing pharmacodynamic and pharmacokinetic properties are intrinsically related to the chemical identity of the active principle, and to the pharmacodynamic inertia of the excipients. For this reason, the so far developed deterministic canonical concepts of pharmacodynamics and pharmacokinetics make sense solely when referring to a single molecule (the active principle), or a representative thereof (a functional marker), yet they fall short when referring to natural self-assembled matrices. In a time correctly chasing the marvels of artificial intelligence, it appears necessary to acknowledge the existence of a natural self- determining intelligence, yielding self-assembling entities with distinct properties to be approached via the construction of a novel state-of-the-art inspired by the tools of systems theory rather than determinism.

Thus, the need of providing suitable methods for recognising and validating the networks-over-network mechanism of action typical of natural matrices.

By way of example, the existing Medical Device (MD) EU Regulation 2017/745, imposes the need of discriminating a pharmaceutical mode of action from a non-pharmaceutical mode of action.

### Physiological mode of action:

The Medical Device (MD) EU Regulation 2017/745 (Regulation) officially published in Europe May on 5th, 2017 [REGULATION (EU) 2017/745 OF THE EUROPEAN PARLIAMENT AND OF THE COUNCIL of 5 April 2017 on medical devices, amending Directive 2001/83/EC, Regulation (EC) No 178/2002 and Regulation (EC) No 1223/2009 and repealing Council Directives 90/385/EEC and 93/42/EEC] and introduced a completely new governance into all aspects of the lifecycle of a MD. The term Medical Device, according to the Regulation, comprises products which do achieve a therapeutic effect but not with a pharmacological, immunological, or metabolic (Ph.IM) mode of action (MOA), i.e. with a physiological mode of action. The Ph.I.M MOA is the mode of action characterized by a key-lock model where the selected API obeys the rules of SAR and acts on its target receptor. In particular, the Regulation also indicates that a product which modifies a pathological or physiological state or process through a non-Ph-IM mechanism of action is a MD.

It is noted that the MD regulation 2017/745, appears to refer to devices capable of modifying a physiological or pathological state, therefore, appears to extend the definition of medical devices to include products capable of interacting with the human body in such a way as to modify its state. The modification over time of a pathological or physiological state (e.g., an altered physiological state) results in the modification of a pathological or physiological process.

It is therefore necessary to provide methods for assessing whether a therapeutic or beneficial product interacts with the human body in such a way as to modify a state as opposed as to merely modify a function, and to assess whether this state modification is ascribable to a physiological mechanism of action.

Summarising, at the regulatory level it has become of great importance to establish whether a therapeutic product modifies one or few functions or a whole state and the mode of action by which the therapeutic effect is obtained.

It is herein reminded that without marketing authorisation a therapeutical product cannot be produced and put into the market.

In fact, although the regulations regarding medical devices vary by country or region, in many places, medical devices must undergo a regulatory approval process before they can be marketed or sold. This approval process typically involves demonstrating that the device is safe and effective for its intended use.

It's essential for manufacturers to understand and comply with the regulatory requirements specific to the regions where they intend to market their medical devices to ensure legal market access and patient safety.

The present evolution of the regulations (as discussed above) opens new scenarios for manufacturers that at present cannot be explored and exploited due to the absence of suitable tools for assessing and demonstrating the compliance with regulatory requirements.

The present invention provides methods for assessing when a therapeutic or beneficial product consisting of one or more natural matrices, exerts its therapeutic or beneficial effect through a physiological mode of action, i.e., a non-Ph.I.M. mode of action. These methods solve a crucial practical problem that a manufacturer encounters when preparing the dossiers for the marketing authorisation of therapeutic or beneficial products as it allows to define whether said products fulfil the regulatory requirements set out e.g., by various existing Medical Device regulations and, in the affirmative, to support said fulfilment.

### SUMMARY OF THE INVENTION

As known in the art, in pharmacology, when two formulations of the same drug or two drug products are claimed bioequivalent, it is assumed that they will provide the same therapeutic effect or that they are therapeutically equivalent. Two drug products are considered pharmaceutical equivalents if they provide identical amounts of the same active ingredient. Two drugs are identified as pharmaceutical alternatives to each other if both contain an identical therapeutic moiety, but not necessarily in the same amount or dosage form or as the same salt or ester. Two drug products are said to be bioequivalent if they are pharmaceutical equivalents (i.e., similar dosage forms made, perhaps, by different manufacturers) or pharmaceutical alternatives (i.e., different dosage forms) and if their rates and extents of absorption do not show a significant difference to which the active ingredient or active moiety in pharmaceutical equivalents or pharmaceutical alternatives become available at the site of action when administered at the same molar dose under similar conditions in an appropriately designed study.

As explained in detail above, active principles in pharmaceutical drugs are known to act through a pharmacological mode of action.

Conversely, to act with a physiological mechanism of action, a product must be 100% natural. Natural materials, such as products comprising or consisting of natural matrices, are entities which maintain at least in part the autopoietic properties of their starting materials which belong to the living domain, and display own properties which are represented by networks of material and immaterial relationships, that interact with the network of relationships of the treated subject (networks-to-network interactions) thereby recapitulating an interaction with features and complexities that are physiological-like.

Therefore, according to the present description, a product comprising or consisting of one or more natural matrices is a product which is 100% natural, which means that the product does not contain additional artificial substances, i.e., substances of chemical synthesis made by man through laboratory processes.

In addition, according to the present description, a product comprising one or more natural matrix, does also not contain any added isolated molecule, e.g., excipient/s or active principle/s even if of natural origin.

The authors of the present invention surprisingly found, when analysing therapeutic products comprising or consisting of one or more natural matrices, that, contrary to API based therapeutic products, they appear to act in a way that does not involve (as opposed to the pharmacological key-lock paradigm) a one-to-one interaction with specific particular constituents of the human body. Indeed, the authors found that different batches of the examined products, although not homogeneous in their quali-quantitative composition at molecular level, exerted a homogeneous and conserved overall therapeutic or beneficial effect notwithstanding their variable composition.

In particular, as disclosed in Patent applications PCT/IB2024/050280; US18/410,096; UK 2400422.8; JP 2024-003433; CA 3,225,879; AU 2024200219, the authors of the present invention, could validate the therapeutic effectiveness of different batches of products comprising or consisting of natural matrices, with reference to a reference batch whose therapeutic effect was ascertained at least in preclinical experiments although the examined batches did not have the same quali-quantitative composition of said reference batch.

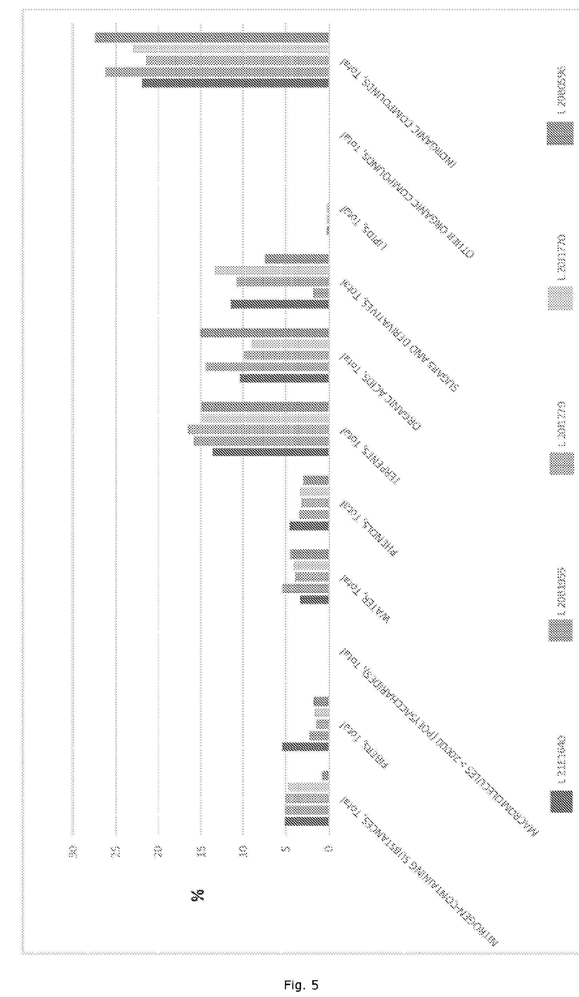

In fact, as expected for products comprising or consisting of natural matrices, although each batch was prepared following standardised procedures to obtain *a priori* a high degree of homogeneity between different batches, a detailed quali-quantitative analysis of all the tested batches (see figure 5) clearly demonstrated relevant batch-to-batch quali-quantitative differences that would have lead, with conventional validation methods used for synthetic or isolated drugs, to the discard of batches that were indeed therapeutically valid, as well as the impossibility to hypothesise the presence of an API.

It is herein reminded that, due to their own nature, natural matrices are variable in their composition even when obtained from the same kind of raw source, by way of example, the skilled person knows very well that a natural matrix obtained from an individual of a plant species, will never be absolutely identical to another natural matrix obtained from a different individual of the same plant species, even among plants in the same field, due to the genetic and epigenetic variability of each living organism.

The authors of the present invention analysed the qualitative and quantitative chemical composition of different batches of products comprising one or more natural matrices and their therapeutic or beneficial action. Figure 5 shows, e.g., the different qualitative and quantitative composition of different batches of product A, also named Arté-GX herein, comprising natural matrices (see also examples). Notwithstanding the different qualitative and quantitative chemical composition of all the batches analysed, the authors surprisingly found that, in all the examined products comprising one or more natural matrices the different molecular entities within each matrix appeared to interact in a redundant manner with each other both functionally and structurally providing the same therapeutic or beneficial (homeostasis-adjuvant) effect despite their differences in quali-quantitative molecular composition.

Indeed, the products showed a functional (therapeutical or beneficial) resilience despite the variability in their qualitative and quantitative molecular composition.

In other words, the authors surprisingly found that for all the examined products, different batches of the same product showed a consistent regulation (in terms of trend and magnitude) of all the examined biological activities, relevant to the desired therapeutical or beneficial effect, notwithstanding batch-to-batch qualitative and quantitative composition differences, herein also defined as "functional resilience effect". The observed maintenance of the biological activity is likely due to the fact that, as said above, the emerging properties of a natural matrix are due to the matrix networks acting as a whole entity with distinctive properties, and may not be ascribable to each single molecule as if it were in isolation, the therapeutic action being through a non-pharmacological mechanism of action different from the classical therapeutic products based on the pharmacological relationship between structure and activity (SAR) which is the most relevant relationship in classical pharmacological activities between an active pharmaceutical ingredient (API) and the receptor targeted by said API, which is considered at the level of single molecules.

This agrees with the likelihood that the products analysed by the inventors, may exert their therapeutic or beneficial action by acting on a whole pathophysiological or altered physiological state.

Materials such as natural materials appear to be particularly compliant with the EU directive MD (Medical Device) definition. An example of a natural material is a natural matrix (e.g., a vegetal matrix). It is characterized by a large number of components interacting within the matrix in a way similar to that in the organism of origin. This is possible when the manufacturing process does not isolate single component molecules by processes that are considered artificial for this reason, thus producing MPs (medical products)/MDMS (medical devices made of substances) of natural origin. The matrix is thus characterized by interactive networks of components. The interactions affect the reactivity of the components, resulting in the so-called "matrix effect" or "emergent properties". The matrix effect shows that the structural and functional properties of the matrix cannot be attributed based on the properties of the individual isolated components when these are studied in isolation (Yong et al., 2022 "Supramolecular assemblies based on natural small molecules: Union would be effective". Materials Today Bio. 15.doi.org/10.1016/j.mtbio.2022.100327; Lehn, 2002 Toward complex matter: Supramolecular chemistry and self-organization. PNAS 99, 4763-4768 doi:10.1073/pnas.072065599). Typically, a matrix has self-assembling and self-organizing properties, resulting in supramolecular structures and functional interactions that can respond to different environmental conditions (Lehn, 2002). This phenomenon has been specifically attributed to living matter. The networked interactions within a matrix are relevant to the networked interaction between the physiological functions of the human body when they maintain a physiological state or re-establish the physiological state from a pathological state (Stear, 1973 Systems Theory Aspects of Physiological Systems. IFAC Proceedings Volumes. Volume 6, Issue 4, p. 496-500. ISSN 1474-6670. doi.org/10.1016/S1474-6670(17)68074-1; Bartsch et al., 2015 Network Physiology: "How Organ Systems Dynamically Interact" PLoS One 10, ;10(11):e0142143. doi: 10.1371/journal.pone.0142143 ; Ivanov, 2021 "The New Field of Network Physiology: Building the Human Physiolome. Front. in Network Phys. 1, doi:10.3389/fnetp.2021.711778).

It appears that the MDR has specifically defined these products as devices by specifying that a device "modifies ... a physiological or pathological process or state". Compared to Directive 93/42, which limited the device to the modification of pathological processes, the mandate to modify a "state" of the human body seems an invitation to evolve the state of the art. It is also an alternative to MPs which, by definition, modify single biological functions. Thus, it seems that the "networks over a network" interaction between natural materials and the human body could be considered as characteristic of medical devices. Such interaction of a natural material with the human body differs fundamentally from the "pinpointed" interaction of a substance with its receptor(s) (the pharmacological, immunological, or metabolic, hereinafter, PhIM mechanism of MPs) and from the mechanical/chemical/physical mechanisms of MDMS. The network mechanism accompanies, in each specific context, the physiological actions underlying the state in question, in a coordinated, circular, nonlinear manner.

Natural materials are fundamentally different from "substances", including substances of natural origin. Since they are not represented by their individual components, they need a dedicated model. Therefore, to describe natural materials, it is necessary to extend the reductionist approach and use the innovations of the last century. Conceptually, this means referring to systems theory. From an experimental point of view, preclinical evidence involves systems biology approaches such as omics sciences (e.g., transcriptomics) and bioinformatics evaluations.

These allow appropriate assessments of the matrix (the acting networks), the human body (the receiving network) and allow to consider the interaction between the two as a "networks over a network" interaction. A mechanism that accompanies, in each specific context, the coordinated redundancy and resilience that characterize physiology corresponds to a 'physiological mechanism of action' and could be characterized by a network paradigm, distinct from the targeted and non-targeted models that describe the PhIM and the mechanical/chemical/physical mechanism, respectively.

In the present application, the authors demonstrate how to verify whether the therapeutic or beneficial action exerted by products comprising one or more natural matrices (or consisting of one or more natural matrices), is exerted through the modification of a pathological or altered physiological state and not, as classical drugs, through the modification of one or few pathological or physiological functions and provide a method, previously not available in the art, for validating that a therapeutic or beneficial effect modifies a pathological state (as opposed to one or few pathological functions) or an altered physiological state (as opposed to one or few altered physiological functions). For the reasons explained above, when a pathophysiological or altered physiological state is modified by a product comprising one or more natural matrices, and said product shows functional (therapeutic or beneficial) resilience, the mechanism of action of said product is determined to be a physiological mechanism of action.

The possibility of demonstrating the different mode of action with which metabolic networks such as natural matrices may interact with the metabolic networks of the receiving organisms with respect to the mode of action of an artificial API can allow the evolution towards new therapeutic protocols more in conformity with the integrity of living organisms thereby allowing to substitute, as much as possible, artificial products and processes for the preparation thereof with physiological products and processes. To move in this direction, i.e., a direction allowing to move to an holistic vision of the natural complex systems such as natural matrices, it will be necessary to understand the self-assembly of the natural matter and to provide suitable methods for assessing whether a therapeutic or beneficial product exerts its desired activity by modifying a physio pathological or altered pathological state rather than one or few functions (i.e., through a networks-network interaction) and whether the mode of action of said product is not merely not pharmacological (displaying SAR) but, rather, physiological.

The prospective and evolutionary change in the medical field will hopefully be that of moving from the classic pharmacochemical dictates, which at present tend to focus on single symptoms, organs, active principles, reactions and so on, to a holistic view in which each actor, form the therapeutic product to the receiving subject, is considered as a complex network and not as a singled out point, with an expected gain based on the complexity of the interaction with the body, which should deliver an improvement of therapies of complex diseases such as syndromes.

Accordingly, object of the present invention are defined in the appended claims and comprise:
A method for measuring different batches of a therapeutic product comprising one or more natural matrices, to determine that said product exerts its therapeutic effect through a physiological mechanism of action, wherein said physiological mechanism of action is a non-pharmacological, -immunological, -metabolic (Ph.IM) mode of action, and is characterized by the regulation of a network of biological activities that underly hallmarks defining a pathological state, and by the therapeutic functional resilience of said product among said different batches the method comprising:
a) selecting a therapeutic product comprising one or more natural matrices and providing different batches of said product;
b) providing a list of hallmarks representative of said pathological state;
c) identifying for each of said hallmarks one or more biological activities modifications underlying said pathological state thereby pinpointing a network of biological activities whose modulation concurs to said pathological state;
d) identifying one or more gene expression patterns whose modulation concurs to the modulation of said one or more biological activities underlying the therapeutic effect of the product tested and determining the modulation trend in terms of up or down modulation of said one or more biological activities, in said network, concurring to said pathological state or to a healthy state
e) performing, on each of said different batches, at least one *in vitro* cell-based assay wherein the read out of said cell-based assay is representative of the modulation of one or more biological activities underlying the desired therapeutic effect, on the following groups of cells
(e1) at least one control group and at least two test groups of cells having the diseased phenotype relevant to the intended use of the therapeutic product; or
(e2) at least one group of cells with a healthy physiological phenotype; and at least one control group and at least two test groups of said cells with a healthy physiological phenotype wherein the diseased phenotype relevant to the intended use of the therapeutic product is induced,
and treating each of said test groups of cells with one of said different batches of therapeutic product;
   (f) determining the modulation or modulation pattern of each of said gene expression patterns on each of said groups of cells of step (e) and calculating the respective modulation values for each of said one or more biological activities;
   (g) said therapeutic product is shown to exert its therapeutic effect through said physiological mechanism when, comparing said modulation values, the conditions (g1) and (g2) are met:
      (g1) at least 50% of said one or more biological activities for each hallmark are modulated by each product batch with the modulation trend of the network concurring to the healthy state, and the modulation values determined in (f) of each of said at least 50% one or more biological activities for said test groups of cells of (e1) differ, respectively, from the ones of said control group of cells of (e1) of at least 0.15, or
      at least 50% of said one or more biological activities for each hallmark are modulated by each product batch with the modulation trend of the network concurring to the healthy state, and the modulation values determined in (f) of each of said at least 50% one or more biological activities for said test groups of cells of (e2) differ, respectively, from the ones of said control group of cells of (e2) by at least 15%, and
      (g2) functional resilience of the product is demonstrated by the modulation values for each one or more biological activities of each said test groups of cells differing by less than 20% from the average of said values;
      wherein gene expression is assessed by transcriptomic analysis; and

A method for measuring different batches of a beneficial product adjuvating homeostasis in an altered physiological state the product comprising one or more natural matrices, to determine that said product exerts its beneficial effect through a physiological mechanism of action wherein said physiological mechanism of action is a non-pharmacological, -immunological, -metabolic (Ph.IM) mode of action, and is characterized by the regulation of a network of biological activities that underly hallmarks defining a pathological state, and by the beneficial functional resilience of said product among said different batches the method comprising:
a) selecting a beneficial product comprising one or more natural matrices and providing different batches of said product;
b) providing a list of hallmarks representative of a pathological state that can stem from said altered physiological state;
c) identifying for each of said hallmarks one or more biological activities modifications underlying said pathological state thereby pinpointing a network of biological activities whose modulation concurs to said pathological state;
d) identifying one or more gene expression patterns whose modulation concurs to the modulation of said one or more biological activities underlying the beneficial effect of the product tested and determining the modulation trend in terms of up or down modulation of said one or more biological activities, in said network, concurring to said pathological state or to a healthy state
e) performing, on each of said different batches, at least one *in vitro* cell-based assay wherein the read out of said cell-based assay is representative of the modulation of one or more biological activities underlying the desired beneficial effect, on the following groups of cells:
   at least one control group of cells having healthy phenotype or at least one control group of cells wherein the aberrantly modulated phenotype targeted by said beneficial product is suitably induced, and at least two test groups of cells taken from said control group
   and treating, each of said test groups of cells, with one of said different batches of beneficial product;
(f) determining the modulation or modulation pattern of each of said gene expression patterns on each of said groups of cells of step (e) and calculating the respective modulation values for each of said one or more biological activities;
(g) said therapeutic product is shown to exert its beneficial effect through said physiological mechanism when comparing said modulation values the conditions (g1) and (g2) are met:
   g1) at least 50% of said one or more biological activities for each hallmark are modulated by each product batch with the modulation trend of the network concurring to the healthy state and the modulation values determined in (f) of each of said at least 50% one or more biological activities for said test groups of cells differ, respectively, from the ones of said control group of cells of at least 0.15 and
   g2) functional resilience of the product is demonstrated by the modulation values for each one or more biological activities of each said test groups of cells differing by less than 20% from the average of said values;
   wherein gene expression is assessed by transcriptomic analysis.

### DETAILED DESCRIPTION OF THE FIGURES

### Legend of the batches:

### Figures 2-5:

Product A (Arté-Gx) in lyophilised form (see detailed composition of the product in Example 1):
Batch 20B1955 also L 20B 1955
Batch 20B0596 also L 20B0596
Batch 20I1297 also L 20I1297
Batch 21E1640 also L 21E1640
Batch 20J1770 also L 20J1770

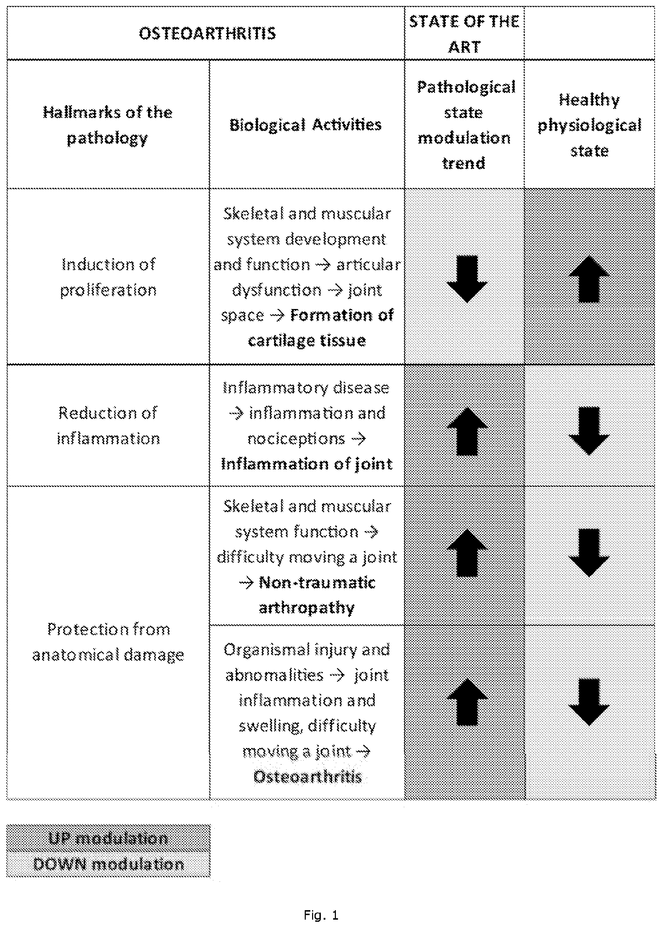

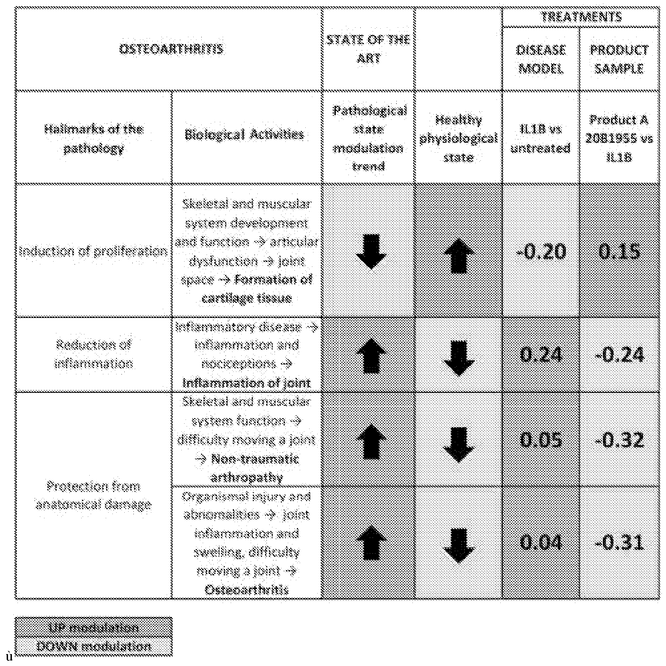

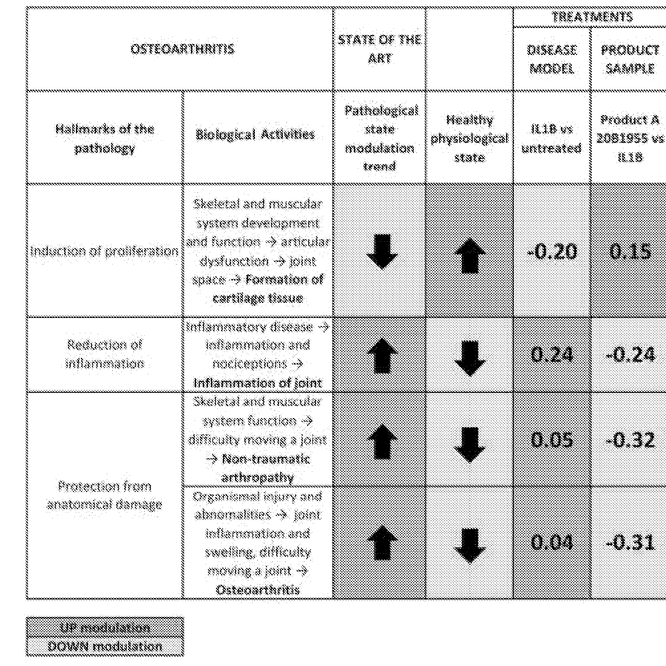

Figure 1 Exemplification of hallmarks of osteoarthritis including the trend of the hallmark representing an improvement of the pathological state, (column 1), biological activities concurring to define pathological state (column 2) and modulations thereof in the pathological state (column 3). Modulation of each of said biological activities representative of a healthy physiological state (column 4). Dark grey: up modulation light grey: down modulation.

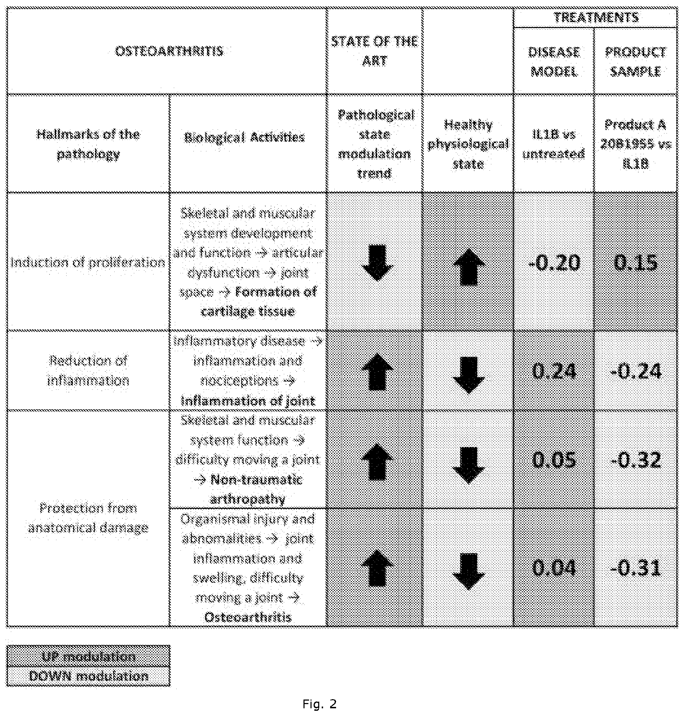

Figure 2 Modulation of selected biological activities in a chondrocytes cell-based assay in osteoarthritis: column 1 hallmarks including the trend of the hallmark representing an improvement of the diseased state, column 2 biological activities, column 3 modulation trend of a network of biological activities in pathological state, column 4 desired modulation of a network of biological activities concurring to the healthy physiological state, column 5 cell-based assay without therapeutic treatment (control group) representing a network of biological activities modulation in the pathological state, column 6 modulation induced by tested product. The cell-based assay shows that product sample modulates the selected network of activities according to the trend concurring to the healthy physiological state profile. The numbers reported in each square represent the value quantifying the modulation (in terms of z-score) calculated according to the examples representing the modulation of each biological function observed (parameters used: transcriptomics).

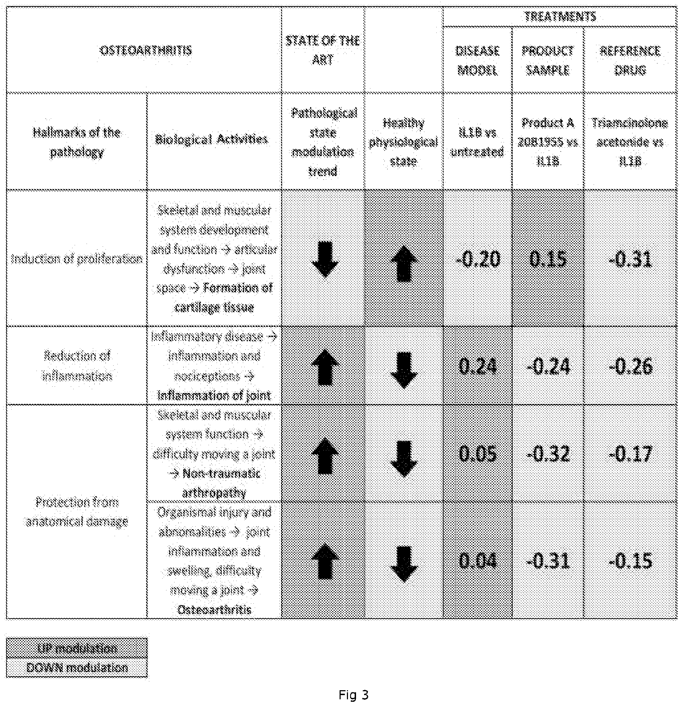

Figure 3 Modulation of selected biological activities in a chondrocytes cell-based assay in osteoarthritis: column 1 hallmarks including the trend of the hallmark representing an improvement of the diseased state, column 2 network of biological activities, column 3 modulation trend of a network of biological activities in the pathological state, column 4 desired modulation of the network of biological activities concurring to the healthy physiological state, column 5 cell-based assay without therapeutic treatment representing the biological activities network modulation of the pathological state (control group), column 6 modulation induced by tested product, column 7 modulations induced by a reference drug used in treatment of osteoarthritis (Triamcinolone Acetonide).The cell-based assay shows that the product sample modulates all the network of selected activities according to trend concurring to the healthy physiological state profile showing the modulation of a whole pathological state. On the other hand, the reference drug is not able to effectively modulate all the biological activities needed to define the healthy physiological state therefore (no modulation of a pathological state). The numbers reported in each square represent the value quantifying the modulation (in terms of z-score) calculated according to the examples representing the modulation of each biological activity observed.

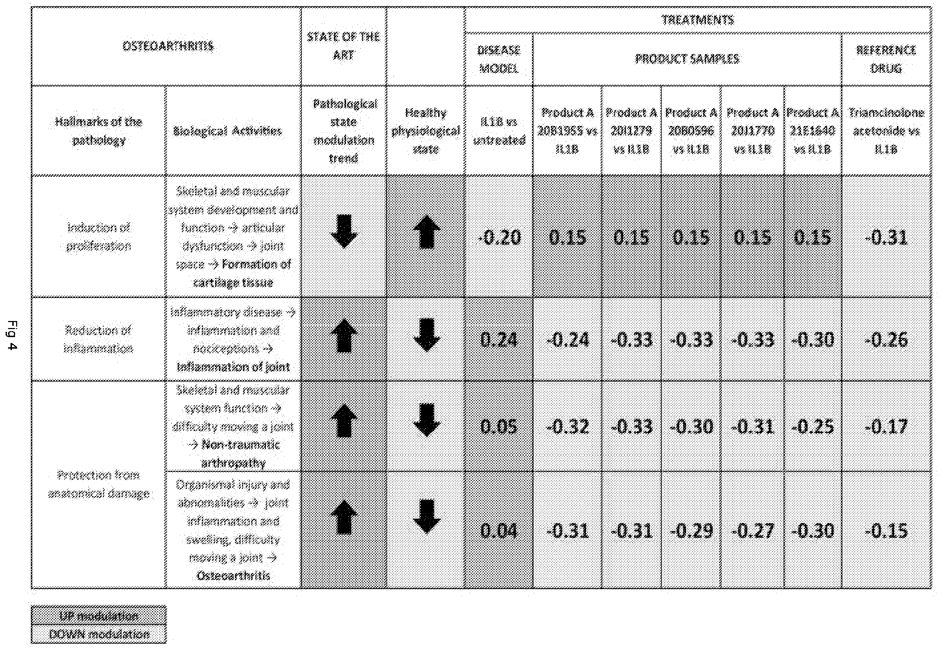

Figure 4 Modulation of a network of selected biological activities in a chondrocytes cell-based assay in osteoarthritis: column 1 hallmarks including the trend of the hallmark representing an improvement of the diseased state, column 2 network of biological activities, column 3 modulation trend of the network of biological activities in the pathological state, column 4 desired modulation of the network of biological activities concurring to the healthy physiological state, column 5 cell-based assay without therapeutic treatment representing the biological activities network modulation in the pathological state (control group), column 6-10 modulation induced by different batches of the tested product, column 11 modulations induced by a reference drug (Triamcinolone Acetonide).The cell-based assay shows that all the tested product batches modulate the network of all the selected activities according to modulation trend concurring to the healthy physiological state profile (modulation of a state and functional resilience). On the other hand, the reference drug is not able to effectively modulate all the biological activities needed to define the healthy physiological state (it does not act on the network). The numbers reported in each square represent the value quantifying the modulation (in terms of z-score) calculated according to the examples representing the modulation of each biological activity of the network observed.
Figure 5 Targeted metabolomics of five batches of product A main chemical classes. The figure clearly shows that all the batch tested differ from one another in terms of quali-quantitative composition. Comparison with tests of the selected biological activities of the same batches on the cell-based-assay (figures 3 and 4), shows that the quali-quantitative analysis of different batches of a product for use in the treatment of a pathological condition, when the product comprises or consists of one or more natural matrices does not allow to correctly estimate its activity profile.

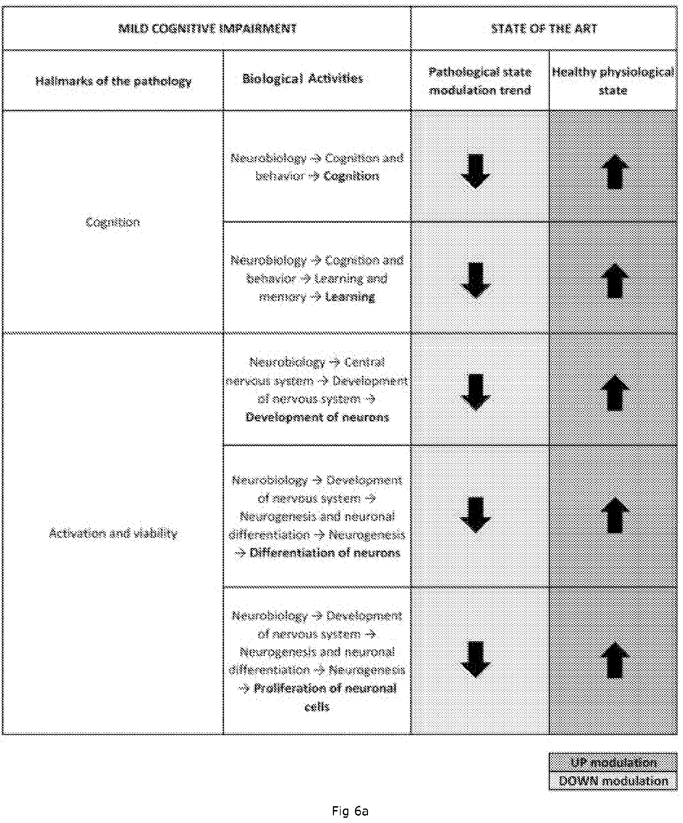

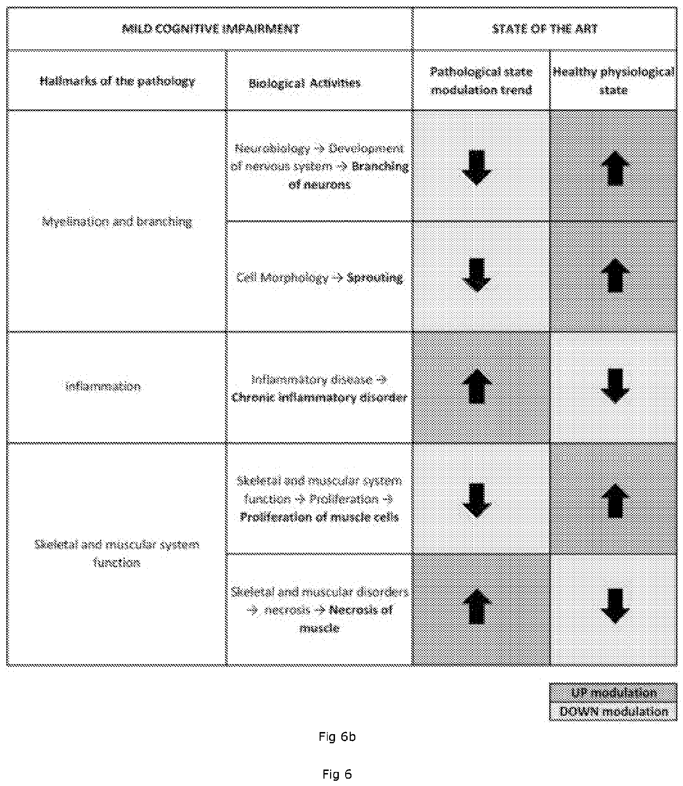

Figure 6a and 6b Exemplification of hallmarks of mild cognitive impairment, (column 1), network of selected biological activities concurring to define pathological state (column 2) and modulations thereof in the pathological state (column 3). Modulation trend of each of said biological activities network concurring to the healthy physiological state (column 4). Dark grey: up modulation light grey: down modulation.

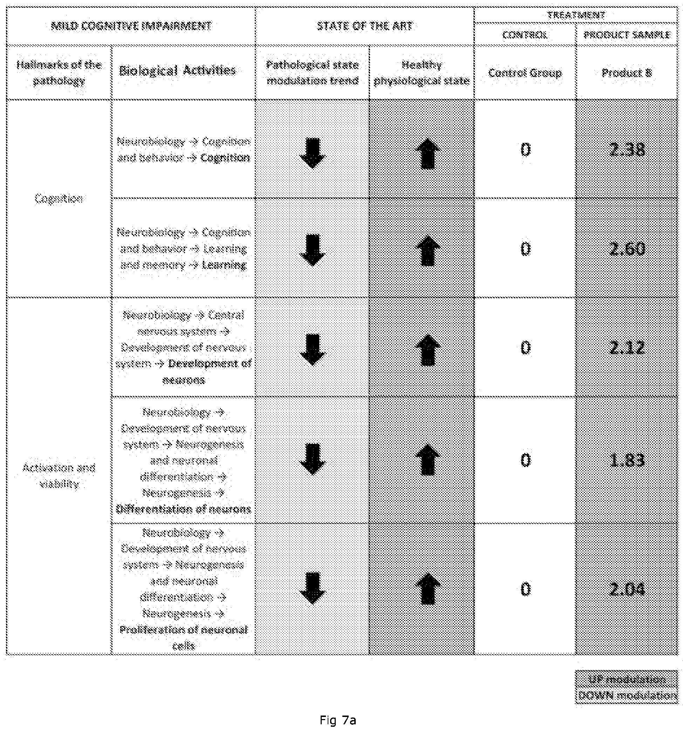

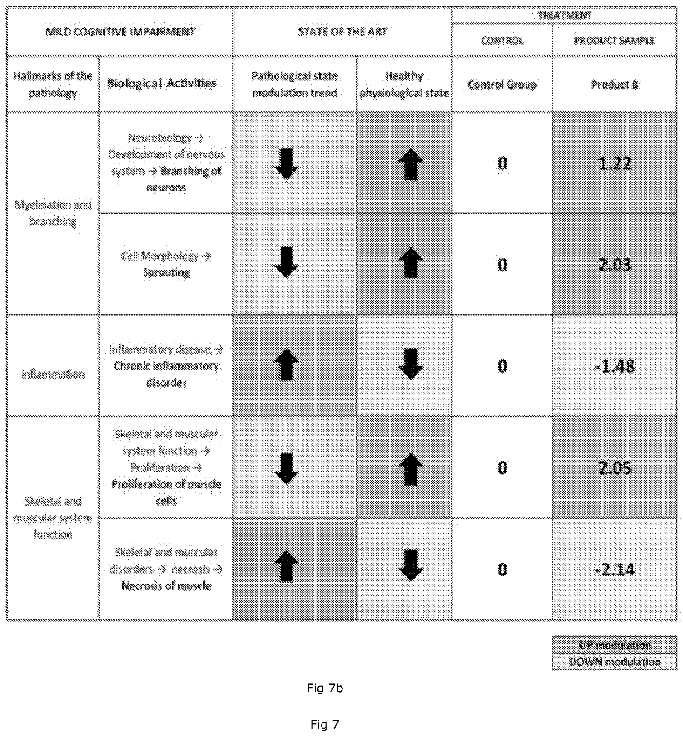

Figure 7a and 7b Modulation a network of selected biological activities in a neuroblastoma cell-based assay in mild cognitive impairment: column 1 hallmarks, column 2 biological activities, column 3 modulation trend of biological activities in pathological state, column 4 desired modulation of biological activities in healthy physiological state, column 5 untreated cells representing the biological activities network modulation of the pathophysiological state control, column 6 modulation induced by tested product (Product B). The cell-based assay shows that product sample modulates the selected activities network according to the trend concurring to the healthy physiological state profile. The numbers reported in each square represent the value quantifying the modulation (z-score) calculated according to the examples representing the modulation of each biological activity observed.

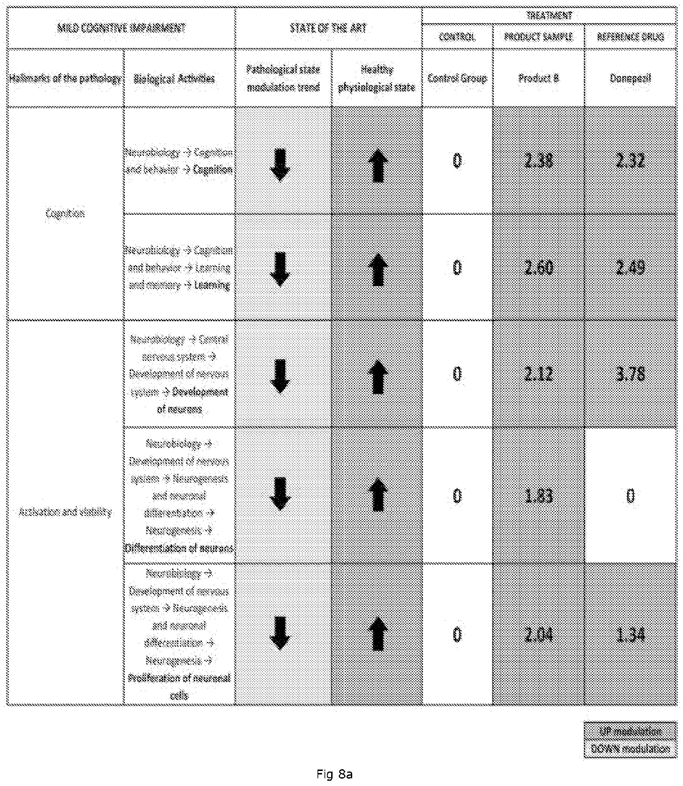

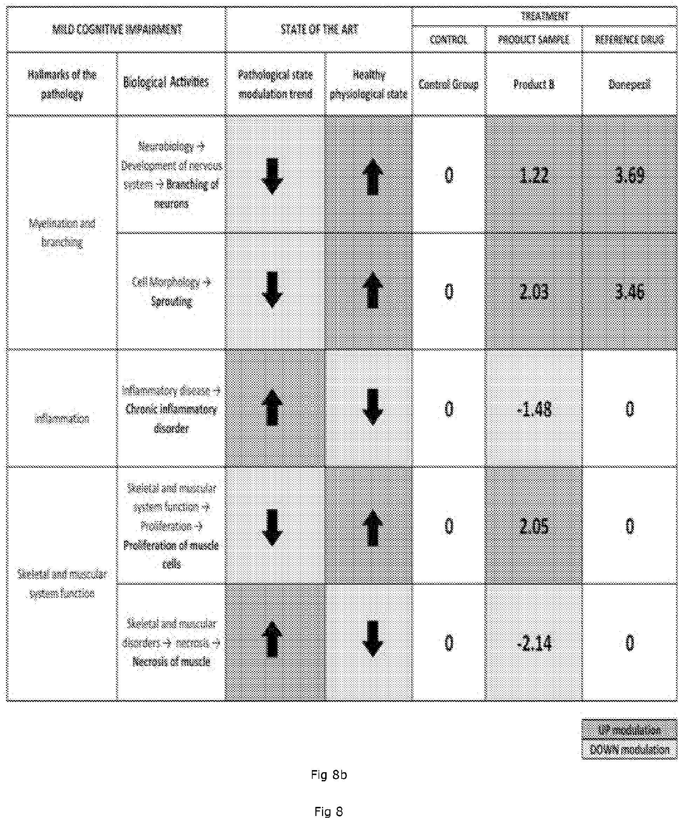

Figure 8a and 8b Modulation of a network of selected biological activities in a neuroblastoma cell-based assay in mild cognitive impairment: column 1 hallmarks, column 2 network of biological activities, column 3 modulation trend of the network of biological activities in the pathological state, column 4 modulation trend of the network of biological activities concurring to a healthy physiological state, column 5 untreated cells representing the biological activities network modulation of the pathophysiological state control, column 6 modulation induced by tested product (Product B), column 7 modulations induced by a reference drug (Donepezil).The cell-based assay shows that product sample modulates the network of all the selected activities according to the modulation trend concurring to the healthy physiological state profile. On the other hand, the reference drug is not able to effectively modulate whole network of biological activities needed to define the healthy physiological state The numbers reported in each square represent the value quantifying the modulation (z-score) calculated according to the examples representing the modulation of each biological activity observed.

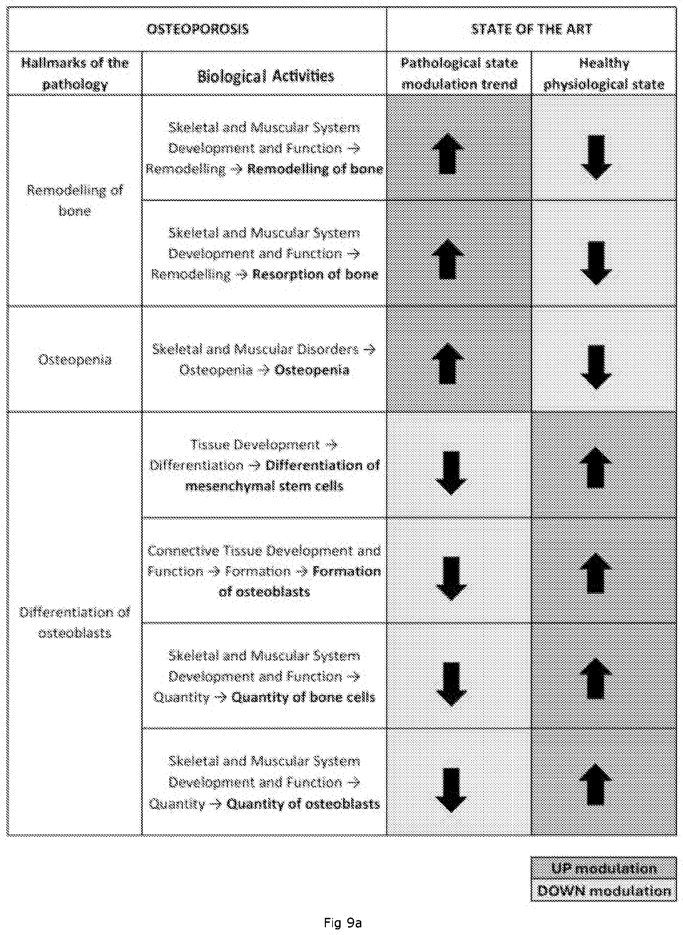

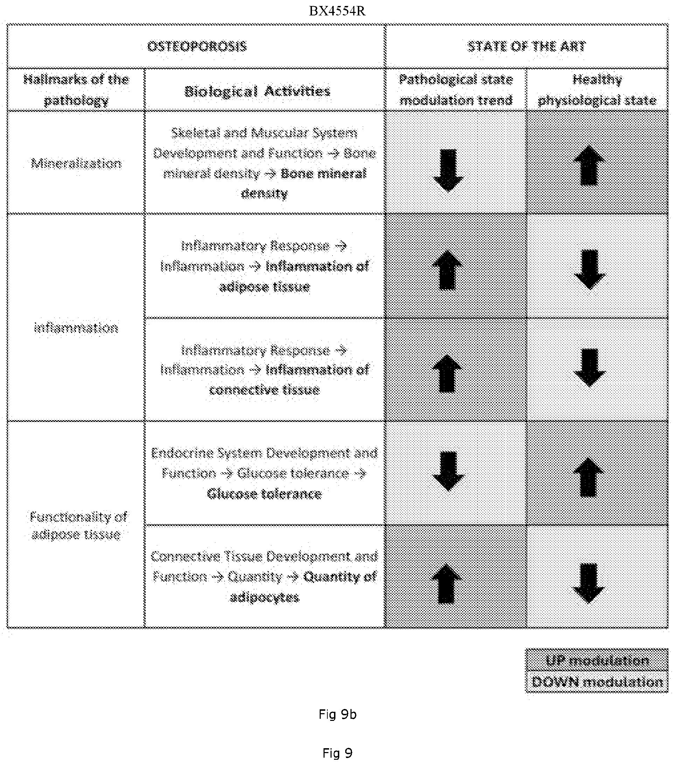

Figure 9a and 9b Exemplification of hallmarks of osteoporosis, (column 1), network of biological activities concurring to define pathological state (column 2) and modulations thereof in the pathological state (column 3). Modulation trend of the network of said biological activities concurring to a healthy physiological state (column 4). Dark grey: up modulation light grey: down modulation.

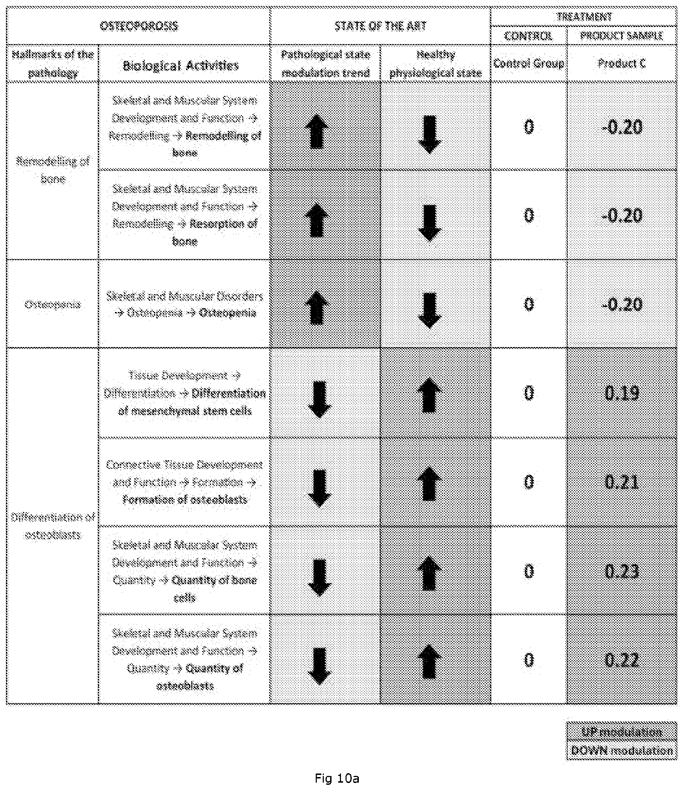

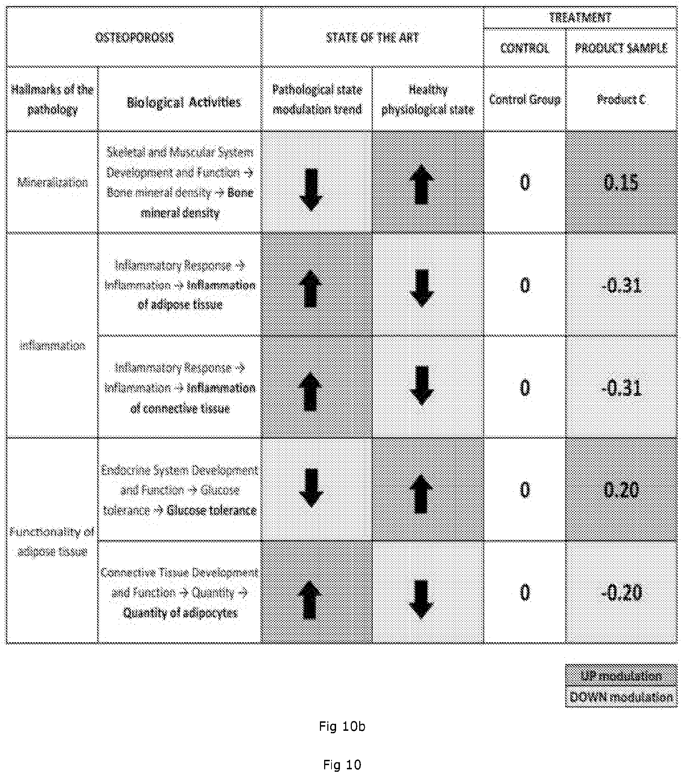

Figure 10a and 10b Modulation of a network of selected biological activities in a human, adipocyte-derived, mesenchymal stem cell lines (hADMSC), capable of differentiating into osteoblasts and mineralize the extracellular matrix (ECM) cell-based assay in osteoporosis: column 1 hallmarks, column 2 network of biological activities, column 3 modulation trend of the network of biological activities in the pathological state, column 4 modulation trend of the network of biological activities concurring to a healthy physiological state, column 5 suitably induced cells representing the network of biological activities modulation of the non-modulated state control, column 6 modulation induced by tested product (Product C). The cell-based assay shows that product sample modulates the whole network of selected activities according to the trend concurring to the healthy physiological state profile. The numbers reported in each square represent the value quantifying the modulation (z-score) calculated according to the examples representing the modulation of each biological activity observed.

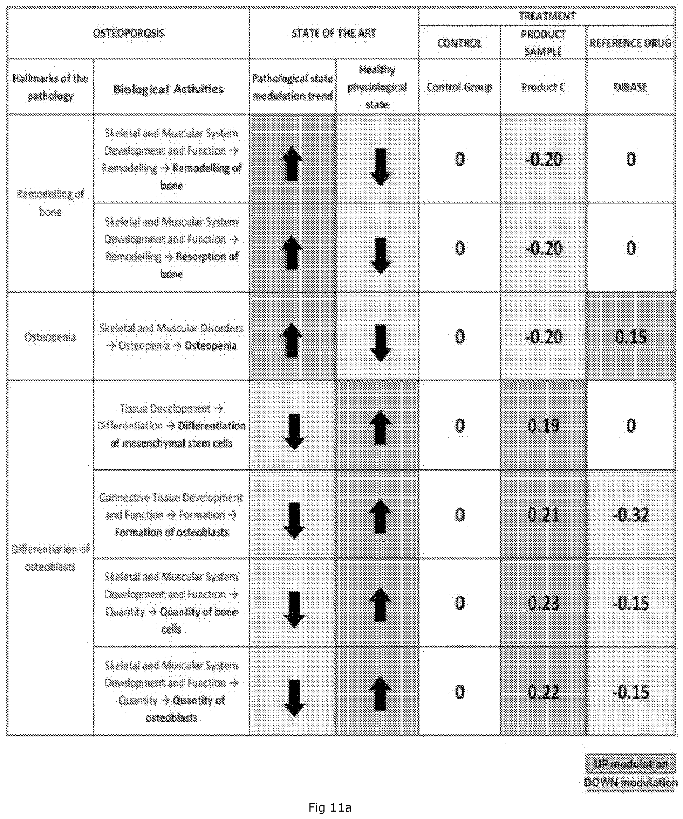

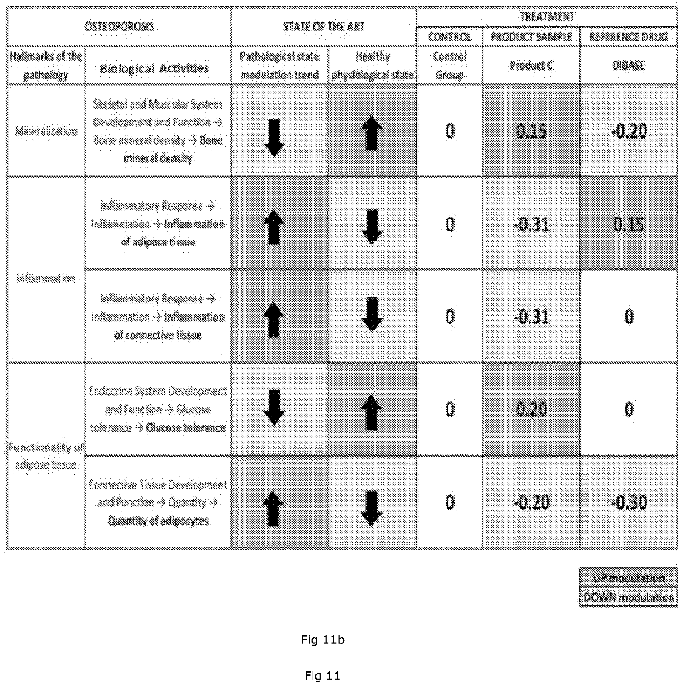

Figure 11a and 11b Modulation of a network of selected biological activities in a human, adipocyte-derived, mesenchymal stem cell lines (hADMSC), capable of differentiating into osteoblasts and mineralize the extracellular matrix (ECM) cell-based assay in osteoporosis: column 1 hallmarks, column 2 network of biological activities, column 3 modulation trend of the network of biological activities in the pathological state, column 4 modulation trend of the network of biological activities concurring to the healthy physiological state, column 5 suitably induced cells representing the biological activities network modulation of the non-modulated state control, column 6 modulation induced by tested product (Product C), column 7 modulations induced by a reference drug (DIBASE).The cell-based assay shows that product sample modulates all the selected activities of the network with a modulation trend according to the one concurring to the healthy physiological state profile. On the other hand, the reference drug is not able to effectively modulate all the whole network of biological activities needed to define the healthy physiological state The numbers reported in each square represent the value quantifying the modulation (z-score) calculated according to the examples representing the modulation of each biological activity observed.

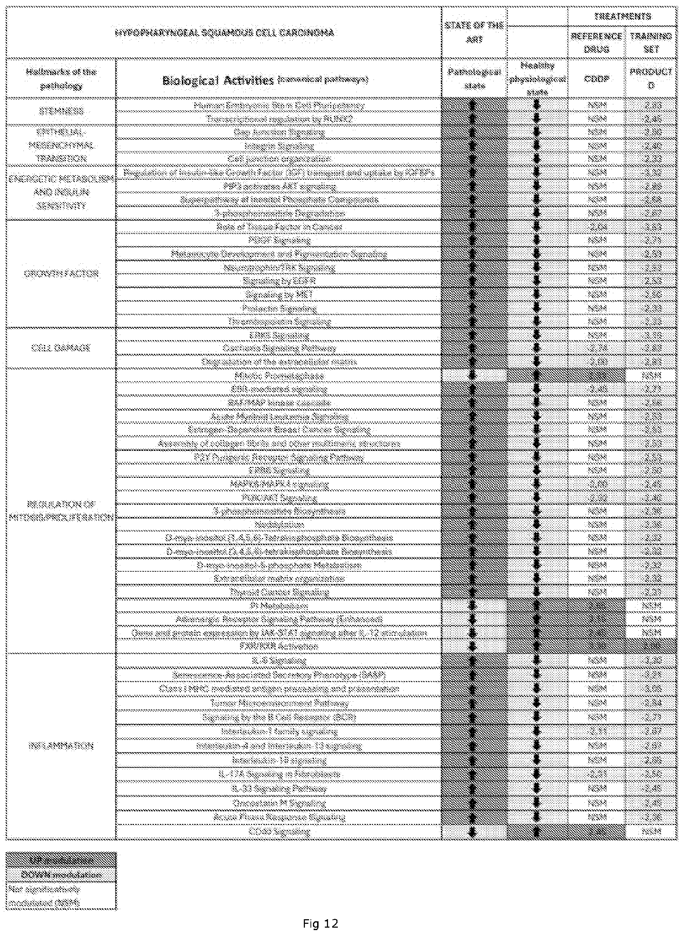

Figure 12 Modulation of a network of selected biological activities in a squamous carcinoma cell line-based assay: column 1 hallmarks of the diseased state, column 2 network of biological activities (represented by canonical pathways), column 3 predicted modulation of the network of biological activities concurring to the pathological state, column 4 modulation trend of the network of biological activities concurring to the healthy physiological state, column 5 cell-based assay modulations induced by reference drug represented by Cisplatin : cis-diamminedichloroplatinum (II) (CDDP) and column 6 modulation induced by Product D . The cell-based assay shows that Product D modulates a higher number of desired activities, according to the trend concurring to the healthy physiological state, than treatment with CCDP. The numbers reported in each square represent a Z-score calculated with the method of the invention representing the modulation of each biological activity observed.

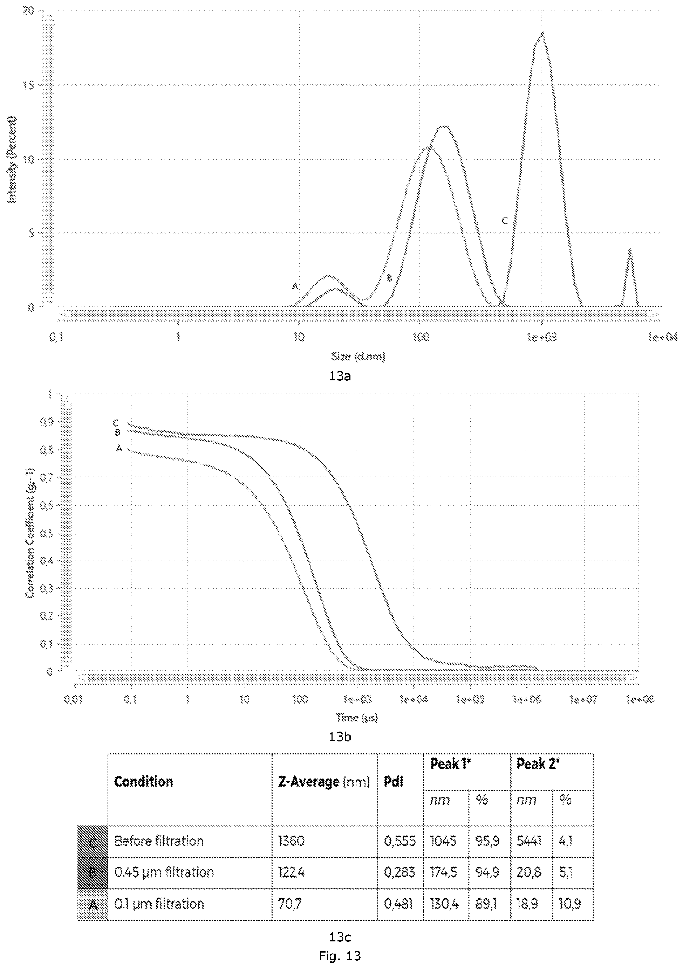

Figure 13 DLS detection of supramolecular structures in aqueous sweet fennel extracts, the figure shows the average particle size distributions (Fig 13a) and the average correlation functions, Fig. 13b (obtained from the 3 measurements). Averaged results of Z-Average, PdI and Peaks results obtained from 3 repeated measurements are reported in the figure 13c. Z-Average is the intensity weighted mean diameter, PdI is the polydispersity index.

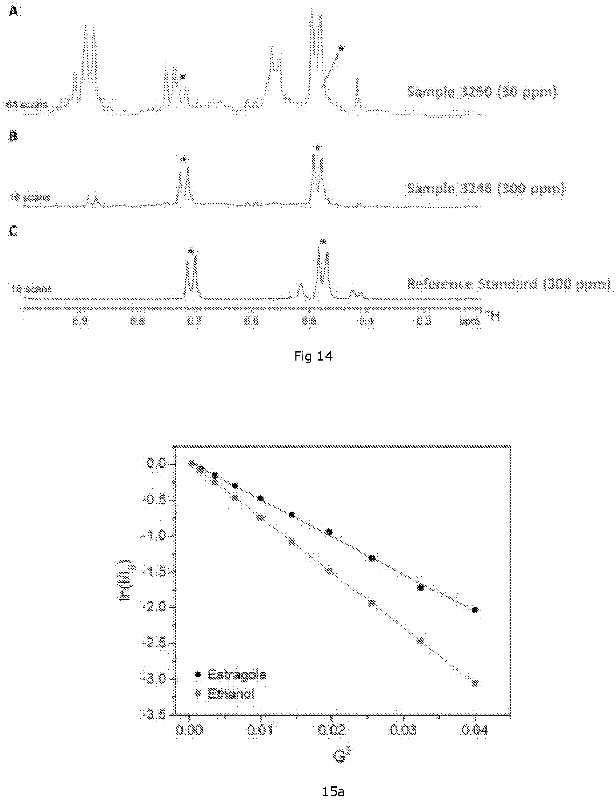

Figure 14 1H NMR spectra of the extracts of sweet fennel (A; Lot 3250) bitter fennel (B; Lot 3246) and reference standard of estragole (C). Peaks associated to estragole's aromatic protons are highlighted with a star sign.

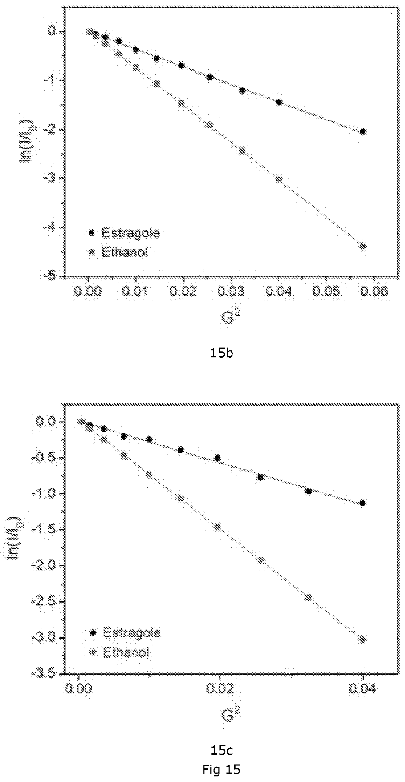

Figure 15 Plots of log(I/I0) versus G2. Black points refer to the estragole reference standard, i.e., estragole isolate(a), the extracts of bitter fennel (b; Lot 3246) sweet fennel (c; Lot 3250). Red points refer to the calibration reference.

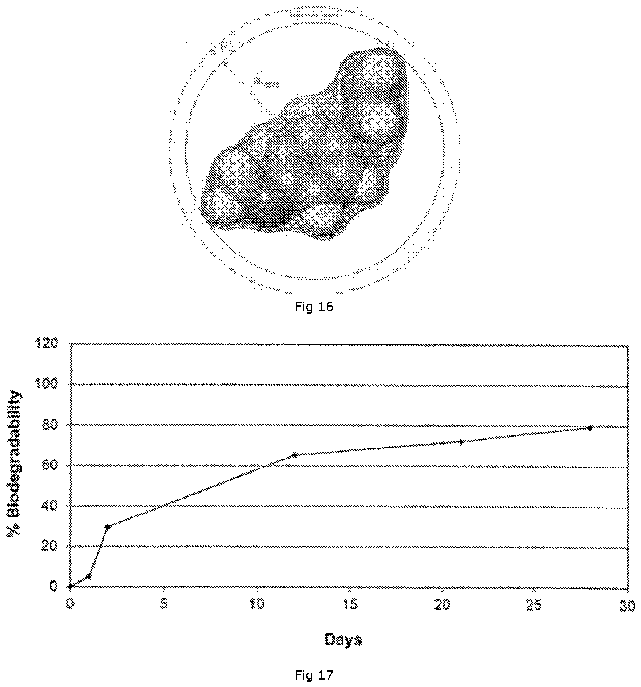

Figure 16 Theorical van der Waals radius (R_{νdW}) of estragole, and its hydrodynamic radius (RH) obtained extending the former by the solvent shell.
Figure 17 Graphic of aerobic biodegradation of product A during biodegradability test according to method OECD 310: 2014 (example 7).

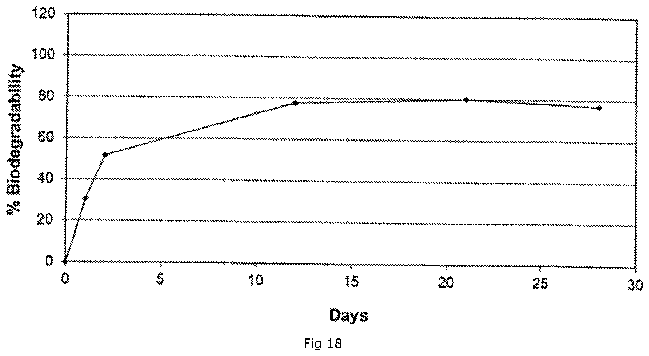

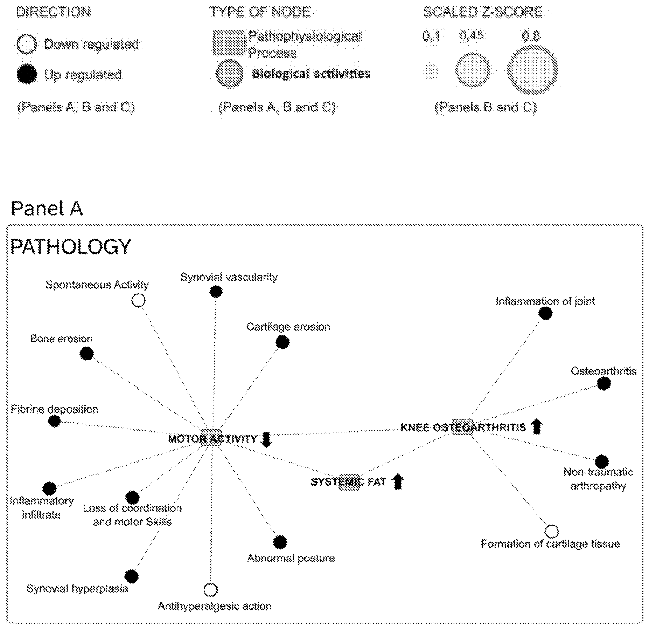

Figure 18 Graphic of aerobic biodegradation of the reference substance sodium benzoate during biodegradability test according to method OECD 310: 2014 (example 7).

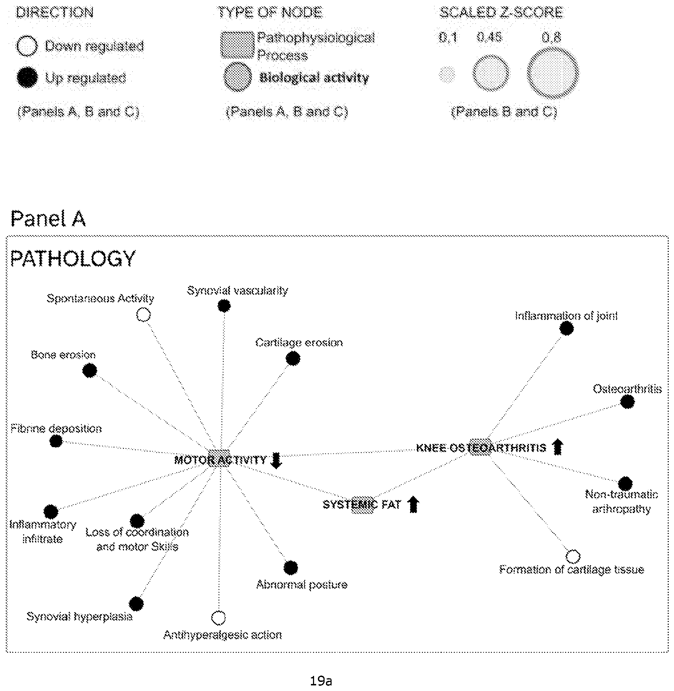

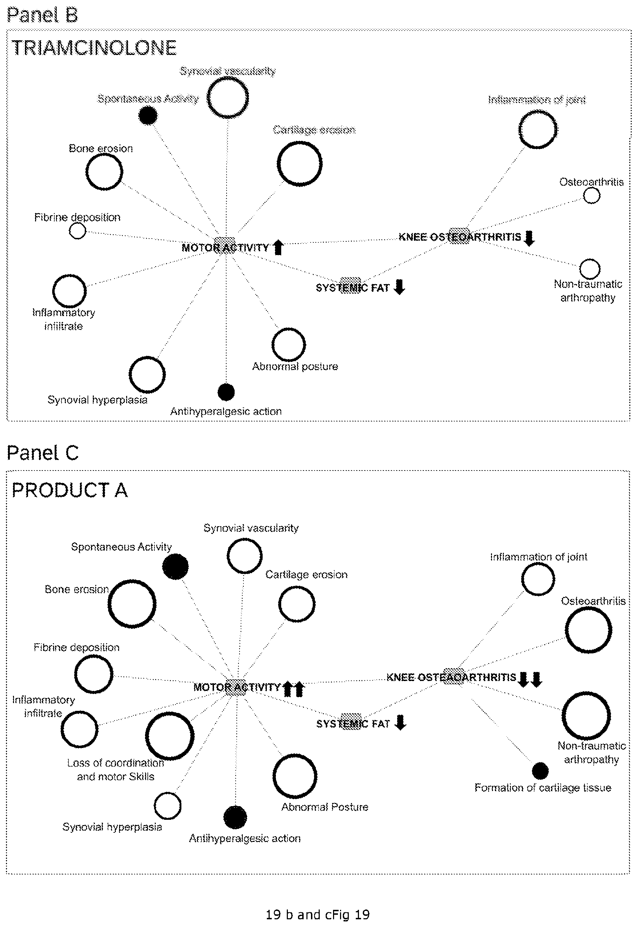

Figure 19 network analysis of osteoarthritis (19 Panel A) and treatment with reference drug (19 Panel B) vs. treatment with product A (19 Panel C) The grey squares represent both the fundamental nodes characterizing the pathophysiological or altered physiological state and the specific sites through which the pathology interconnects with the body. The arrows next to the nodes indicate the specific modulation for each situation described, and the intensity is shown through multiples of the arrows themselves. The grey squares are in turn linked to the network of biological activities, the modulation of which, has been demonstrated experimentally. These biological activities are represented by black (upregulation) or white (downmodulation) circles whose amplitude is directly proportional to the magnitude of their experimentally proved modulation. The network analysis shows that product A influences the body in a systemic way, modulating a higher number of desired activities, according to trend concurring to the healthy physiological state, than treatment with reference drug.

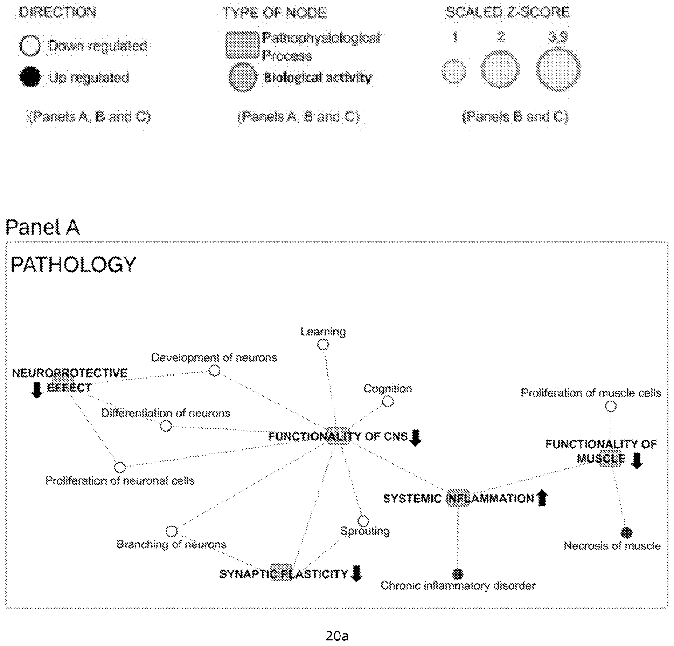

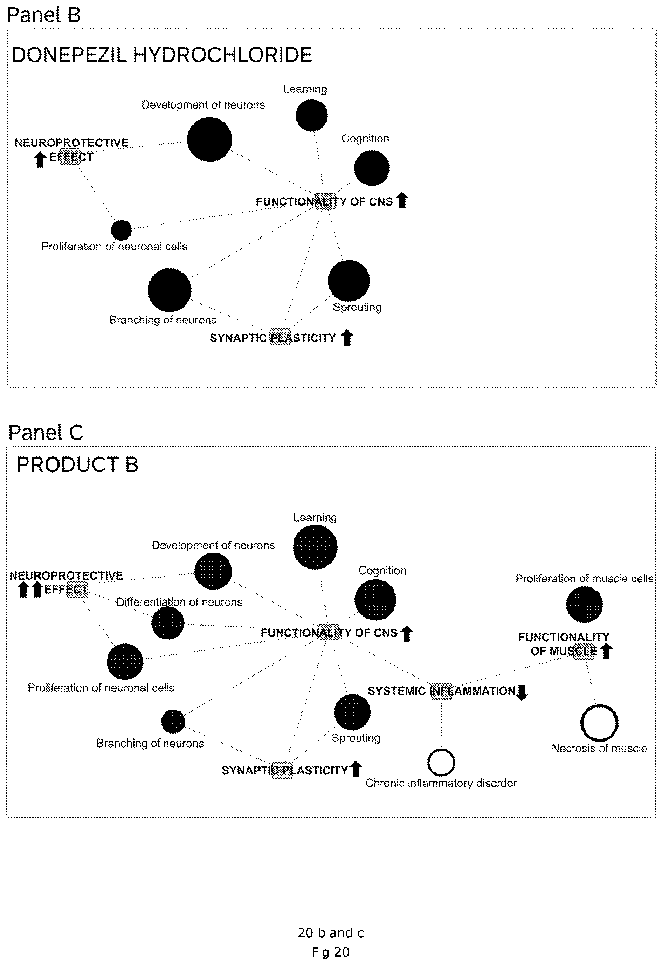

Figure 20 network analysis of mild cognitive impairment (20 Panel A) and treatment with reference drug (20 Panel B) vs. treatment with product B (20 Panel C). The grey squares represent both the fundamental nodes characterizing the pathophysiological or altered physiological state and the specific sites through which the pathology interconnects with the body. The arrows next to the nodes indicate the specific modulation for each situation described, and the intensity is shown through multiples of the arrows themselves. The grey squares are in turn linked to network of biological activities, the modulation of which, has been demonstrated experimentally. These biological activities are represented by black (upregulation) or white (downmodulation) circles whose amplitude is directly proportional to the magnitude of their experimentally proved modulation. The network analysis shows that product B influences the body in a systemic way, modulating a higher number of desired activities, according to modulation trend concurring to the healthy physiological state, than treatment with reference drug.

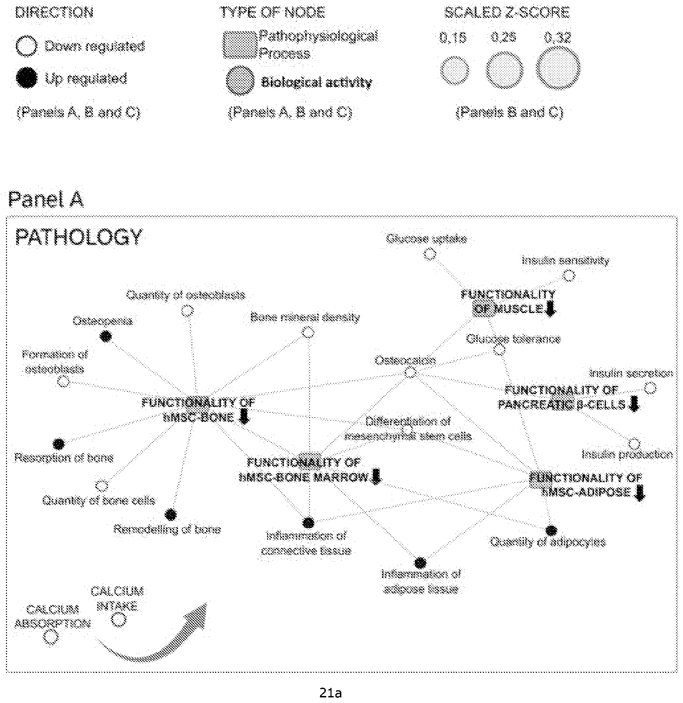

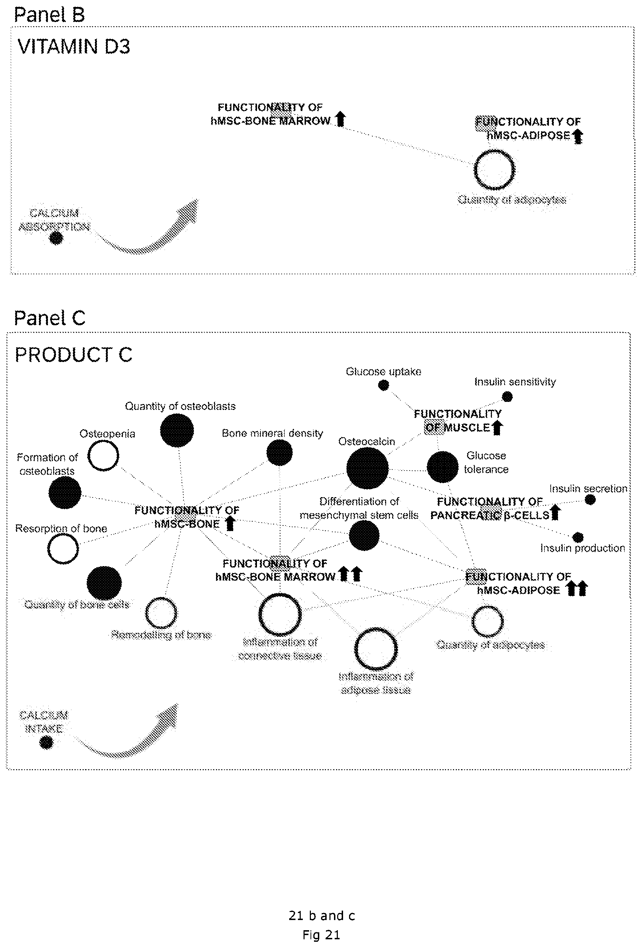

Figure 21 network analysis of osteoporosis (21 Panel A) and treatment with reference drug (21 Panel B) vs. treatment with product C (21 Panel C). The grey squares represent both the fundamental nodes characterizing the pathophysiological or altered physiological state and the specific sites through which the pathology interconnects with the body. The arrows next to the nodes indicate the specific modulation for each situation described, and the intensity is shown through multiples of the arrows themselves. The grey squares are in turn linked to the network of biological activities, the modulation of which, has been demonstrated experimentally. These biological activities are represented by black (upregulation) or white (downmodulation) circles whose amplitude is directly proportional to the magnitude of their experimentally proved modulation. The network analysis shows that product C influences the body in a systemic way, modulating a higher number of desired activities, according to the modulation trend concurring to the healthy physiological state, than treatment with reference drug.

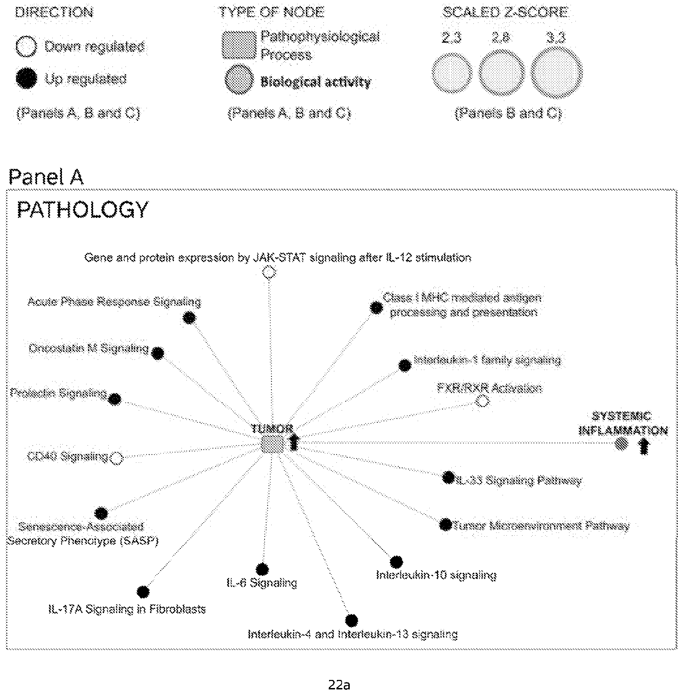

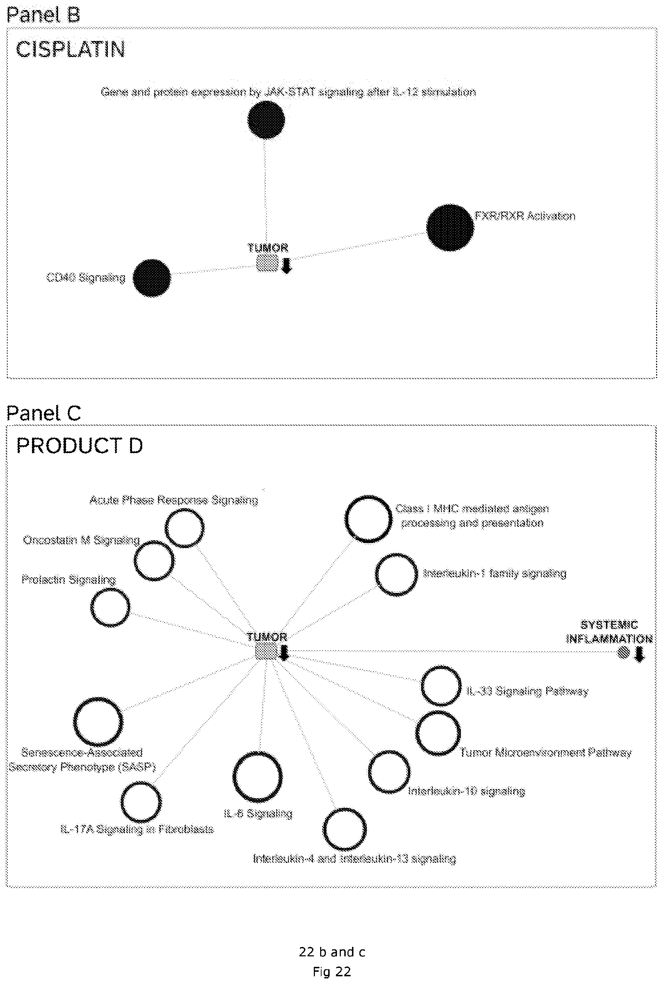

Figure 22 network analysis of squamous carcinoma (22 Panel A) and treatment with reference drug (22 Panel B) vs. treatment with product D (22 Panel C). The grey squares represent both the fundamental nodes characterizing the pathophysiological or altered physiological state and the specific sites through which the pathology interconnects with the body. The arrows next to the nodes indicate the specific modulation for each situation described, and the intensity is shown through multiples of the arrows themselves. The grey squares are in turn linked to a network of biological activities, the modulation of which, has been demonstrated experimentally. These biological activities are represented by black (upregulation) or white (downmodulation) circles whose amplitude is directly proportional to the magnitude of their experimentally proved modulation. The network analysis shows that product D influences the body in a systemic way, modulating a higher number of desired activities, according to the trend concurring to the healthy physiological state, than treatment with reference drug.

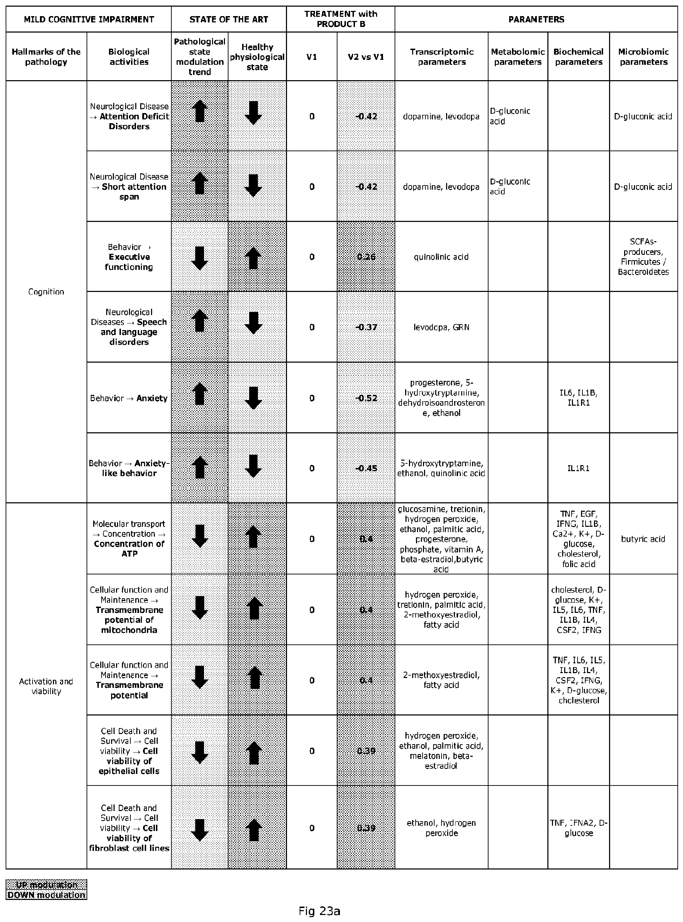

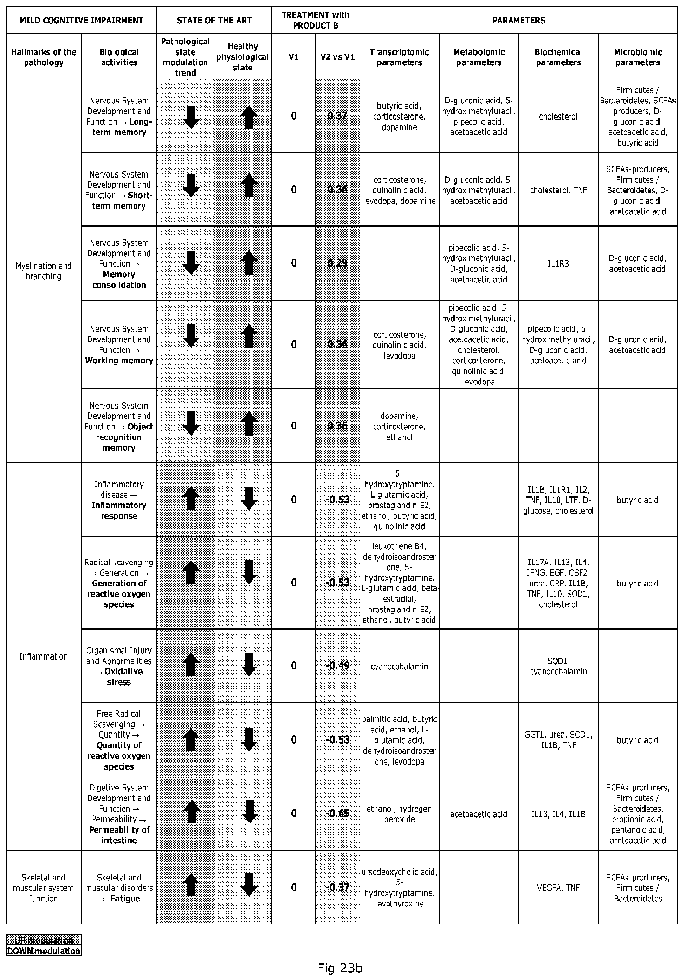

Figure 23 a and b Modulation of a network of selected biological activities in a clinical trial on mild cognitive impairment: column 1 hallmarks, column 2 network of biological activities, column 3 modulation trend of the network of biological activities in the pathological state, column 4 modulation trend of the network of biological activities concurring to a healthy physiological state, column 5 average modulation values of V1 samples representing the biological activities network at time 0 of the clinical trial, before administration of the product set as baseline with a value of 0, column 6 modulation induced by tested product (Product B) after 6 months of treatment average modulation value of V2 samples vs V1, columns 7-10 parameters selected. The results of the clinical trial shows that product sample modulates the network of all the selected activities according to the modulation trend concurring to the healthy physiological state profile. The numbers reported in each square represent the value quantifying the average modulation (z-score) calculated according to the examples representing the average modulation of each biological activity of the group V2 vs the group V1.

### GLOSSARY

A **"natural matrix"** in the present application refers to a material consisting of a network represented by a broad number of components/constituents obtained (e.g. extracted) directly from a member of the natural kingdom or a naturally occurring portion thereof (i.e., from a natural raw source), without significant processing or synthetic alteration, wherein "without significant processing or synthetic alteration" is intended that no denaturing processes are used for obtaining the matrix from the raw source. In other words, the natural raw source is processed only by manual, mechanical or gravitational means e.g. by dissolution in water or other naturally occurring solvents, such as water, water-alcohol solutions etc.; by flotation; by extraction with water or other naturally occurring solvents; by steam distillation or by heating solely to remove water or any other naturally occurring solvent; or extracted from air by any means and with the provision that "natural matrix" excludes said member of the natural kingdom or a naturally occurring portion thereof as such. In particular, according to the present description, a natural matrix is a 100% natural and biodegradable material, consisting of natural components that have not been denatured by the process for the production of the matrix from the starting raw materials without intentional addition of synthetic products along the whole process. In the present description, 100% biodegradable is considered as "readily biodegradable" according to an OECD biodegradability test. These features guarantee the maintenance of the matrix effect which is conferred to the matrix by the presence of structural interactions by its components (material interactions) and functional interactions that become evident upon exposure of a biological system to the natural matrix (immaterial interactions). In other words, a natural matrix, or a mixture of natural matrices, are materials obtained from entities that are self-assembled in nature and processed so to preserve their native bio-physical characteristics which determine their physiological interaction with other living organisms, such as the human organism. Their emerging properties can be expressed by contributing to the rebalancing of metabolic processes or states of the receiving organism and/or of some organs or tissues alongside the physiological actions that will be activated in each specific context. According to the present invention the natural matrix can be from a material obtained from any source in the life kingdoms i.e., Monera, Protista, Fungi, Plantae and Animalia. The term hence encompasses a plant natural matrix, an animal natural matrix, a fungi natural matrix, a protista (archaea or bacteria) natural matrix, a monera natural matrix. A natural matrix may also comprise natural inorganic materials such as minerals obtained from natural raw materials. A synonym of natural matrix or one or more natural matrices in the present description is "complex natural system" or "natural material" as defined below.

An example of naturally occurring portion of an organism may be represented by e.g., roots, leaves, bark, fruit, flower, of a plant or sections thereof, organs, tissues.

In any part of the description the general term natural matrix can be substituted with:
a plant natural matrix or a natural matrix obtained from a plant,
an animal natural matrix or a natural matrix obtained from an animal or from an animal product such as eggs or milk,
a fungi natural matrix or a natural matrix obtained from a fungus,
a protista natural matrix or a natural matrix obtained from a protista,
a monera natural matrix or a natural matrix obtained from a monera,
or with a plant material and/or extract, an extract from an animal tissue or organ, fungi and/or a fungi extract, or a mixture thereof wherein the extraction process does not encompass denaturing steps (e.g., temperature or the use of denaturing solvents). Plant is synonymous with herb.

The term "natural" matrix emphasizes the retaining the integrity and complexity of networks of constituents/components as in the original natural source due to the absence of denaturing treatments for the obtainment thereof. A natural matrix hence does not encompass compositions of natural origin that are enriched in specific molecules of artificial synthesis or isolated from a natural raw material. In addition, a natural matrix is obtainable only with processes that do not act through extensive processing or chemical modification, isolation, purification, or molecular extraction.

Due to the supramolecular self-assembly of the constituents/components of a natural matrix and the presence of functional interactions among them, the whole matrix behaves as a complex network that does not interact with a single target molecule but that interacts with a network of recipients (also organised as a network) in the receiving organism. Therefore, the interaction natural matrix-receiving organism is not, as for common pharmaceutical APIs the result of a point-to-point interaction, but the result of an "interactor" networks (i.e., the matrix)-"receiver" network (i.e., the organism to whom the matrix is administered) interaction.

The term natural matrix can be also substituted in any part of the description and claims with complex natural system.

Nowhere in the description and in the claims the term natural matrix can be interpreted as "a product of nature" as such, rather, a natural matrix is a product obtained from a natural organism and processed (e.g. extracted) therefrom by techniques that do not substantially alter biological structure and the relevant supramolecular and functional interconnections among the components within the matrix as mentioned above, i.e., without the use of denaturing techniques and that does not comprise additional isolated or synthesised molecules or classes of molecules.

**Emerging properties** according to the present description and to the art, the term defines the properties of a natural matrix or of a natural material according to the present specification, i.e., properties that are not represented by the mere sum of properties of each singled out constituent/component of said matrix/material but by the both functional and structural interactions among all constituents/components of the matrix/material that are also the result of the supramolecular self-assembly of said components/constituents within the matrix/material itself.

"Emerging properties" hence refer to technical effects, such as therapeutic or homeostasis - adjuvating properties (i.e., beneficial effect), that the interactions and relationships among the constituents/components of a natural matrix exert on a receiving living system. By definition, emergent properties are properties that are not immediately evident or even predictable based solely on the individual characteristics of each constituent/component of the matrix. Instead, they "emerge" when all the constituents/components of the matrix networks interact with one another and with the living system receiving network in a dynamic and complex way. Emerging properties have been broadly discussed in the art in various scientific and systems-oriented fields, including physics, chemistry, biology, and complex systems theory.

Emerging properties are hence properties that cannot be predicted *a priori* by the quali-quantitative knowledge of each component of a given composition or matrix and that, consequently, cannot be ascribed to one or more specific API. Hence, although multidrug compositions can show unpredicted synergic effects, the properties of said compositions are still ascribed to the specific APIs and quantities thereof contained therein.

In the case of emerging properties, characteristic of natural matrices, the observed emerging properties cannot be reconducted to specific APIs and are maintained in different batches of a given matrix or a given mixture of matrices notwithstanding the different quali-quantitative composition of said batches (functional resilience see below).

### Key points about emerging properties include:

System Complexity: Emerging properties are associated with systems that exhibit a certain level of complexity. In simple systems, the interactions between constituents/components are limited, and properties are more easily deducible from the properties of individual constituents. In complex systems, however, the interactions between components and their supramolecular organisation can give rise to novel and unexpected features.

Nonlinearity: Emergent properties often result from nonlinear interactions, where the relationship between cause and effect is not proportional.

Holism: The concept of emerging properties emphasizes a holistic perspective, recognizing that the whole system is more than the sum of its parts.

A **product comprising or a product consisting of one or more natural matrices** (alias "a product comprising, or consisting of, one or more complex natural system/s") according to the present invention is a product that comprises, or consists of, one or more natural matrices, and is herein also defined as a "natural material". In particular, according to the present description, a product comprising or consisting of one or more natural matrices is a product which is 100% natural, which means that the product does not contain substances that are of chemical synthesis, i.e., made by man.

In addition, according to the present description, a product comprising one or more natural matrix, does not contain any added isolated molecule, e.g., excipient/s or active principle/s even if of natural origin. A product consisting of one or more natural matrices according to the present invention is a product consisting of a defined mixture of natural matrices, e.g., n natural matrices, wherein n is an integer number from 1 to 100, preferably from 1 to 50, preferably from 1 to 20, 1 to 10, 1 to 7, 1 to 6, 1 to 5 each natural matrix being specifically defined.

In any part of the description and the claims "a product comprising, or consisting of, one or more natural matrices" can be replaced by "a product comprising, or consisting of, one or more plant matrix" or by "a product comprising, or consisting of, complex natural system/s" "a natural material or a material of natural origin".

Furthermore, the term "product comprising, or consisting of, one or more natural matrices" according to the present description can be an intermediate, or the final formulation for intended use (e.g. resuspended dry product) or, in particular when the formulation for intended use is in liquid form, the term can define a dry or a lyophilised or a concentrated form thereof to which water will be added by the user or by the physician to prepare the formulation for administration.

Nowhere in the description and in the claims "a product comprising, or consisting of, one or more natural matrices" can be intended as a product of nature *as such.* When a mixture of natural matrices is comprised in, or consists of, the product, said mixture is a mixture of selected natural matrices made by man, and said mixture cannot be found *as such* in any of the natural products of origin of each matrix contained therein. Therefore, when the product comprises, or consists of, a plurality of natural matrices, said natural matrices have been combined by man and the resulting product is endowed of new emergent properties.

In the present description and in the claims the term natural material is a synonym for "product consisting of one a natural matrix or of a mixture of natural matrices (more natural matrices)".

According to the present description, the expression **"one or more biological activities related to a pathological condition"** refers to one or more biological activities, in particular to a network of biological activities, associated to a distancing/deviation from homeostasis which may reach the onset, progression, worsening, of a pathological condition. Hence, the expression "one or more biological activities modifications (or modifications of a network of biological activities) related to a pathological condition" refers to modulations or changes in the normal (healthy) physiological state of one or more said biological activities (or processes) within an organism (preferably a human) that are directly associated to an alteration/impairment of homeostasis and can end up into or are observable in a pathological state or disease. In other words, it describes the specific adjustments or deviations from the healthy physiological state of one or more biological activities that occur as a result or that concur to the onset, progression, worsening, of a pathological condition or disease. By way of example, in the case of diabetes, one or more biological activities related to glucose metabolism, insulin production and management are modified in ways that are directly associated to the pathological condition of diabetes and are hence related to the pathological condition or state of diabetes according to the present description.

**Synthetic** according to the present description has the meaning conventionally accepted in chemistry.

Conventionally, in chemistry, the term "synthetic" refers to the origin or source of a material or substance. Synthetic substances or materials are produced by man through artificial synthesis i.e., through laboratory chemical reactions usually by reacting simpler chemicals to create more complex ones through processes that often use different pathways, temperature conditions, pressure conditions, energy sources and/or catalysers from those used by living organisms.

Examples: Synthetic substances or materials include plastics, pharmaceutical drugs, and many industrial chemicals. For example, nylon is a synthetic polymer made through chemical synthesis, and aspirin is a synthetic drug produced through specific chemical reactions.

**Functional resilience** according to the present description is intended as a therapeutic or beneficial (homeostasis adjuvant) resilience of a therapeutic or beneficial product comprising or consisting of one or more natural matrices; the term describes the maintenance of the therapeutic or beneficial properties of different batches of a given product comprising (or consisting of one or more natural matrices) notwithstanding the different batch to batch qualitative and quantitative composition, which is necessarily present (inherent) in products comprising or consisting of one or more natural matrices. As known by the skilled person, each time a different batch of starting raw material is used, the resulting natural matrix has a unique quali-quantitative composition at the molecular level which is typical of the individual diversity between living organisms also of the same species.

An ***in vitro* cell-based assay,** in the present description has the meaning conventionally used in the art, in particular, it refers to an analytical procedure based on cells, for evaluating the cell behaviour and reaction to insults or stimuli, in the context of a disease or of a pathological or pre-pathological condition or of a condition wherein homeostasis is altered. This type of assay is designed to study the biological response of cells in a controlled environment, often in a petri dish or a well plate. According to the invention, suitable *in vitro* cell-based assays are assays whose read out is associated with the modulation of one or more biological activities, or a network of biological activities, associated to one or more hallmark of a given pathological or pre-pathological condition or of a condition wherein homeostasis is altered (altered physiological state). The cells can be derived from humans, animals, plants, or in general cell lines that mimic specific tissues or organs or are in general cells that are known models for mimicking a disease.

In the context of a disease or pathological/pre-pathological condition, or of a condition wherein homeostasis is altered, a cell-based assay is specifically designed to simulate or mimic conditions related to the disease or pathological/pre-pathological condition or to the condition wherein homeostasis is altered. In particular, cell-based assays can be used to evaluate *in vitro* the therapeutic, adjuvating and/or beneficial action of different compounds or products. It can involve exposing cells to factors known to be associated with the disease or pathological condition, to potential therapeutic or adjuvating products adjuvating the restoration of the physiological state or using cells that are or have been genetically modified to carry disease or pathological condition-specific traits. The selected cells can be treated to induce the pathological, pre-pathological or altered homeostasis condition or can be cells already presenting the desired altered phenotype.

A **healthy physiological state** refers to the condition of an organism's body, organ, apparatus, system or body district, and its internal processes when they are functioning optimally and within normal parameters for that individual, i.e., the state to which homeostasis tends. A healthy physiological state, in the context of one or more biological activities known to contribute to hallmarks of a given disease or pathological condition or of an altered physiological state, refers to the state in which said one or more biological activities are operating optimally and within normal (healthy) parameters. This state is characterized by the absence of significant aberrant cellular or molecular processes associated with the specific disease under consideration. When the modification trend of one or more biological activities which concurs to a pathological, pre-pathological condition is known, the healthy physiological state can be considered represented by the opposite modification trend for each of said activities.

The term considers the hallmarks of a particular disease, which are distinctive features or characteristics that are typically observed in individuals affected by that disease. These hallmarks can include specific cellular behaviours, molecular pathways, canonical pathways, or physiological responses that play a key role in the development or progression of the disease.

In summary, a healthy physiological state in the context of a specific disease or pathological/altered condition is a state in which the one or more biological activities related to the known hallmarks of that disease or pathological condition are modulated in a direction that is consistent with a non-pathological/non-altered state, in other words, opposed to the pathological/altered state.

A healthy physiological state according to the invention, therefore, also indicates the direction of the modulation of one or more biological activities that are known hallmarks of a pathological condition in homeostasis, i.e., before the onset of a pathological condition, in other words the homeostatic direction of the modulation of one or more biological activities ascribed to a specific system, district, apparatus or organ of a healthy subject.

**Altered physiological states** and **altered homeostasis** are closely related concepts that describe deviations from the normal functioning and balance of the body's internal environment. While they overlap, there are some distinctions between the two terms: **Altered Physiological States:** This term encompasses a broad range of changes in the body's normal functioning, including disruptions in organ systems, biochemical processes, and cellular functions. Altered physiological states can result from various factors such as disease, injury, medication, environmental factors, and psychological stress. Examples include fever, inflammation, hormonal imbalances, and impaired organ function.

**Altered Homeostasis:** Homeostasis refers to the body's ability to maintain a stable internal environment despite external changes. This stability is achieved through regulatory mechanisms that control variables such as body temperature, blood pressure, pH balance, and blood glucose levels within narrow ranges. Altered homeostasis occurs when these regulatory mechanisms fail to maintain balance, leading to deviations from the body's normal set points. These deviations can be temporary or chronic and may involve compensatory mechanisms to restore balance.

In summary, altered physiological states describe the observable changes in the body's normal functioning, while altered homeostasis refers to the underlying disruption of the body's regulatory mechanisms that maintain internal stability.

An altered homeostasis underlies altered physiological states, as disruptions in homeostatic mechanisms that can lead to physiological imbalances and manifestations of illness or dysfunction.

**Hallmark of a disease or of a pathological or medical condition** according to the present description has the meaning conventionally used in the art. Hallmarks of a disease are known to be indicators that can mark the progression or control of a given disease or pathological or pre-pathological condition and taken together are usually representative of the general pathological state associated to a given pathology. These hallmarks (also called 'key indicators') are typically a set of features or patterns that a physician would monitor, over time, to track the onset, the progression or regression of a particular illness. In summary, a hallmark of a disease is a defining feature or characteristic whose modification is indicative of a given pre-medical or medical condition, aiding in its identification, diagnosis, monitoring and understanding. By way of example, for neurodegenerative diseases (NDDs) at least the following eight hallmarks of NDD are known in the art: (pathological protein) aggregation, synaptic and neuronal network (dysfunction), (aberrant) proteostasis, cytoskeleton (abnormalities), (altered) energy homeostasis, DNA and RNA (defects), inflammation (increase), and neuronal cell death (increase). In cancer research, the hallmarks of cancer are a set of distinctive characteristics that are commonly found in cancer cells. These hallmarks include (sustained) proliferative signalling, (evasion of) growth suppressors, (resistance to) cell death, (enabling) replicative immortality, (inducing) angiogenesis, and (activating) invasion and metastasis.

Hallmarks of a disease, parameters related to said hallmarks (e.g., biomarkers), one or more biological activities associated to said hallmarks etc. are a framework to study a disease or a pathological or medical condition using an integrated/ holistic approach. The **hallmarks of an altered physiological** state typically include observable changes in various aspects of the body's functioning, which may manifest through symptoms, signs, or laboratory findings.

Altered physiological states typically reflect disruptions in the body's homeostatic mechanisms, leading to deviations from normal physiological parameters. These imbalances may involve alterations in temperature regulation, fluid and electrolyte balance, acid-base balance, glucose metabolism, or other regulatory processes. Overall, the hallmarks of an altered physiological state provide valuable clues for healthcare providers to identify the underlying cause, assess severity, and guide appropriate interventions to restore normal functioning and promote recovery.

**"Having Therapeutic effect":** according to the present description a product having a therapeutic effect is a product which, upon administration to a subject affected by a pathological condition, reduces the severity of the subject's condition (i.e., the severity is at least partially reduced or mitigated), and/or provides some alleviation, mitigation or decrease in at least one clinical symptom and/or in a delay in the progression of said condition or reinstates (completely or in part) a healthy physiological condition in the district affected by said pathological condition.

Having beneficial effect according to the present description encompasses a product whose administration to a healthy subject or a subject which is healthy but not in homeostasis, or to an in vitro cell assay representing an adequately healthy status, results in an *in vitro* or *in vivo* evidence of a reinstatement or adjuvating homeostasis upon administration in said cell assay or in the recipient's system/district/apparatus/organ of interest.

The terms **"prevent," "preventing,"** and **"prevention of"** (and grammatical variations thereof) refer to reduction and/or delay of the onset and/or progression of a disease, disorder and/or a clinical symptom(s) in a subject and/or a reduction in the severity of the onset and/or progression of the disease, disorder and/or clinical symptom(s) relative to what would occur in the absence of the methods of the invention. The prevention can be complete, e.g., the total absence of the disease, disorder and/or clinical symptom(s). The prevention can also be partial, such that the occurrence of the disease, disorder and/or clinical symptom(s) in the subject and/or the severity of onset and/or the progression is less than what would occur in the absence of a composition according to the present invention.

Having a "beneficial/healthy/beneficial to health" effect according to the present description encompasses a product whose administration to a healthy subject or to a healthy subject who is not in homeostasis or to an in vitro cell assay representing an adequately healthy status, results in an *in vitro* or *in vivo* evidence of a reinstatement or adjuvating homeostasis upon administration in said cell assay or in the recipient's system/district/apparatus/organ of interest.

According to the present description the term **homeostasis** has the meaning conventionally accepted in the art, and refers therefore, to the physiological process by which living organisms maintain a stable internal environment despite external changes. This stability is crucial for the proper functioning of cells, tissues, and organs. The goal of homeostasis is to ensure that the internal conditions of an organism remain within optimal ranges for survival and proper physiological functioning (healthy physiological state). Homeostasis is obtained by organisms by the modulation of one or more biological activities, preferably a network of biological activities, and processes aimed to maintain a healthy physiological state.

A product adjuvating homeostasis is a product that modulates one or more biological activities, preferably a network of biological activities, in the direction of a healthy physiological state, hence, a product that is suitable for a healthy individual and that supports the homeostatic mechanisms that contribute to the healthy physiological state and that can be used by a healthy individual to adjuvate the homeostatic regulations of one or more biological activities, preferably a network of biological activities, associated to hallmarks of a given pathological condition.

**Medical device,** also MD, according to the present description is a product as defined above, according to the definition in EU Regulation 2017/745 Article 2 (1) indents 1-3, necessarily used for therapeutic purposes hence, 'medical device' means any ... [omissis] ... material intended by the manufacturer to be used, alone or in combination, for human beings for one or more of the following specific medical purposes:
-treatment or alleviation of disease,
-treatment, alleviation of, or compensation for, an injury or disability,
-modification of a physiological or pathological process or state,
... omissis...
and which does not achieve its principal intended action by pharmacological, immunological or metabolic means, in or on the human body, but which may be assisted in its function by such means.

'Performance' of a medical device means the ability of a medical device as herein defined to achieve its intended purpose as stated by the manufacturer.

'Clinical performance' of a medical device means the ability of a medical device as herein defined, resulting from any direct or indirect medical effects which stem from its technical or functional characteristics, including diagnostic characteristics, to achieve its intended purpose as claimed by the manufacturer, thereby leading to a clinical benefit for patients, when used as intended by the manufacturer.

'Clinical benefit' of the medical device means the positive impact of a device as herein defined on the health of an individual, expressed in terms of a meaningful, measurable, patient-relevant clinical outcome(s), including outcome(s) related to diagnosis, or a positive impact on patient management or public health.

Also, if a composition, (e.g. a contrivance, such as product having therapeutic properties comprising one or more natural matrices), is intended to have a medical purpose, such as diagnosis, treatment, mitigation, or prevention of a disease or to affect the structure or function of the body, and it meets the criteria outlined in the definition, it may be classified as a medical device by the US FDA.

Per Section 201(h)(1) of the Food, Drug, and Cosmetic Act, a device is:
An instrument, apparatus, implement, machine, contrivance, implant, in vitro reagent, or other similar or related article, including a component part, or accessory which is:
(A) recognized in the official National Formulary, or the United States Pharmacopoeia, or any supplement to them,
(B) intended for use in the diagnosis of disease or other conditions, or in the cure, mitigation, treatment, or prevention of disease, in man or other animals, or
(C) intended to affect the structure or any function of the body of man or other animals, and which does not achieve its primary intended purposes through chemical action within or on the body of man or other animals and which is not dependent upon being metabolized for the achievement of its primary intended purposes. The term "device" does not include software functions excluded pursuant to section 520(o). The classification of medical devices within a risk class is typically based on factors such as intended use, indications for use, and risk associated with the device.

**A pathophysiological or physio pathological state** refers to an abnormal physiological condition or process within the body that is typically associated with disease or dysfunction. In the present description and claims it is also defined "pathological state". It involves the study of the functional changes that occur as a result of disease or injury and how these alterations manifest at the cellular, tissue, organ, and systemic levels. Pathophysiology encompasses the understanding of both the underlying mechanisms of disease and the body's response to these disruptions to diagnose, treat, and manage various health conditions. Understanding pathophysiological states is crucial in medicine for both research and clinical practice.

Pathophysiology involves the study of how various factors, such as genetic abnormalities, environmental influences, or disease processes, disrupt the normal physiological functions of the body, leading to the development of health disorders or diseases. Understanding physio pathological states is essential for diagnosing, treating, and managing a wide range of medical conditions across different specialties in healthcare.

**Different batches or lots of a product** in the present description as in the state of the art, refer to distinct groups of items produced or manufactured at different times or under different conditions, but still belonging to the same product line. In different batches, the starting raw materials can be from the same stocks or from different stocks. Each batch or lot typically receives a unique identifier which distinguishes it from other batches. These identifiers help in quality control, inventory management, and traceability throughout the production process and supply chain.

Batches of products comprising one or more natural matrices or consisting of one or more natural matrices are expected to vary in their qualitative and quantitative chemical composition due to factors like raw material variations. In addition, the batches can vary as any other product batch, due to production conditions, or equipment used.

### DETAILED DESCRIPTION OF THE INVENTION

The present invention provides methods, as defined in the appended claims, for determining whether a therapeutic or beneficial product, said product comprising or consisting of one or more natural matrices, exerts its therapeutic or beneficial effect through a physiological mechanism of action.

To act with a physiological mechanism of action, a product must be 100% natural. Therefore, according to the present description, a product comprising or consisting of one or more natural matrices is a product which is 100% natural, which means that the product does not contain additional artificial substances, i.e., substances of chemical synthesis made by man through laboratory processes.

In addition, according to the present description, a product comprising one or more natural matrix, does also not contain any added isolated molecule, e.g., excipient/s or active principle/s even if of natural origin.

The present description discloses in the examples experimental data on a Products A, B, C and D (see examples for the composition of the products), respectively for the treatment of osteoarthritis, mild cognitive impairment, osteoporosis, and cancer (product A, herein also indicated as Arté GX, is also disclosed in WO 2018/138678).

All the tested products are for use in the treatment or in adjuvating the treatment of a pathological or altered condition, the matrices therein being obtained from natural material suitably processed without denaturing steps and formulated so to obtain a final natural material (i.e., product) which is able to modify, upon administration, a pathological state or altered physiological state and to promote the restoration of a healthy physiological state. In accordance with the definition of natural matrices provided in the glossary, all the tested products were prepared through processes that did not denature the components within the natural matrices therein, said matrices consisting only of 100% natural and biodegradable components. A detailed description of the tested products is provided in the examples section.

In the tested products, the hundreds of components coming from the coarse raw plant parts have not been deprived of the ability, characteristic of materials of natural, biological origin, to establish multiple combinations of molecular and supramolecular interactions among themselves and with the target tissues and therefore maintain therapeutic emerging properties.

In particular, the products tested by the Applicant have been prepared, starting from the preparation of the soil to the production of the final product, following eubiotic protocols that have been selected and standardised by Applicant throughout more than 40 years of experience to render, *a priori,* the final products as homogeneous as possible and therefore increase the efficiency in terms of yield of valid batches. The eubiotic protocol developed by the applicant, has been construed to standardise as much as possible each step leading to the desired final product respecting the eubiotics of each step, so to provide a final product which is 100% natural as opposed to a synthetic or partially synthetic product (i.e., a product that does not comprise a single component obtained by artificial chemical synthesis) wherein each component is produced under eubiotic conditions. It is possible to develop a fully eubiotic, standardised, production process with the integration of inter-sectoral technologies and research in several different fields, from the agricultural one to -omics and mathematical sciences ones by comparing, or rather integrating, the reductionist approach discussed above with that of systems theory and quantum biology.

As stated above, the present inventors have surprisingly found that products comprising one or more natural matrices, maintain their therapeutic or beneficial effects notwithstanding a different qualitative and quantitative composition (due to their natural origin) showing a functional resilience wherein the emergent properties of the matrices are maintained. I.e., contrary to traditional paradigms, the quali-quantitative variability in the matrices composition does not affect the final matrix effects and that the matrices maintain at least part of the autopoietic properties of the systems from which they derive. The present inventors also found that this kind of products, due to the networks-to-network interactions between the matrices and the subject treated, exert their therapeutic or beneficial action by modifying a state (pathological, pre-pathological or of altered homeostasis) rather than a single or few functions. In particular, the functional resilience observed by the inventors is evident in terms of different regulation of different genes (due to the quali-quantitative variability of the natural matrices in the products analysed), which nevertheless results in the same modulation effect on the examined activities (see examples).

Together, these characteristics, are indicative of a physiological mechanism of action as opposed to the well-known key-lock pharmacological mechanism of action. The present invention, thanks to the identification of the key characteristics of a physiological mechanism of action and to the provision of methods for verifying the presence or absence of said characteristics, provides for the first time a method for determining whether a product, therapeutic or beneficial, acts through a physiological mechanism of action.

This is crucial to allow manufacturers to assess and demonstrate compliance with the new requirements set forth in the regulations for medical devices that at present are not available.

A physiological activity is an activity that can be exerted by a natural and biodegradable material, such as natural matrices, which consists only of natural components that were not degraded in the matrices production process and therefore possess a matrix effect conferred both by structural (material) interactions among the components of the matrix and functional, including immaterial, interactions that become evident only when the matrix interact with a biological system. Said interactions do not appear attributable to previous experimental models based on the existence of a direct and unambiguous relation at the molecular level between structure and activity of selected components. Natural materials, comprising or consisting of natural matrices, are entities which maintain at least in part the autopoietic properties of their starting materials which belong to the living domain, and display own properties which are represented by networks of material and immaterial relationships, that interact with the network of relationships of the treated subject (networks-to-network interactions) thereby recapitulating an interaction with features and complexities that are physiological-like.

Herein provided are means for verifying the presence of said properties and therefore for assessing whether a product (therapeutical or beneficial) exerts its therapeutic or beneficial effect through a physiological mechanism of action.

The present invention hence provides:
A method for measuring different batches of a therapeutic product comprising one or more natural matrices, to determine that said product exerts its therapeutic effect through a physiological mechanism of action, wherein said physiological mechanism of action is a non-pharmacological, -immunological, -metabolic (Ph.IM) mode of action, and is characterized by the regulation of a network of biological activities that underly hallmarks defining a pathological state, and by the therapeutic functional resilience of said product among said different batches the method comprising:
a) selecting a therapeutic product comprising one or more natural matrices and providing different batches of said product;
b) providing a list of hallmarks representative of said pathological state;
c) identifying for each of said hallmarks one or more biological activities modifications underlying said pathological state thereby pinpointing a network of biological activities whose modulation concurs to said pathological state;
d) identifying one or more gene expression patterns whose modulation concurs to the modulation of said one or more biological activities underlying the therapeutic effect of the product tested and determining the modulation trend in terms of up or down modulation of said one or more biological activities, in said network, concurring to said pathological state or to a healthy state
e) performing, on each of said different batches, at least one *in vitro* cell-based assay wherein the read out of said cell-based assay is representative of the modulation of one or more biological activities underlying the desired therapeutic effect, on the following groups of cells
(e1) at least one control group and at least two test groups of cells having the diseased phenotype relevant to the intended use of the therapeutic product; or
(e2) at least one group of cells with a healthy physiological phenotype; and at least one control group and at least two test groups of said cells with a healthy physiological phenotype wherein the diseased phenotype relevant to the intended use of the therapeutic product is induced,
and treating each of said test groups of cells with one of said different batches of therapeutic product;
   (f) determining the modulation or modulation pattern of each of said gene expression patterns on each of said groups of cells of step (e) and calculating the respective modulation values for each of said one or more biological activities;
   (g) said therapeutic product is shown to exert its therapeutic effect through said physiological mechanism when, comparing said modulation values, the conditions (g1) and (g2) are met:
(g1) at least 50% of said one or more biological activities for each hallmark are modulated by each product batch with the modulation trend of the network concurring to the healthy state, and the modulation values determined in (f) of each of said at least 50% one or more biological activities for said test groups of cells of (e1) differ, respectively, from the ones of said control group of cells of (e1) of at least 0.15, or
at least 50% of said one or more biological activities for each hallmark are modulated by each product batch with the modulation trend of the network concurring to the healthy state, and the modulation values determined in (f) of each of said at least 50% one or more biological activities for said test groups of cells of (e2) differ, respectively, from the ones of said control group of cells of (e2) by at least 15%, and
(g2) functional resilience of the product is demonstrated by the modulation values for each one or more biological activities of each said test groups of cells differing by less than 20% from the average of said values;
wherein gene expression is assessed by transcriptomic analysis; and

A method for measuring different batches of a beneficial product adjuvating homeostasis in an altered physiological state the product comprising one or more natural matrices, to determine that said product exerts its beneficial effect through a physiological mechanism of action wherein said physiological mechanism of action is a non-pharmacological, -immunological, -metabolic (Ph.IM) mode of action, and is characterized by the regulation of a network of biological activities that underly hallmarks defining a pathological state, and by the beneficial functional resilience of said product among said different batches the method comprising:
a) selecting a beneficial product comprising one or more natural matrices and providing different batches of said product;
b) providing a list of hallmarks representative of a pathological state that can stem from said altered physiological state;
c) identifying for each of said hallmarks one or more biological activities modifications underlying said pathological state thereby pinpointing a network of biological activities whose modulation concurs to said pathological state;
d) identifying one or more gene expression patterns whose modulation concurs to the modulation of said one or more biological activities underlying the beneficial effect of the product tested and determining the modulation trend in terms of up or down modulation of said one or more biological activities, in said network, concurring to said pathological state or to a healthy state
e) performing, on each of said different batches, at least one *in vitro* cell-based assay wherein the read out of said cell-based assay is representative of the modulation of one or more biological activities underlying the desired beneficial effect, on the following groups of cells:
   at least one control group of cells having healthy phenotype or at least one control group of cells wherein the aberrantly modulated phenotype targeted by said beneficial product is suitably induced, and at least two test groups of cells taken from said control group
   and treating, each of said test groups of cells, with one of said different batches of beneficial product;
(f) determining the modulation or modulation pattern of each of said gene expression patterns on each of said groups of cells of step (e) and calculating the respective modulation values for each of said one or more biological activities;
(g) said therapeutic product is shown to exert its beneficial effect through said physiological mechanism when comparing said modulation values the conditions (g1) and (g2) are met:
   g1) at least 50% of said one or more biological activities for each hallmark are modulated by each product batch with the modulation trend of the network concurring to the healthy state and the modulation values determined in (f) of each of said at least 50% one or more biological activities for said test groups of cells differ, respectively, from the ones of said control group of cells of at least 0.15 and
   g2) functional resilience of the product is demonstrated by the modulation values for each one or more biological activities of each said test groups of cells differing by less than 20% from the average of said values;
   wherein gene expression is assessed by transcriptomic analysis.

In the present description as well as in the claims, functional resilience is intended as the maintenance of the therapeutic or beneficial properties of different batches of a given product comprising one or more natural matrices, notwithstanding the different batch to batch qualitative and quantitative composition of said product. As already explained, in products comprising one or more natural matrices, the different batch to batch qualitative and quantitative composition is an inherent feature of natural matrices themselves, therefore, a product comprising or consisting of one or more natural matrices will necessarily have a different batch to batch composition due to the impossibility of obtaining two identical (in terms of quali-quantitative composition) natural matrices starting from the same kind of raw material.

According to the present invention, the essential features for a product, to exert a therapeutic or beneficial action through a physiological mechanism of action, is the presence in said product, of one or more natural matrices and the assessment of the maintenance of at least two features that are typical of the living kingdom, i.e., the conservation of the functional resilience of the product, wherein permutations of the molecular composition still mediate the same biological activity, and the modification on a physiological state (pathological or altered but not yet pathological). The maintenance in time of the modification of a physiological state being representative of the modification of a physiological process.

As stated already, in the present description and in the claims, a batch of a product refers to a quantity of a product produced at one time, under given conditions. With products comprising natural matrices, different batches necessarily will vary from each other in their quali-quantitative composition since they are produced by different sets of raw materials. It is implicit that the method of the invention is carried out on product batches whose therapeutic or beneficial effectiveness has been validated by the manufacturer and are hence produced prepared following the same manufacturing protocol and conditions from the same kind of starting raw materials.

Hence, the selected therapeutic or beneficial product is a product, comprising or consisting of one or more natural matrices, for use in the treatment of a pathological condition or, alternatively, for adjuvating homeostasis when an altered physiological state exists in a healthy subject. Therefore, the therapeutic product, when administered to a patient suffering of a given pathological condition, reduces the severity of the subject's condition (i.e., the severity is at least partially improved or ameliorated), and/or provides some alleviation, mitigation or decrease in at least one clinical symptom of said condition and/or delays in the progression of the said condition; whereas the beneficial product, when administered to a healthy subject with an altered physiological condition, adjuvates the reinstatement of the correct homeostatic response, helping the organism to restore the normal (non-altered) physiological condition.

The subject that can be treated, according to the invention, is an animal including humans (hence the product is for human or for veterinary use), or even a plant.

A non-limiting example of natural matrices according to the invention is represented by one or more of: cut or pulverized plant parts, plant extracts, processed plant parts, fractions of plant extracts such as, for example, the fractions obtained by filtration on a semi-permeable membrane (microfiltration, ultrafiltration, nanofiltration) or by treatment on adsorption resins, microorganisms, honey, propolis, silk, wax, plant resins, plant gums, plant exudates, vegetable oils, vegetable essential oils, animal tissues lysates, plant or animal fluids.

Preferably, said microorganisms are inactivated microorganisms such as tyndallized organisms.

In a mostly preferred embodiment, the product provided (selected) in the method of the invention, is a product consisting of 100% of natural components, intended as components that are not obtained by man through chemical synthesis reactions, therefore, when the product comprises one or more natural matrices, it can also comprise minerals, and in general any other organic or inorganic material found in nature. Preferably, the product subject to methods and processes of the invention, is a product obtained or obtainable according to a standardised protocol, more preferably through an eubiotic standardised protocol.

According to the invention said product can be a food supplement, a novel food, a medical device, a medicament, a cosmetic.

Although different batches of a product comprising one or more natural matrices are, by definition, batches wherein the qualitative and quantitative composition is necessarily variable as thoroughly discussed above, according to one embodiment, a qualitative and/or quantitative analysis of each batch can be performed to demonstrate the existing qualitative and/or quantitative difference in the molecular composition of each batch. This can be achieved through conventional techniques; a non-limiting example comprises chromatography. spectrophotometry, atomic absorption spectroscopy (AAS), atomic emission spectroscopy (AES), inductively coupled plasma (ICP) techniques, chromatography coupled with detectors, and the like or combinations thereof. The analysis can be focused on a limited number of selected classes of substances (e.g., figure 5) or on all the components of the product (see examples).

According to the invention, the *in vitro* cell-based assay is an assay whose read out is associated to the pathological condition or altered physiological condition treated by the product under examination. *In vitro* cell-based assays are well-known laboratory techniques that involve the use of isolated cells to study biological processes or test the effects of drugs, chemicals, or other substances. In the present invention, the *in vitro* cell-based assay used, is designed to simulate conditions related to the pathology or pathophysiological condition or to simulate conditions related to the altered physiological state. According to the invention, cell culture models such as monolayer cultures, or three-dimensional (3D) systems can be used. When appropriate, disease-specific cell lines can be used, depending on the disease of interest (e.g., cancer) also proliferation assays can be used.

By way of example, an assay designed to mimic a disease/pathology either by using diseased cells or e.g., by inducing a diseased phenotype in cells treated with specific compounds, therefore with cells wherein a pathophysiological state control of said pathology can be induced i.e., a "disease model assay" or "disease-in-a-dish" model. Alternatively, the assay can be with cells that represent without any additional treatment during the assay, the pathophysiological state control of the pathology of interest. When the product is a beneficial product, i.e., a product that adjuvates homeostasis in an altered physiological state (not pathological) the non-modulated state control can be represented by the untreated cells, or the aberrantly modulated state control can be represented by suitably treated, differentiated, or induced cells. The cell-based assay is an assay already known in the art, optionally adapted if necessary, or it can be construed still based on the knowledge in the art with respect to the pathology or alteration of interest. Preferably, validated, and well-known cell-based assay are used. Depending on the pathology or aberrant physiological state of interest, the skilled person will select the most appropriate cell-based assay from the state of the art and optionally further adapt it with no use of inventive skill.

According to a well-established protocol, a cell line, or primary cells relevant to the disease being modelled are selected. By way of example, if studying osteoarthritis, OA, a suitable and recognised cell-based assay is with chondrocytes that, upon treatment with IL1B, are a disease model of OA. Still by way of example, if studying neurodegenerative diseases, neuronal cell lines like SH-SY5Y, primary neurons may be selected or primary cells in which the desired phenotype can be induced by an "insult" with a given compound can be used. The cell-based assay preferably includes appropriate control groups, such as untreated cells, vehicle-treated cells, and cells treated with compounds known to have no impact on the disease phenotype. These controls help distinguish the specific effects of the tested compounds. A non-limiting example of various cell-based assays suitable for the method of the invention are provided in the example section.

According to the invention, the read out of the cell-based assay provides quali-quantitative data on the modulation of selected parameters and related one or more biological activities thereto, associated to one or more hallmark of the pathological condition or of altered physiological state of interest.

Depending on the pathological condition of interest, the hallmarks can be one or more, preferably more hallmarks are selected, however, e.g., in the case of cancers, the skilled person is well aware that the more relevant hallmark is the proliferation of the cancer cells, therefore the elected cell-based assay will be in this case, an assay verifying the viability of neoplastic cells or of tumour masses upon administration of the product of interest. Still according to the invention it is preferred to use a single cell-based assay, however, more than one cell-based assays can also be used, in a non-limiting example, when for the analysis of different hallmarks different cell-based assays are deemed more suitable, additional cell-based assays can be carried out.

The skilled person will be able to select the more suitable parameters depending on the selected hallmarks of the pathological condition of interest.

Said parameters can be, by way of example, gene expression patterns, ROS, oxidative stress, cell viability and others.

A non-limiting example of pathologies of interest comprises mild cognitive impairment (MCI), osteoporosis (OP), including post or peri menopausal osteoporosis (PMO), osteoarthritis (OA), cancer.

Without limitation thereto, cancers of major interest such as head and neck cancer, melanoma, breast cancer, bladder and osteosarcoma are included.

Hallmarks of most pathologies are well-known in the art. The skilled person can easily retrieve in the scientific literature the hallmarks of pathological conditions of interest as well as the one or more biological activities underlying said hallmarks. It is evident that the skilled person will select the one or more biological activities underlying a given hallmark considering the specific pathological condition of interest.

By way of example, the skilled person can investigate the pathophysiological state-of-the-art of the disease of interest using different prior art sources.

The skilled person wishing to define hallmarks of a disease of interest will be able to retrieve the desired information from the scientific literature. A non-limiting example of sources that can be used is Robbins & Cotran Pathologic Basis of Disease (Robbins Pathology) 10th Edition; https://calgaryguide.ucalgary.ca/; Biomedical literature from PubMed Central (https://pubmed.ncbi.nlm.nih.gov/) and the like.

For each of said hallmarks several biological activities underlying them are also known in the art, the skilled person can select said activities from the ones disclosed in the art. When more biological activities are ascribed in the art to a given hallmark of a given pathology in the method of the invention, preferably at least two, at least three or more of said activities are selected.

When wishing to use specific software available for easily carrying out certain steps of the method of the invention such as finding one or more biological activities underlying a hallmark of a disease, the skilled person may wish to adapt the retrieved hallmarks adapting their definitions to better interrogate the software used. By way of example, in case the software used is Qiagen IPA (IPA version 94302991 Qiagen), the information found in the aforementioned resources can be used, if necessary, to redefine said hallmarks to interrogate IPA. To rapidly identify the one or more biological activities underlying a hallmark of a disease, when IPA is used, the following procedure can be followed:
Hallmarks can be written, one by one, in the "disease and functions" query box and the search is then launched.

The obtained resuming table allows the skilled person to filter disease/function that come from many lines of evidence. By way of example, the source for the relationship can be the Ingenuity Knowledge Base, including curation from journal articles, OMIM, JAX and ClinicalTrials.gov.

The tool is therefore able to associate with each one or more biological activities a defined number of genes whose regulation contributes to the modulation thereof. Examples of known hallmarks associated with pathological conditions are shown in figures 1-12.

In case of a beneficial product, hallmarks representing the pathological condition that can result from said alteration can be selected.

Merely by way of example, hallmarks of OA known in the art comprise: Proliferation (Skeletal and muscular system development and function such as articular dysfunction and joint space); Inflammation; Anatomical damage. Still by way of example, suitable one or more biological activities underlying the OA hallmarks above can comprise the following:
Proliferation: the one or more biological activities interested being skeletal and muscular system development and function such as articular dysfunction and joint space, articular disfunction, joint space and formation of the cartilage tissue,

Inflammation: the one or more biological activities interested being inflammatory disease, inflammation and nociception, inflammation of the joint,

Protection from anatomical damage: the one or more biological activities interested being organismal injury and abnormalities such as joint inflammation and swelling, difficulty moving a joint: osteoarthritis.

Possible state of the art keywords defining said activities are indicated in figures 1-4.

Further, by way of example, known hallmarks of MCI may comprise Cognition (impairment), Activation and viability (neuronal, decrease), Myelination and branching (decrease), Inflammation (increase), Skeletal and muscular system function (decrease).

Cognition: the one or more biological activities interested being cognition and learning;
Activation and viability: the one or more biological activities interested being development, differentiation of neurons, proliferation of neuronal cells;
Myelination and branching: the one or more biological activities interested being branching of neurons, neuronal sprouting;
Inflammation: the biological function interested being chronic inflammatory disorder;
Skeletal and muscular system function: the one or more biological activities interested being proliferation of muscle cells and necrosis of muscle.

Possible state of the art keywords defining said activities are indicated in 6-8.

Still by way of example, known hallmarks of OP, including PMO may comprise Mineralisation, Inflammation (increase), Functionality of adipose tissue (increase), Remodelling of bone, Osteopenia, Differentiation of osteoblasts (decrease).

Mineralisation: the biological activity interested being bone mineral density;
Inflammation: the one or more biological activities interested being inflammation of adipose and connective tissue;
Functionality of adipose tissue: the one or more biological activities interested being glucose tolerance and quantity of adipocytes;
Remodelling of bone: the one or more biological activities interested being remodelling and resorption of bone;
Osteopenia: the biological activity interested being osteopenia itself;
Differentiation of osteoblasts: the one or more biological activities interested being differentiation of mesenchymal stem cells, formation and quantity of osteoblasts and quantity of bone cells.

Possible keywords linked to said activities are indicated in figures 9-11.

For each of said hallmarks one or more biological activities underlying them are known in the art.

Tumour: Although a typical tumour hallmark is the proliferation of tumoral cells, each known tumour has specific hallmarks depending on the organ or system or tissue affected, an example of hallmarks of head and neck tumour (hypopharyngeal squamous cell carcinoma) is provided in figure 12.

For each of said one or more biological activities the modulation causally related to the pathological condition is also known in the art, therefore the opposite modulation can be considered as the modulation concurring to the desired therapeutical effect, consequently, a panel indicating the desired modulation pattern of each of said activities can be designed, said panel being representative of the modulation trend of said activities in a healthy physiological state (see figures 1-12). Modulation in the present description is intended as up or down modulation of the given activity. Up modulation refers to the modification in the activity, expression, of particular genes, proteins, cellular or molecular pathways or cellular components resulting in an enhancement of a given biological activity. Downregulation is the opposite of upregulation. It involves a modification in the activity, expression of genes, proteins, cellular or molecular pathways or cellular components, leading to a reduction in a given biological activity.

A non-limiting example of the modulation of the one or more biological activities causally linked to the pathological condition (OA, MCI, OP, including PMO and head and neck tumour) is depicted in figures 1-12.

Therefore, conversely, the healthy physiological state referred to the pathology of interest can be designed as the inverse modulation of each selected one or more biological activities. By way of example but not limited thereto, for OA, MCI, OP (in particular PMO) and tumours, the modulation leading to the healthy physiological state is also depicted in figures 1-12.

When the modulation pattern leading to the healthy physiological state is observed upon treatment with a given product, for each or most of the biological activities causally related to the hallmarks of a given disease, then the product can be identified as a product having the desired therapeutic effect. Therefore, a product inducing the modulation of the all the selected biological activities causally related to the hallmarks of a given pathological condition in the direction toward the healthy physiological state, can be identified as a product having the desired therapeutic action on the overall pathological state. When only some of the biological activities causally related to the hallmarks of a disease are in conformity with the physiological standing as defined above, then the product has only a partial therapeutic action that does not satisfy the requirement of modulation of a state.

When gene expression is selected as a marker, the genes and the expression pattern of each of said genes underlying the modification of each biological activity causally linked to each selected hallmark of a pathological condition can be retrieved by the skilled person from the state of the art, this work can be facilitated by using *ad hoc* bioinformatics tools. The same Qiagen IPA indicated above is suitable for a fast sorting out of said information from scientific literature, as it is an aggregator of scientific references that allows to search for information on genes/proteins and the construction of networks that predict the behaviour of biological systems according to their gene expression status.

The pathophysiological features (hallmarks) available in the state of the art for a given pathological condition are used to interrogate IPA via the "IPA Bioprofiler" tool, using them as key words, additional keywords related to said hallmarks can also be added.

The use of "IPA Bioprofiler" allows the skilled person to identify the expressed genes causally linked to each of the identified one or more biological activities and the specific molecular pathway underpinning them. Information concerning the measured gene expression data (by way of example, following the manufacturer's instructions Fold change value cut-off ≤-2 and ≥ +2 and p-value≤0.05) induced by each batch was then superimposed on the networks obtained, to define influenced genes and modulation of the connected biofunction.

Once the most relevant genes and the expression pattern thereof underlying the modification detectable in the pathological condition of interest for each of the selected one or more biological activities are identified, for each of said genes the expression pattern opposite to the one identified is set as the expression pattern indicative of the healthy physiological state of said one or more biological activities.

As stated above, modulation of biological activity is either a down-modulation or up-modulation depending on the related genes regulation. The resulting expected calculated impact, based on the literature, on the related biofunctions can be determined by "IPA Molecule Activity Predictor" tool (MAP) and resumed in a heatmap visualization using a colour code that can easily be transformed into numeric values by the user.

When performing the cell-based assay according to the method of the invention, the following steps can be carried out:
- identifying one or more parameters whose modulation analysis in a cell-based assay allows a quali-quantitative evaluation of the modulation of one or more biological activities underlying the therapeutic or beneficial effect of the product tested and determining the modulation pattern representative of the desired healthy physiological state;
- performing the *in vitro* cell-based assay by treating, for each different batch analysed, a different group of cells of the assay with a different batch of said product, and providing at least one control group of cells and detecting in each cell group, the modulation pattern of each of the parameters identified;
- determining the quali-quantitative modulation of each of said parameters, in said control cell group and in each treated cell group, and calculating the respective modification values (values quantifying and qualifying the modulation) for each of said one or more biological activities;
- comparing said values for each one or more biological activities calculated for each treated cell group.

In particular, the step of identifying one or more parameters whose modulation analysis in a cell-based assay allows a quali-quantitative evaluation of the modulation of one or more biological activities underlying the therapeutic or beneficial effect of the product tested and determining the modulation pattern representative of the desired healthy physiological state can be performed by:
- providing a list of hallmarks representative of the pathological state associated to a pathology of interest or to an imbalance that can derive from a non pathological altered physiological state of interest (depending on whether the product under examination is a therapeutic product or a beneficial product adjuvating homeostasis);
- identifying for each of said hallmarks one or more biological activities modifications underlying said pathology or non pathological altered physiological state and determining the modulation thereof representing the pathophysiological state or said non pathological altered physiological state associated to said pathology or alteration (respectively) and assessing the opposite modulation as the modulation pattern of each of said activities representing a healthy physiological state;
- identifying one or more marker and the modulation pattern thereof underlying the modification detectable in said pathological state, or of said non pathological altered state, for each of said one or more biological activities and setting for each of said parameters the modulation pattern opposite to the one identified, as the modulation pattern concurring to said healthy physiological state.

From the results obtained with the cell-based assay it is possible to determine the quali-quantitative modulation of each of the parameters previously identified, induced by each product batch sample with respect to the control group and it is therefore possible to obtaining values representing the qualitative (which activity is modulated and in which direction, i.e., up modulation positive value or down-modulation negative value) and quantitative (how much is each activity modulated with respect to the control group) modulation induced by each batch on each of said one or more biological activities; the magnitude of the value representing the distance (in terms of %, folds etc.) with respect to the control group. The modulation of each activity can be expressed as a numerical value, such a statistical numerical value.

The comparison of the values calculated for each group and for each one or more biological activities allows to verify whether the product regulates a pathological state or altered physiological state, and whether the product shows functional resilience, i.e., whether, notwithstanding the possible different regulation of each marker due to the different qualitative and quantitative difference among batches, the modulation induced by each batch of the product is substantially qualitatively and quantitatively equivalent thereby resulting in the same therapeutic or beneficial effect.

Depending on the parameters selected, the modulation of a biological activity can be defined and characterized by the skilled person by commonly used various quantitative values depending on the specific context and the type of biological activity being studied. Some common values used to define modulation include: fold change i.e., the ratio of the value of the biological activity under a particular condition or treatment to its value under a control or reference condition; by way of example, a fold change of 1 indicates no change, while values greater than 1 indicate upregulation and values less than -1 indicate downregulation; log fold change i.e., the logarithm (usually base 2 or base 10) of the fold change; percentage change, i.e., the percentage difference between the value of the biological activity under a particular condition and its value under a control or reference condition; z-score, which z-score represents the deviation of the observed value of the biological activity from its mean value, normalized by the standard deviation (a positive z-score indicates an increase in activity, while a negative z-score indicates a decrease); effect size, i.e., a measure of the magnitude of the difference between two groups, often standardized to facilitate comparisons across studies or datasets (Cohen's d is a common effect size measure, calculated as the difference in means divided by the standard deviation); area under the curve (AUC), for dynamic biological activities, such as signalling pathways or physiological responses, the AUC can be used to quantify the overall activity or response over a period of time.

These values can be used individually or in combination to provide a comprehensive characterization of the modulation of a biological activity under different conditions or treatments. The choice of which value(s) the skilled person decides to use depends on the specific research question, the nature of the biological activity, and the available data.

In the context of biological activities, a z-score can be used to express the modulation or alteration of a specific biological activity relative to its typical or baseline behaviour or relative to pathological condition. This is often employed in fields like systems biology, where researchers analyse high-dimensional datasets to understand complex biological processes.

In this case, the z-score represents how much a particular biological activity deviates from its expected or average behaviour within a given context, often in response to some stimulus, treatment, or condition.

For example, in gene expression analysis, a z-score might be calculated to assess how much the expression level of a gene changes in response to a therapeutic treatment compared to its expression in control conditions. A positive z-score indicates upregulation, while a negative z-score indicates downregulation.

Mathematically, a suitable formula for calculating the z-score of a biological activity modulation might involve comparing its observed value under a specific condition to the mean and standard deviation of its values across multiple conditions or replicates:$z = x - μ / σ$

Where *x* represents the observed value of the biological activity (e.g., gene expression level), µ is the mean value of the activity across all conditions, and σ is the standard deviation of the activity across all conditions.

This z-score approach allows researchers to identify and prioritize biological activities that are significantly altered under certain experimental conditions, providing insights into the underlying mechanisms of biological systems.

The results of the transcriptomic data provided in the examples below show that, when gene expression parameters are used, although regulating different sets of genes, different batches of the tested product provide the same regulation of the one or more biological activities of interest and therefore in the same therapeutic effect.

From the calculated values, which are representative of the qualitative and quantitative modulation induced by each batch on each one or more biological activities it is possible, following the teachings herein provided, to assess whether a whole state rather than one or few activities is regulated by the product of interest, and whether the different batches of product analysed maintain a functional resilience (therapeutical or beneficial).

Assessment of the modification of a state and of functional resilience.

The capability of the product under examination of modifying a pathophysiological state or an altered physiological state (e.g., by adjuvating the homeostasis response of the organism) is a crucial feature for establishing the physiological mode of action of a therapeutic or beneficial product. Indeed, a product acting with networks- to -network interaction is a product that is expected to modify a state rather than a single function when administered to a living organism.

In other words, this feature is likely to be satisfied by a therapeutic or beneficial product comprising one or more natural matrices or consisting of one or more natural matrices given the networks-network (product-receiver) interactions exerted by natural matrices. However, this capability must be determined and at present no procedures are available for this determination.

In addition, a product exerting a physiological mode of action is also expected to be 100% natural and to show the flexibility and self-administrating mechanisms that are observable in living organisms wherein different messages within the cell and among cells as well as different regulations of gene pathways can provide the same result notwithstanding the different messages triggered within the cell. For a therapeutic or beneficial product this equates to the functional resilience of different batches of the product (with a quali-quantitative variability in their chemical composition).

The present description provides modes for determining whether a therapeutic or beneficial product exerts its therapeutic or beneficial effect by modifying a pathological state or an altered physiological (not yet pathological) state and whether the product shows or not functional resilience (i.e., the maintenance of the therapeutic or beneficial properties of different batches of a given product comprising one or more natural matrices, notwithstanding its different batch to batch qualitative and quantitative composition).

According to the invention, this can be verified by carrying out a cell-based assay as described in the specification, and by analysing and interpreting the data obtained therefrom as defined in the claims.

Depending on the cell-based assay selected, the analysis of the data can vary slightly and is aimed to verify that modulation of all the selected one or more biological activities underlying the hallmarks of the pathology, or the altered physiological state is in the direction of a healthy physiological state (i.e., opposed to the modulation of said activities underlying the pathological state or altered physiological state).

It is understood that in the embodiments described below, the cell-based assay can be performed on one or more batch of the product in parallel, creating, besides the control group of cells, several test groups of cells (i.e., groups of cells treated with one batch of the product) equal to the number of different batches tested (or duplicates or triplicates thereof).

Assessment of functional resilience (therapeutic or beneficial).

As indicated in the glossary and in the specification above, in the present description and claims, functional resilience is the maintenance of a therapeutic or beneficial measurable efficiency in different batches of a product notwithstanding variability in their qualitative and quantitative molecular composition.

It is clear that the batches are intended as batches that are identical in terms of manufacturing processes and type and amounts of each ingredient (as the main ingredients of the selected products are natural matrices, this means that each matrix in the product is produced from the same kind of starting materials and with the same procedures e.g. a given kind of extract from the same plant part of the same plant species), therefore, the variability in their qualitative and quantitative molecular composition cannot be ascribed to different manufacturing procedures or to different ingredients but can only originate from the inherent difference between natural matrices obtained with the same procedure from different organism/s of the same species. When different batches of the same product comprising one or more natural matrices maintain in a measurable and verifiable way their final modulatory activities underlying their therapeutic or beneficial properties irrespectively of their quali-quantitative composition, functional resilience of the product can be validated.

In an embodiment of the invention, the *in vitro* cell-based assay can be an assay in which a pathophysiological state control of said pathology can be induced in cells, e.g., subjecting said cells to an appropriate treatment, thereby inducing in the cells said pathophysiological state control. In this case, one or more group of induced cells can be treated, respectively, with one or more different batch of the product of interest.

According to an embodiment of the invention the method comprises, before performing the one or more cell-based assay (1) providing a list of hallmarks representative of said pathological state; (2) identifying for each of said hallmarks one or more biological activities modifications underlying said pathological state thereby pinpointing a network of biological activities whose modulation concurs to said pathological state and (3) identifying one or more parameters whose modulation concurs to the modulation of said one or more biological activities underlying the therapeutic effect of the product tested and determining the modulation trend in terms of up or down modulation of said one or more biological activities, in said network, concurring to said pathological state or to a healthy state.

**In** other words, the method can comprise
(1) providing a list of hallmarks representative of the pathological state associated to a pathology or to a pathology that can stem from said non-pathological altered physiological state, i.e., providing a list of hallmarks representative of the disease or pathophysiological condition treated by the product of interest or that may derive from the non-pathologically altered physiological state in which homeostasis is adjuvated by the beneficial product of interest;
(2). identifying for each of said hallmarks one or more biological activities modifications underlying said pathology and determining the modulation thereof representing the pathophysiological state associated to said pathology and assessing the opposite modulation as the modulation pattern of each of said activities representing a healthy physiological state; and
(3). identifying one or more marker and the modulation pattern thereof underlying the modification detectable in said pathological state for each of said one or more biological activities and setting for each of said parameters the modulation pattern opposite to the one identified, as the modulation pattern concurring to said healthy physiological state.

In case a therapeutic product is examined, according to an embodiment, the method of the invention may comprise the following steps:
(a) performing said at least one *in vitro* cell-based assay on the following groups of cells
(a1) at least one control group and at least two test groups of cells having the diseased phenotype relevant to the intended use of the therapeutic product; or
(a2) at least one group of cells with a healthy physiological phenotype; and at least one control group and at least two test groups of said cells with a healthy physiological phenotype wherein the diseased phenotype relevant to the intended use of the therapeutic product is induced,
and treating each of said test groups of cells with one of said different batches of therapeutic product;
(b) determining the modulation or modulation pattern of each of said parameters on each of said groups of cells of step (a) and calculating the respective modulation values for each of said one or more biological activities;
(c) comparing said modulation values, wherein:
   said therapeutic product is shown to exert its therapeutic effect through a physiological mechanism when
at least 50% of said one or more biological activities for each hallmark are modulated by each product batch with the modulation trend of the network concurring to the healthy state, and the modulation values determined in (b) of each of said at least 50% one or more biological activities for said test groups of cells of (a1) differ, respectively, from the ones of said control group of cells of (a1) of at least 0.15, or
at least 50% of said one or more biological activities for each hallmark are modulated by each product batch with the modulation trend of the network concurring to the healthy state, and the modulation values determined in (b) of each of said at least 50% one or more biological activities for said test groups of cells of (a2) differ, respectively, from the ones of said control group of cells of (a2) by at least 15%, and
functional resilience of the product is demonstrated by the modulation values for each one or more biological activities of each said test groups of cells differing by less than 20% from the average of said values.

This means, that the modulation value of a given biological activity of the test group is confronted, respectively, with the modulation value of the same biological activity of the control group, therefore the difference of at least 0.15 or of at least 15% is the difference between the modulation value of a given activity in the treated group of cells with respect to the modulation value of the same activity in the group of cells representing the control baseline.

In other words, the method may also be described as a method of assessing whether a therapeutic product exerts its effect to treat a pathological state through a physiological mechanism of action comprising:
- providing different batches of a therapeutic product, the product comprising one or more natural matrix
- providing a list of hallmarks representative of said pathological state, identifying for each of said hallmarks a set of parameters whose modulation allows an evaluation of a network of biological activities underlying the therapeutic effect of the product tested and determining the modulation trend in terms of up or down modulation of said activities in said network in the diseased and healthy state;
   (a) performing at least one *in vitro* cell-based assay on the following groups of cells
      (1) at least one control group and at least two test groups of cells having the diseased phenotype targeted by the therapeutic product; or
      (2) at least one group of cells with a healthy physiological phenotype; and at least one control group and at least two test groups of said cells with a healthy physiological phenotype wherein the diseased phenotype targeted by the therapeutic product is induced,
         and treating each of said test groups of cells with one of said different batches of therapeutic product;
   (b) determining the modulation or modulation pattern of each of said parameters on each of said groups of cells and calculating the respective modulation values for each of said biological activities;
   (c) comparing said modulation values for each biological activity in each cell group of step (b), wherein:
      the therapeutic product is shown to exert its therapeutic activity through a physiological mechanism of action when
      at least 50% of said biological activities for each hallmark are modulated by each product batch with the modulation trend of the healthy state determined in (b) and the modulation values determined in (b) of each of said at least 50% one or more biological activities for said test groups of cells of (a) (1) differ, respectively, from the ones of said control group of (a) (1) of at least 0.15 or
      at least 50% of said biological activities are modulated by each product batch with the modulation trend of the healthy state determined in (b) and the modulation values determined in (b) of each of said at least 50% one or more biological activities for said test groups of cells of (a) (2) differ, respectively, from the ones of said control group of (a) (2) by at least 15%,
      and functional resilience of the product is demonstrated by the modulation values for each biological activity of each said test groups of cells differing by less than 20% from the average of said values.

The expression at least 50% of said one or more biological activities for each hallmark are modulated by each product batch with the modulation trend of the network concurring to the healthy state, means that, in case of a single biological activity for a given hallmark, in order to fulfil the requirement above, 100%, i.e., the single activity, must be modulated by the product tested, with the modulation trend concurring to the healty state, The expression "the modulation values of each said at least 50% one or more biological activities determined in (b)" refers to the modulation values determined in (b) of that at least 50% of biological activities fulfilling the requirement of being modulated according to the modulation trend concurring to the healthy state. This applies, mutatis mutandis, to all the embodiments disclosed herein.

In case a beneficial product (i.e., a product exerting a beneficial effect on an altered but not yet pathological physiological state by adjuvating homeostasis) is examined, according to an embodiment, the method of the invention may comprise the following steps:
(a) performing said at least one *in vitro* cell-based assay the following groups of cells:
   at least one control group of cells having healthy phenotype or at least one control group of cells wherein the aberrantly modulated phenotype targeted by said beneficial product is suitably induced, and at least two test groups of cells taken from said control group
   and treating, each of said test groups of cells, with one of said different batches of beneficial product;
(b) determining the modulation or modulation pattern of each of said parameters on each of said groups of cells of step (a) and calculating the respective modulation values for each of said one or more biological activities;
(c) comparing said modulation values, wherein:
   said beneficial product is shown to exert its homeostasis adjuvating effect through a physiological mechanism of action when
   at least 50% of said one or more biological activities for each hallmark are modulated by each product batch with the modulation trend of the network concurring to the healthy state and the the modulation values calculated in (b) of each of said at least 50% one or more biological activities for said test groups of cells differ, respectively, from the ones of said control group of cells of at least 0.15 and
   functional resilience of the product is demonstrated by the modulation values for each one or more biological activities of each said test groups of cells differing by less than 20% from the average of said values.

This means, that the modulation value of a given biological activity of the test group is confronted, respectively, with the modulation value of the same biological activity of the control group, therefore the difference of at least 0.15 or of at least 15% is the difference between the modulation value of a given activity in the treated group of cells with respect to the modulation value of the same activity in the group of cells representing the control baseline.

In other words, the method can be defined as a method of assessing whether a beneficial product exerts its homeostasis adjuvating effect by regulating an altered physiological state comprising:
- providing different batches of a beneficial product adjuvating homeostasis, the product comprising one or more natural matrix
- providing a list of hallmarks representative of a pathological state that can stem from said altered physiological state, identifying for each of said hallmarks a set of parameters whose modulation allows an evaluation of a network of biological activities underlying the therapeutic effect of the product tested and determining the modulation trend in terms of up or down modulation of said activities in said network in the diseased and healthy state;
   (a) performing at least one *in vitro* cell-based assay on the following groups of cells:
      (1) at least one control group of cells having a healthy phenotype or at least one control group of cells wherein the aberrantly modulated phenotype targeted by said beneficial product is suitably induced, and at least two test groups of cells taken from said control group
         and treating each of said test groups of cells with one of said different batches of beneficial product;
   (b) determining the modulation or modulation pattern of each of said parameters on each of said groups of cells of step (a) and calculating the respective modulation values for each of said biological activities;
   (c) comparing said modulation values for each biological function in each cell group of step (a), wherein:
      the beneficial product is shown to exert its homeostasis adjuvating effect through a physiological mechanism when
      at least 50% of said biological activities for each hallmark are modulated by each product batch with the modulation trend of the healthy state determined in (b) and the modulation values determined in (b) of each of said at least 50% one or more biological activities for said test groups of cells of (a) (1) differ, respectively, from the ones of said control group of (a) (1) of at least 0.15,
      and functional resilience of the product is demonstrated by the modulation values for each biological activity of each said test groups of cells differing by less than 20% from the average of said values.

In a preferred embodiment, the control group, is considered as the reference modulation baseline and the quali-quantitative modulation value for each of said one or more biological activities is considered to be 0.

As previously indicated, the method of the invention comprises:(1) providing a list of hallmarks representative of the pathological state of interest (i.e., the pathological state treated by the product analysed or the pathological state that can stem from the altered physiological state on which the beneficial product analysed exerts its homeostasis adjuvating activity); (2) identifying for each of said hallmarks one or more biological activities modifications underlying said pathological state thereby pinpointing a network of biological activities whose modulation concurs to said pathological state and (3) identifying one or more parameters whose modulation concurs to the modulation of said one or more biological activities underlying the therapeutic effect of the product tested and determining the modulation trend in terms of up or down modulation of said one or more biological activities, in said network, concurring to said pathological state or to a healthy state.

All the embodiments above allow to determine whether a therapeutic or beneficial product exerts its therapeutic or beneficial effect by modifying a state or merely a limited number of activities or even a single function underlying the pathology an aberrant physiological state treated by said product and whether a therapeutic or beneficial product as the ones selected maintains a functional resilience as herein defined.

The modification of a state is a feature that cannot be obtained with a one-API pharmaceutical product, therefore, this feature rules out classical pharmaceutical modes of action. However, the modification of a state could, in principle, be obtained with a pharmaceutical product comprising a cocktail of APIs.

A physiological mode of action requires that the therapeutic or beneficial product regulates the state in a manner that involves an overall cellular response with a networks over network interaction, and not in a points over network interaction that is at the basis of APIs mode of action (be it a single API or a cocktail thereof).

This implies, besides the regulation of a state, also the capability of the product of acting with a functional resilience (either therapeutic or beneficial), i.e., providing the same batch to batch therapeutic/beneficial effect notwithstanding the different batch to batch quali-quantitative composition which, in other words, is the result of regulating the various parameters selected in variable ways and nevertheless providing a conserved functional result.

The present invention hence also provides modes for demonstrating the functional resilience of therapeutic or beneficial products.

As already explained in the glossary and in the description above, functional resilience (therapeutic or beneficial) is the capability of a given product of modulating one or more biological activities underlying a pathological state or an altered physiological state notwithstanding variability in the qualitative and quantitative composition of different batches of the same product and therefore notwithstanding the possibility of reaching the same final result triggering different signals within the cell i.e., showing bioequivalence intended as same final result. As already stated above is known that pharmaceutical (API based) products, with a different qualitative and quantitative composition are not considered bioequivalent.

The physiological mode of action, which implies an overall interaction with the cells of the subject treated and not with singled out cellular molecular targets, is the mode exerted by living organisms as the result of networks-network interactions. Physiological systems in the body often exhibit functional redundancy to maintain homeostasis and adapt to changes or disruptions, redundancy being a well-known physiological mechanism in the living for ensuring that a given goal is reached (e.g., a response of the organism in the production of various proteins, in the activation of various pathways etc.). When a therapeutic product interacts with these systems, it may engage multiple pathways or mechanisms, including redundant ones, to achieve its desired effect. This redundancy, which results in a functional resilience, contributes to the physiological mode of action of the product.

Functional resilience in therapeutic/beneficial products (intended as the ability of a therapeutic or beneficial product to maintain its intended functionality and effectiveness despite variability in its batch-to-batch qualitative and quantitative composition) is therefore an essential feature of a physiological mode of action.

According to an embodiment of the invention the selected parameters can be genes whose expression pattern underlines the modification detectable in the pathological condition of interest, in this embodiment, (3) as exemplified above is:
identifying the genes and the expression pattern thereof underlying the modification detectable in said pathological condition for each of said one or more biological activities and setting for each of said genes the expression pattern opposite to the one identified as the expression pattern indicative of said healthy physiological state.

When the parameters and modulation pattern thereof correspond to the genes and the expression pattern thereof underlying the modification detectable in pathological condition for each the selected one or more biological activities is analysed, a transcriptomics analysis on a suitable in vitro cell-based assay representing the pathological condition of interest is carried out, and the genes and the expression pattern thereof underlying the modification detectable in the pathological condition for each of the selected one or more biological activities can be identified. In the case in which the disease phenotype is caused by the administration of a specific agent to the cultured cells, the modification representing the pathological condition is the modification insulted cells vs. pre-insulted, untreated cells and the modification induced by the product batch is the modification insulted cells vs. insulted cells + product batch.

The transcriptomic analysis can be carried out with any suitable technique known in the art, including next generation sequencing and gene expression microarrays and the transcriptomic expression profile in the basal cells as opposed to the cells treated to mimic the pathological condition can be evaluated to identify the significantly differentially expressed genes and their expression patterns. According to the method of the invention, once the expression pattern of the significantly differentially expressed genes of the cells representing the disease phenotype vs. the cells before the insult inducing said diseased phenotype is assessed, the opposite expression pattern is considered representative of the healthy physiological state.

When gene expression is the selected marker, for each one or more biological activities identified in (2) the genes and the expression pattern thereof underlying the modification detectable in said pathological condition for each of said one or more biological activities can be identified from the state of the art using appropriate tools.

By way of example, the skilled person can derive this information using any suitable approach including using software specifically designed for this scope such as Ingenuity Pathway Analysis (IPA version 94302991 Qiagen).

The interpretation of high-throughput gene-expression data is greatly facilitated by the consideration of prior biological knowledge. This can be done using statistical gene-set-enrichment methods where differentially expressed genes are intersected with sets of genes that are associated with a particular one or more biological activities or pathway (Abatangelo, L. et al. (2009) Comparative study of gene set enrichment methods. BMC Bioinform., 10, 275). One more recent approach involves the application of causal networks that integrate previously observed cause-effect relationships reported in the literature (Chindelevitch, L. et al. (2012a) Causal reasoning on biological networks: interpreting transcriptional changes. Bioinformatics, 28, 1114-1121; Felciano, R.M. et al. (2013) Predictive systems biology approach to broad-spectrum, host-directed drug target discovery in infectious diseases. Pac. Symp. Biocomput., 2013, 17-28; Kumar, R. et al. (2010) Causal reasoning identifies mechanisms of sensitivity for a novel AKT kinase inhibitor, GSK690693. BMC Genom., 11, 419; Martin, F. et al. (2012) Assessment of network perturbation amplitudes by applying high-throughput data to causal networks. BMC Syst. Biol., 6, 54; Pollard, J. Jr. et al. (2005) A computational model to define the molecular causes of type 2 diabetes mellitus. Diabetes Technol. Ther., 7, 323-336). While still depending on statistics, this is more powerful than gene-set enrichment since it leverages knowledge about the direction of effects rather than mere associations.

In a preferred embodiment, the skilled person can follow the protocol provided in the publication by Kramer et al, Bioinformatics vol 30 no 4 2014, pages 523-530 "Causal analysis approaches in Ingenuity Pathway Analysis provides and discuss a suite of algorithms and tools for inferring and scoring regulator networks upstream of gene expression data based on a large-scale causal network derived from the Ingenuity Knowledge Base" or the manufacturer's instructions of (IPA version 94302991 Qiagen). The method and algorithms disclosed in the paper enable the skilled person to predict downstream effects on one or more biological activities and diseases. In the article, the authors describe causal analysis approaches that have been implemented in Ingenuity Pathway Analysis (IPA) with particular focus on the details of the underlying algorithms, and the application to several real-world use cases. In particular, the identification of the modulation trend of the biological activities selected in the pathological and healthy state can be readily carried out by the skilled person by using Ingenuity Pathway Analysis (IPA version 94302991 Qiagen) which is well-known pathway analysis application among the life science research community cited in tens of thousands of articles that allows to understand causal connections between and among diseases, genes and networks of upstream regulators.

Once the desired modulation of the one or more biological activities is defined (i.e., the one representing the healthy physiological state) and the desired regulation of the related marker (e.g. ROS scavenging activity, genes, etc.) is identified for the pathological condition of interest , an analysis of the selected marker modulation (e.g. for gene expression a transcriptomic analysis) in the same in vitro cell-based assay representative of the pathological condition is carried out upon treatment of the cells, with a sample of the therapeutic product under examination is carried out. The quali-quantitative modulation of the marker/s (e.g., of the expression of the genes of interest) induced by the treatment for the generation of the physio pathological state control group vs. the untreated cells and by the sample of the examined product vs. the pathophysiological stat is determined and values quantifying the modulation for each of the selected one or more biological activities are calculated and subsequently used in the comparison in step.

The values quantifying the modulation are calculated to assess the directionality and magnitude of the modulation for each one or more biological activities exerted by a given sample of product. For transcriptomics, the Core analysis of the IPA version 94302991 Qiagen can be used by the skilled person to readily obtain the aforementioned values in terms of z-score following the manufacturer's instructions.

In case the Core analysis does not yield sufficient relevant information, alternative approaches can be employed. An example of an alternative approach is provided below (overlay analysis).

In a non-limiting embodiment of the invention the identification of one or more biological activities modifications underlying said pathological state for each selected marker (thereby pinpointing a network of biological activities whose modulation concurs to the pathological state of interest) and the identification of one or more parameters whose modulation concurs to the modulation of said one or more biological activities underlying the therapeutic effect of the product tested and determining the modulation trend in terms of up or down modulation of said one or more biological activities, in said network, concurring to said pathological state or to a healthy state can be carried with the aid of IPA version 94302991 Qiagen out as follows:
Definition of pathophysiological state of the art of the disease and of the pathophysiological hallmarks of the disease with which to interrogate IPA

The pathophysiological state-of-the-art of the disease of interest is investigated using different specific sources:
- Robbins & Cotran Pathologic Basis of Disease (Robbins Pathology) 10th Edition
- https://calgaryguide.ucalgary.ca/
- Biomedical literature from PubMed Central (https://pubmed.ncbi.nlm.nih.gov/)

The information found in the aforementioned resources are used to identify hallmarks to interrogate IPA by the following procedure:
- Hallmarks are written, one by one, in the "disease and functions" query box and the search is then launched.
- The obtained resuming table allows you to filter disease/function that come from many lines of evidence. The source for the relationship is the Ingenuity Knowledge Base, including curation from journal articles, OMIM, JAX and ClinicalTrials.gov.
- In silico model will be limited to genes and mRNAs.

The tool is therefore able to associate with each one or more biological activities a defined number of genes whose modulation influences the modulation of the one or more biological activities itself.

The following applies to any step of the method of the invention in any embodiment herein described, wherein an in vitro cell-based assay is carried out treating the cells with the disease phenotype with a given compound, be it the golden standard, a different batch of the product of interest, a reference drug and the like.

### Transcriptome raw data analysis

Whole transcriptome expression profile is evaluated in the *in vitro* cellular model representing the disease for control cells and for model cells. A Human Clariom^{™} S Pico Assay HT (Applied Biosystems, ThermoFisher Scientific) on a GeneTitan MC Instrument (Applied Biosystems, ThermoFisher Scientific), can be used following the manufacturer's instructions. CEL Intensity files can be generated by Affymetrix GeneChip Command Console Software (AGCC, ThermoFisher Scientific). Data analyses can be performed using Transcriptomic Analysis Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package). This phase allows the user to obtain a list of differentially expressed genes (DEGs), identified based on their expression fold changes with respect to a relevant control experimental condition, in this case one that reproduces the pathological state *in vitro.*

The transcriptional modifications profile thus obtained is subjected to a functional pathway enrichment analysis. One of the commercial tools that can be used is Ingenuity Pathway Analysis (IPA version 94302991, Qiagen) [Krämer et al. (2014)]. The use of IPA allows the user to estimate how and to what extent the modulation of gene expression in the cellular system (cell-based assay) influences the one or more biological activities related to the pathology of interest.

IPA pre-analysis filtering (contextual data analysis) of transcriptional profile

In preparation for subsequent analyses, transcriptomic profiles undergo a filtration process to identify relevant genes and their corresponding measurement values. This filtration aims to select only significantly perturbed genes, as indicated by their fold change compared to the pathological condition. Typically (e.g., as according to the manufacturer's instructions), the fold change threshold is set to encompass values ≤ -2 and ≥ +2, accompanied by a statistical significance denoted by a p-value of ≤ 0.05. However, the skilled operator has the flexibility to adjust the cut-off based on their expertise, considering the successful performance of negative controls (a sample representing the pathological state) or reference standard known to be able to counteract fully or partially the pathological state.

The possible methodological approaches to extract biological meaningfulness from a list of modifications of gene expression profiles through IPA can be of two types: "Core analysis" and "Overlay analysis of in silico model of pathophysiological state" (abbreviated as overlay analysis).

At this stage of the procedure, hence, two different, alternative, options can be pursued.

### Core Analysis

The list of Differentially expressed genes (DEGs) after administration of the reference batch or of any other product batch/reference drug or other, and corresponding data measurement values (fold change with respect to pathological state) identified in the different experimental conditions are uploaded into the application. Available identifiers are mapped to their corresponding entity in QIAGEN's Knowledge Base.

By launching a "Core Analysis", significantly perturbed DEGs, called Network Eligible molecules, are overlaid onto a global molecular network developed from information contained in the QIAGEN Knowledge Base. Networks of Network Eligible Molecules are then algorithmically generated based on their connectivity.

The core analysis provides a comprehensive list of approximately top 500 one or more biological activities derived from the generated networks. The associations Biological activities-genes are always supported by annotations corresponding to scientific peer review publications that substantiate, through the automatic association of a value quantifying the modulation (z-score) [Krämer et al. (2014)], the calculated directionality and magnitude of modulation of the biological activities. In essence, this value represents a statistical metric that assesses the similarity between the observed pattern of Differentially Expressed Genes (DEGs) and the expected pattern based on existing literature for a given annotation.

It is the responsibility of the proficient operator to carefully choose the one or more biological activities that are relevant to the specific pathology under investigation. The selection of one or more biological activities will be structured based on the identified hallmarks of the pathology of interest.

The associated values quantifying the modulation (e.g., z-scores) will then be used to indicate the directionality and magnitude of modulation for each one or more biological activities.

### Overlay Analysis

If the core analysis does not yield sufficient relevant information, an alternative approach called "Overlay analysis" can be employed. This analysis focuses on the one or more biological activities identified by the "in silico model of the pathophysiological state". The selection of one or more biological activities is structured based on the identified hallmarks of the pathology of interest.

The "Overlay analysis" is structured by establishing relationship between patterns of differentially expressed genes and selected one or more biological activities (always supported by annotations corresponding to scientific peer review publications that substantiate the directionality and magnitude of modulation of the one or more biological activities) using the following procedure:
□ Import the set of one or more biological activities selected from the *in silico* model of the pathophysiological state into a new sheet called "my pathway."
□ Utilize the "Build tool" and "Grow tool" to identify Differentially Expressed Genes (DEGs) belonging to the transcriptomic profile under investigation and are linked to regulation of the one or more biological activities selected in the previous step.
□ Modulation of the identified DEGs is represented using green colour (indicating down-modulation) and red colour (indicating up-modulation).
□ To determine the expected calculated impact of such experimentally observed modulations of gene expression on one or more biological activities activity, the "Overlay" and "Molecule Activity Predictor" tool (MAP) are employed. The "Prediction" function is activated within MAP tool to calculate the resulting expected modulation of one or more biological activities. Colour coding is thus established:
   - orange: increase in activity
   - blue: decrease in activity
   - white: not achievable/not predictable

As the "Overlay analysis" does not directly calculate the value quantifying the modulation for each one or more biological activities but provides results in terms of colours indicating the direction of the modification and intensity of the colour signal proportional to the magnitude of the modulation of interest, it is necessary to translate the intensity of the modulation signal (graphically represented in the "my pathway" tab) into a numerical value. This is achieved by converting the colour intensity obtained for each one or more biological activities into a value quantifying the modulation.

The biological pathway generated by QIAGEN's Ingenuity Pathway Analysis software can be converted in numerical data by the use of a specific algorithm able to convert RGB colour intensity model to the LAB model (https://www.xrite.com/it-it/blog/lab-color-space posted on October 2018 by Tim Mouw). This conversion occurs within a Pipeline Pilot "component" that uses a procedure written using R software that is based on specific features of the colorspace package (https://cran.r-project.org/web/packages/colorspace/index.html, details can be found on Zeileis et al 2020journal of statistical software, doi:10.18637/jss.v096.i01).

The value quantifying the modulation allows for an objective comparison of the impacts of different treatments.

The method finally comprises (c) comparing the values quantifying the modulation of each one or more biological activities calculated for each group in and, when the conditions set out above are fulfilled the product is shown to exert its beneficial or therapeutic action through a physiological mechanism (mode) of action.

Step (c) requires that at least 50% of the one or more biological activities analysed are modulated by each product batch with the modulation trend of the network concurring to the healthy state previously determined in.

"with the modulation trend of the healthy state" can be read also as "According to said modulation pattern representative of said healthy physiological state" and it means that the modulation of the analysed activity has the same directionality, i.e., an up or down modulation of the modulation of the same activity in the healthy physiological state as determined, with respect to the one determined for control group of cells.

This means that each hallmark must be modulated in the direction of the desired therapeutic effect for at least 50% of the selected activities for said hallmark. Therefore, where one or two activities are selected for a single hallmark, 100% of the modulation determined, must be consistent with the desired therapeutic effect (i.e., the modulation pattern representative of the healthy physiological state).

In the embodiment wherein the product analysed is a therapeutic product, when the groups of cells used in the assay corresponds to (a) (2) above, step (c) requires that at least 50% of said one or more biological activities are modulated by each product batch with the modulation trend of the network concurring to the healthy state determined and each of said modulation values determined in (b) for said cell groups treated with the product of (a) (2) of said at least 50% one or more biological activities differ from the ones of said control group of (a2) by at least 15%. In other words, the modulation is considered significant when it differs of at least 15% from the values quantifying the modulation calculated for the control cells, and it has to be in the same direction of the desired therapeutic effect, therefore, when the modulation pattern representative of the healthy physiological state for a given activity is an up regulation the modulation induced by the product to said activity has to be with a value quantifying the modulation at least 15% higher than the modulation calculated for the same activity in the pathophysiological state, and when the modulation pattern representative of the healthy physiological state for a given activity is a down regulation the modulation induced by the product to said activity has to be with a value quantifying the modulation at least 15% lower than the modulation calculated for the same activity in the pathophysiological state.

In the embodiment wherein the product analysed is a therapeutic product, when the groups of cells used in the assay corresponds to (a) (1) above, step (c) requires that at least 50% of said one or more biological activities are modulated by each product batch with the modulation trend of the network concurring to the healthy state previously determined and each of said modulation values determined in (b) for said cell groups treated with the product of (a) (1) of said at least 50% one or more biological activities differ from the ones of said control group of (a1) of at least 0.15. In this case, the quali-quantitative modulation determined in (b) is only the one induced by the sample of product with respect to the pathophysiological state control, therefore, the control is considered as the reference modulation baseline (e.g., a value of 0) for each of said one or more biological activities. When these conditions are fulfilled, the product is determined as exerting its therapeutic action by regulating the pathophysiological state associated with said pathology.

In the embodiment wherein the product analysed is a beneficial product, step (c) requires that at least 50% of said one or more biological activities are modulated by each product batch with the modulation trend of the network concurring to the healthy state previously determined and each of said modulation values determined in (b) for said cell groups treated with the product of (a) (1) of said at least 50% one or more biological activities differ from the ones of said control group of (a1) of at least 0.15. For beneficial products, the modulation trend of the selected activity in the healthy state is intended as opposite to the modulation trend of the same activity in the pathological state that could derive from the altered physiological state of interest. By way of example, a non pathological alteration of osteocalcin levels can affect adipose tissue and pancreas metabolism and result in metabolic syndrome, and pre-hypertension can lead to vessel damages and the pathologies associated thereto.

The skilled person knows that beneficial products, i.e., products that are suitable for healthy persons and that have the effect/activity of adjuvating homeostasis, are normally used by healthy persons to adjuvate the homeostatic response to altered physiological conditions that are not yet pathological but that could lead, if no action is taken, to the development of a pathology. These products are not considered therapeutic product in that they do not prevent or treat diseases, but they support the organism in maintaining homeostasis, i.e., in maintaining a state of balance in body systems needed for the body to function correctly.

In the method above, hence, the healthy physiological state modulation that the product adjuvating homeostasis (before the initiation of a pathological state) should show in a cell-based assay is determined.

In step (a), in fact, a suitable cell-based assay is a cell-based assay where the cells are healthy cells, and the modulation trend is observed upon treatment with a sample of the product of interest. Alternatively, an altered state can be induced cells and the modulation trend is observed upon treatment with a sample of the product of interest.

Also in this case, the healthy or induced cells are considered as the reference baseline with a value quantifying the modulation (preferably a value of 0) for each of said one or more biological activities.

In all the embodiments above, the user has the possibility to verify that a product either therapeutic of beneficial for health acts by modifying a state and not a single or few activities as each hallmark is modulated in full or in part in the same direction of a healthy physiological state.

This is the first time that a method allowing to define whether a product fulfils the requirement of modulating a state according, e.g., to the DM EU Regulation 2017/745 is provided.

Preferably the one or more biological activities identified in the method of the invention are at least two and where possible at least three.

As already stated above and explained in detail, according to an embodiment of the invention, said parameters and modulation pattern thereof correspond to the genes and the expression pattern thereof underlying the modification detectable in said pathological state for each of said one or more biological activities. Transcriptomic analysis is a particularly suitable tool for assessing gene expression.

A non-limiting example of product comprising or consisting of natural matrices is a product that comprises of consist of one or more of: cut or pulverized plant parts, plant extracts, fractions of said extracts, such as for example the fractions obtained by filtration on a semi-permeable membrane (microfiltration, ultrafiltration, nanofiltration), or those obtained by treatment on adsorption resins, or microorganisms, honey, propolis, silk, wax, plant resins, plant gums, plant exudates, vegetable oils, vegetable essential oils, animal tissues lysates, plant or animal fluids or a mixture thereof.

According to an embodiment of the invention, the examined product can be a medical device as defined in EU Regulation 2017/745 Article 2 (1) indents 1-3 or be a medical device as defined in FDA Section 201(h)(1) of the Food, Drug, and Cosmetic Act USA; or a medicament, or a food supplement, a special food etc.

In an embodiment, although different batches of a product comprising or consisting of one or more natural matrices, i.e., batches produced according to the same protocols including the protocols for obtaining each natural matrix, are necessarily variable in their qualitative and quantitative composition due to their origin from the living (also genetically identical individuals undergo epigenetic regulation), the method of the invention may further comprise a preliminary step in which a qualitative and/or quantitative analysis of the composition of different batches of the same product is performed and select batches that are verified as to be qualitatively and/or quantitatively different in their composition.

Furthermore, the naturality of products comprising or consisting of natural matrices (i.e., the maintenance of characteristics that are typical of the raw natural materials from which such matrices are derived) can also be additionally verified.

As already stated, it is mandatory that the matrices are obtained through non-denaturing processes, so that the components of the matrices are not artificially denatured, however, when desired, the presence of additional indicators of maintenance of features that are present in the original raw material can be verified.

Hence, the method of the invention may further comprise analysing the one or more batches of therapeutic or beneficial product.

In particular, wherein the therapeutic or beneficial product can be analysed for the presence of supramolecular structures, miRNA, and/or isotopic abundance.

In particular, when isotopic abundance is analysed, the isotopic abundance of one or more of C, H, O, N atoms in the product and in the raw materials from which the one or more natural matrices contained therein are compared.

Additionally, the method may comprise testing said therapeutic or beneficial product for biodegradability using an OECD biodegradability test. A non-limiting example of a suitable test is the OECD 310:2014 biodegradability test.

The features above are indicative of a naturality of the product, intended as maintenance of structures and characteristics that are present in living matter and that can therefore interact with a treated subject in a way that respects the natural networks-over-network communication existing in nature. A non-limiting example of supramolecular structures comprises cellular vesicles, exosomes, well as the result of interactions between various components of the matrix in the product in the form as super aggregates that can be examined for their physicochemical and/or biophysical characteristics. This phenomenon has been described and attributed specifically to living matter, which has a drive to self-assemble and self- organize to form supramolecular complex entities (Lehn J-M. Toward complex matter: Supramolecular chemistry and self-organization. PNAS. 2002; 99(8)4763-4768. https://doi.org/10.1073/pnas.072065599.). This inherent complexity leads to the fact that individual molecules within a natural matrix cannot be considered as contained in isolated and fixed packages, since mutual non-covalent and dynamic interactions continuously occur between them, rendering the study of their evolution in time currently factually impossible to determine and validate. Such interactions are intra- and intermolecular and occur both among molecules of the same type as well as among molecules belonging to different chemical classes.

The presence of exosomes can be verified in the product using any commonly available technique; a non-limiting example of such techniques Electron Microscopy (EM), such as transmission Electron Microscopy (TEM) which allows direct visualization of exosomes by imaging their morphology and size; Dynamic Light Scattering (DLS) which measures the size distribution of particles in a solution by analysing the fluctuations in light scattering caused by Brownian motion and can provide information about the size distribution of exosomes in a sample; Nanoparticle Tracking Analysis (NTA) which tracks the movement of individual nanoparticles in a liquid medium using light scattering and Brownian motion analysis; Flow Cytometry which can be used to analyse individual exosomes labelled with fluorescent markers. It allows for quantitative analysis of exosome populations based on size, surface markers, and other characteristics; Fluorescence-activated cell sorting (FACS) can also be employed for sorting exosomes based on specific markers; Western blotting can be used to detect specific proteins present in exosomes; Exosome lysates are separated by gel electrophoresis, transferred to a membrane, and probed with antibodies against exosome markers; Enzyme-Linked Immunosorbent Assay (ELISA) which can quantify specific proteins or nucleic acids in exosome samples. It involves immobilizing exosome-specific antibodies on a solid surface, capturing exosomes from the sample, and detecting them with enzyme-conjugated antibodies; Surface Plasmon Resonance (SPR) which measures changes in the refractive index at the interface between a sensor surface and the sample solution and can be used to study interactions between exosomes and ligands immobilized on the sensor surface; Polymerase Chain Reaction (PCR) which can detect and quantify exosome nucleic acids, including mRNA, miRNA, and DNA. Specific primers targeting these nucleic acids are used for amplification and detection; Mass Spectrometry which can analyse the proteomic and lipidomic profiles of exosomes. These techniques can be used individually or in combination to characterize and detect exosomes in various biological samples. The choice of technique depends on the specific research or diagnostic goals, as well as the characteristics of the exosome sample desired. According to the present invention, the mere detection of the presence of exosome is sufficient. If desired, the exosomes can also be further characterised. The presence of super aggregates can be carried out by selecting one or more target molecule known to be always present in a given natural matrix (e.g., estragole in fennel extracts) and by comparing said molecule within the matrix itself and in isolated or synthesized form.

Isolated or synthesized the molecule will not be in the form of super aggregates whereas in a natural matrix the molecule is expected to be in said form.

The molecule can be quantified in the matrix with standard techniques.

Then several features can be investigated, by way of example, volatility of the target molecule within the matrix and in purified or synthesised form can be investigated and compared; the 1H-NMR spectra of the target molecule within the matrix and in purified or synthesised form can be investigated and compared. A detailed example of these techniques is provided in the example section.

The present inventors, as shown in the example, by comparing synthetic estragole with natural matrices from sweet and bitter fennel, found that the natural matrices showed the presence of estragole within supramolecular structures, and that the estragole in said matrices behaved in a similar manner (thereby maintaining a broad functional resilience, conserved from sweet to bitter fennel) which was completely different from the synthesised estragole, the latter being, poisonous for cells when administered in the same amount of the one embedded in the matrices.

Non limiting examples of suitable techniques for detecting the presence of supramolecular structures in the form of super aggregates in the product under examination comprise: Dynamic Light Scattering (DLS), which measures the fluctuations in the intensity of scattered light caused by the Brownian motion of particles in solution and can provide information about the size distribution of particles, including the presence of larger aggregates; Static Light Scattering (SLS), which measures the intensity of scattered light at a fixed angle and can provide information about the molecular weight and size of particles in solution; Analytical Ultracentrifugation (AUC), which involves spinning samples at high speeds in a centrifuge and measuring the sedimentation velocity or equilibrium sedimentation and can provide information about the size, shape, and molecular weight of particles in solution, including super aggregates; Transmission Electron Microscopy (TEM), which involves imaging samples with a high-energy electron beam and can provide high-resolution images of individual super aggregates, allowing for direct visualization of their morphology and size; Atomic Force Microscopy (AFM) which uses a sharp probe to scan the surface of a sample and can provide high-resolution images of individual super aggregates immobilized on a surface, allowing for detailed characterization of their structure and size; Small-Angle X-ray Scattering (SAXS), which measures the scattering of X-rays by particles in solution and can provide information about the size, shape, and internal structure of super aggregates at the nanometre scale; Fluorescence Correlation Spectroscopy (FCS), which measures the fluctuations in the fluorescence intensity of fluorescently labelled molecules diffusing through a small observation volume and can provide information about the size and dynamics of super aggregates in solution; Fluorescence Resonance Energy Transfer (FRET) which involves measuring the transfer of energy between fluorescently labelled molecules and can be used to study the proximity and interactions between molecules within super aggregates; Size-Exclusion Chromatography (SEC), which separates particles in solution based on their size and can be used to detect and quantify the presence of super aggregates by comparing the elution profile of the sample to that of molecular weight standards; Nanoparticle Tracking Analysis (NTA), which measures the Brownian motion of nanoparticles in solution using a laser beam and can provide information about the size distribution and concentration of super aggregates in solution.

As known to the skilled person, these techniques can be used individually or in combination to detect and comprehensively characterize molecular super aggregates in biological samples or other complex systems.

To be noted, in cases of products that undergo ultrafiltration, exosomes can be removed whereas molecular super aggregates can remain or reassemble in the ultrafiltered final product.

Another indication of "naturality" of a product comprising natural matrices, can be the presence of miRNAs. Also in this case, ultrafiltration can remove miRNAs, but their presence can still be verified in the product before this step.

miRNAs can be detected and optionally quantified and/or characterised according to any method known in the art. A non-limiting example of said methods comprises Reverse Transcription Quantitative Polymerase Chain Reaction (RT-qPCR); Northern Blotting; Next-Generation Sequencing (NGS), which as here represented can be used for miRNA profiling and discovery and involves high-throughput sequencing of small RNA libraries, followed by bioinformatic analysis to identify and quantify miRNAs present in the sample; Microarray Analysis; Lateral flow assays that typically use gold nanoparticles conjugated with miRNA-specific probes that produce visible signals upon binding to target miRNAs; Enzyme-Linked Immunosorbent Assay (ELISA); Digital PCR; or even Biosensor-based approaches, such as surface plasmon resonance (SPR) or electrochemical sensors which can be used for label-free detection of miRNAs in a product, offering rapid and sensitive detection.

A further indication of "naturality" of a product can be C14-activity. When C14-activity is 100% or more (percentage of modern carbon; pMC) obtained by measuring the ratio of radiocarbon in the material relative to a National Institute of Standards and Technology (NIST) modern reference standard (SRM 4990C), can be considered the product from purely bio-based carbon without evidence of a synthetic source. A further indication of the lack of denaturation in the production process of a product, can be the maintenance of the isotopic abundance one or more of C, H, O, N, S atoms, in the product batch under examination with respect to one or more of the raw materials from which the natural matrices comprised in said batch have been obtained.

Of course, this requires the availability of the starting raw material for the production of a specific product batch and is therefore not always feasible.

In any case, a distribution of the isotopic C14 with value of pMC 100 or more, is a strong pointer of naturality of the product, and the maintenance of the isotopic abundance of additional isotopes (e.g. one or more of N, O, H, S), and the absence of isotopes that are not present in the starting raw material is a strong pointer of a good quality of the product i.e., of the fact that the process for obtaining the natural matrices therein did not lead to a denaturation of the original natural material.

The analysis of isotopic abundance is a way to describe matter from an atomic point of view. The isotopic distribution characterizing the starting materials may be influenced by phenomena of different nature which in turn may lead to significant variations in the final products [ISPRA, Quaderni - Laboratorio 2/2018. ISBN 978-88-448-0873-0]. The isotopic composition of a sample is equal to the ratio between the abundance of the heavy and light isotopic forms (example the relationship 13C/12C) and is expressed as a deviation, in parts per thousand, from an internationally identified standard reference material. A positive value of δ indicates that the heavy isotope is enriched in the sample compared to the standard, while a negative value indicates that the heavy isotope in the sample is impoverished.

A marked difference in isotopic abundance ratios of a sample compared to samples known to be of good quality can account for different intra- and intermolecular interactions between the phytochemical classes that make up the matrix regardless of the quantitative profile of the individual species and the different chemical reactivity kinetics. In the first case this phenomenon is defined as geometric isotope effect (GIE) and is due in particular to hydrogen binding. In fact, the length of the hydrogen bond with oxygen is different from the one between deuterium and oxygen. This may involve a different structural rearrangement of both intra- and intermolecular structures. Isotope abundance also changes the kinetics of reactions, known as Kinetic isotope effect (KIE), which can be either primary or secondary, depending on whether the isotope changes the reaction making it faster or slower than the process of interest. It is therefore clear that the KIE establishes a link between a given isotopic abundance of a material and its ability to interact in a reproducible manner with biological systems. It therefore seems plausible that the analysis of isotopic abundance is a possible tool for monitoring the conformity of the product from a physical-chemical and potentially biological point of view.

In general, comparing isotopic abundance in two samples involves determining the relative proportions of isotopes of a particular element in each sample. There are various companies providing this analysis as a service that the skilled person can use. In addition, techniques for calculating isotopic abundance e.g., by mass spectrometry, are part of the common general knowledge and are hence available to the skilled person. In any case, a general approach to compare isotopic abundance in two samples is synthesised below:
Isotope Ratio Mass Spectrometry (IRMS):
IRMS is a powerful technique used to measure the relative abundances of isotopes in a sample. It separates ions based on their mass-to-charge ratio (m/z) and quantifies the abundance of different isotopes. By comparing the isotopic ratios between the two samples, differences in isotopic composition can be assessed. The isotopic rations can hence be calculated for each sample tested and are typically expressed as the ratio of the abundance of one isotope to another. For example, in carbon analysis, the ratio of ^(13)C to ^(12)C is normally compared. In addition, the 14C activity assessment can define the system as 100% natural. This is because the 14C is an unstable isotope (half-life of 5730 years) and, therefore, tends to accumulate in living material while petroleum derivatives have a very low presence/absence of this unstable carbon isotope.

The isotopic abundance of the product and of the raw material are hence compared by percentage deviation and when the value is ≤ 15% it been considered that, the natural matrix maintained the isotopic abundance of the natural raw material of origin. Investigation of the physiological interaction poses challenges not only at the preclinical level but also at clinical level. In particular, investigation of the state of the patient can be achieved through the collection of a combination of more classically punctual endpoints (eg. glycaemia), accompanied by measurements more relevant to the construction of a comprehensive view of the state of the body as capable of monitoring and representing higher order (rather holistic) information such as those resulting from the application of bioimpedenziometry, tongue imaging, pulse signal, breath frequency and depth and odour analysis, and -omic (thus implying e.g. transcriptomics, metabolomics, genomics, microbiomics) profiling of readily available samples such as hair, whole blood, urine, saliva, sweat, tears and other readily available body fluids. The method of the invention can therefore be validated at the clinical level by performing biological assays on samples from cohorts of patients treated with a therapeutic or beneficial product assessed as acting with a physiological mechanism of action with the cell-based method of the invention. The analysis of biological and clinical data collected with patients can allow to achieve a depiction of the state of the patient capable of highlighting the peculiarity of the multipronged interaction with a therapeutic solution acting physiologically, extending the profound degree of innovation of the research and development paradigm revolving around the concept of physiological mechanism of action to the generation of clinical evidence.

The authors of the present invention carried out a clinical trial on Product B "Randomized controlled trial to evaluate the efficacy of a food supplement in subjects with mild cognitive impairment" study, ClinicalTrials.gov Identifier: NCT03581929. In addition to data on the achievement of endpoints classically derived from the geriatric field and relating to the cognitive and physical performance of the patients enrolled in the study, it was possible to analyse a set of biological data ('multiomics' parameters (transcriptomics, metabolomics and microbiomics) by submitting to biological assays samples collected from the groups of patients in order to analyse the same hallmarks and biological activities identified for the assessment of the physiological mode of action of product B by in vitro cell based assay according to the method of the invention and found that the data resulting from the clinical trials were consistent with the data resulting from the in vitro cell based assay, i.e. the tested product modulates a network of biological activities also in clinical trials as expected for a product with a physiological mode of action. The data obtained from biological assays performed on samples taken from the different groups of patients of the trial at different time moments. V1 beginning of the trial, samples from patients not yet treated and

Vn, e.g., V2, V3 etc, samples from patients at specific time points (in the case of product B V2 corresponding to 6 months from the V1 time moment and V3 corresponding to 12 months from the V1 time moment)

The data obtained confirmed that product B acts also also in clinical trials by regulating a network of biological activities that underly the desired beneficial effect. In the case of product B the study was a 12-month single-center randomized controlled study (including 6 months double-blind versus placebo and 6 months open-label). 50 subjects were enrolled (V1) in the study after eligibility evaluation. The purpose of the study was to evaluate whether treatment with Product B could improve cognitive performance in subjects with MCI.

Therefore, the invention also encompasses a method for validating in a clinical set-up the physiological mechanism of action of a product for treating a pathological state or adjuvating homeostasis in an altered physiological state the method comprising: 1) subjecting to one or more biological assays, at least two groups (i) of biological samples from patients treated with a therapeutic or beneficial product exerting its therapeutic or beneficial effect through a physiological mechanism of action as assessed with the cell-based assay method of the invention, said groups of samples having been collected at different time moments V1 and Vn, n being an integer number >1, and at least two groups (ii) of biological samples from patients treated with a therapeutic or beneficial comparator reference product and/or at least two groups (iii) of biological samples from patients treated with a placebo, said groups of samples having been collected at the same time moments of group (i), wherein the read-out of said one or more biological assays is representative of the modulation of one or more biological activities underlying the desired therapeutic or beneficial effect, and determining from said read out:
whether said product exert its therapeutic or beneficial action by regulating a network of said biological activities that underly said pathological state or altered physiological state related to said condition, and wherein,
said product is clinically confirmed to exert its therapeutic or beneficial action through a physiological mechanism of action when said product is shown to regulate a network of said biological activities that underly said pathophysiological or altered physiological state.

The clinical trials do of course also further validate and confirm the therapeutic/beneficial effect already observed at the preclinical stage.

According to the invention, the clinical validation method is carried out on products for which an assessment of physiological mode of action has already been performed with the in vitro method disclosed above.

The samples used in the method are biological samples collected from the patients during the clinical trial at different time moments. V1 represents the samples collected at the beginning of the trial, i.e., before starting the treatments and Vn represents samples collected at time moments n, wherein n is a progressive integer number 1, such as 2, 3, etc. depending on the number of time moments at which the samples are collected. Depending on the pathological or altered physiological state targeted by the product the suitable time moments of collection will vary as the skilled person knows. Normally, the samples are collected during the clinical trials at time moments corresponding to the check visits and controls of the trial itself.

The collected samples are biological samples such as biological fluids and/or tissues. Suitable samples can be breath, urine, saliva, blood, plasma, serum, tears, faeces or other organic fluids, and tissues can be mucosal samples, or biopsies if previewed by the trial, or hair, nail fragments, skin or the like.

According to the invention, the method for validating the physiological mode of action of a therapeutic or beneficial product, the product comprising one or more natural matrices, preferably further comprises: (1) providing a list of hallmarks representative of said pathological state or of the pathological state that can stem from said altered physiological state; (2) identifying for each of said hallmarks one or more biological activities modifications underlying said pathological state thereby pinpointing a network of biological activities whose modulation concurs to said pathological state and (3) identifying one or more parameters whose modulation concurs to the modulation of said one or more biological activities underlying the therapeutic effect of the product tested and determining the modulation trend in terms of up or down modulation of said one or more biological activities, in said network, concurring to said pathological state or to a healthy state.

In a preferred embodiment of the invention, one or more biological assays of the clinical validation are directed to the same list of hallmarks and network of related biological activities provided and identified in the in vitro method of the invention, the one or more parameters whose modulation concurs to the modulation of said biological activities being not necessarily the same identified for the in vitro cell based assay as the biological samples collected during the clinical trial can enable the skilled person to measure different parameters related to the same biological activity of interest (e.g. circulating cytokines for inflammation etc.), the method therefore also comprises determining the modulation trend in terms of up or down modulation of said one or more biological activities, in said network, concurring to said pathological state or to a healthy state.

According to an embodiment, the clinical validation method of the invention, comprises:
(a) performing at least one biological assays on each of said biological samples
(b) determining the modulation or modulation pattern of each of said parameters and calculating the respective modulation values for each of said one or more biological activities for each of said samples;
(c) comparing said modulation values for each of said one or more biological activities thereby providing an average modulation value for each of said activities for each of said groups of samples, or thereby providing the modulation value for each of said activities for each patient in each of said groups of samples wherein:
   the product is shown to exert its therapeutic or beneficial effect through a physiological mechanism of action when:
   at least 50% of said one or more average biological activities or patient's biological activities for each hallmark are modulated in Vn with the modulation trend of the network concurring to the healthy state, and each of said modulation values of Vn determined in (b) of said at least 50% one or more average biological activities or patient's biological activities differ, respectively, from the ones of V1, of at least 0.15.

According to the invention, hence, the modulation values Vn vs V1 can be compared patient by patient, or an average of the modulation values of each group can be calculated and the average Vn values vs. the average V1 values can be calculated.

Progressive Vn modulation values (either patient by patient or average) can be also compared to the corresponding previous Vn modulation values (i.e. values of the group V3 can be confronted with the values of V2 and of V1 etc.).

As stated above, the modulation values calculated for samples of different groups can be compared either patient by patient (patient x sample of V2 vs patient x sample of V1 etc.) or by group average modulation value (average modulation value of V2 vs average modulation value of V1) for each biological activity.

In the examples, the result of the validation in a clinical set-up of the physiological mechanism of action of product B are shown (see figure 23)

In any part of the description and of the claims the expression "comprising" can be substituted with consisting of.

In any part of the description and of the claims the term "value quantifying the modulation" can be substituted with "z-score".

Examples teaching how to carry out the invention, but not intended as limiting it thereto, are provided below.

### EXAMPLES

### 1. COMPOSITIONOF THE TESTED PRODUCTS

### Product A (also Arté GX) (figures 1-5)

| | |
|---|---|
| Centella asiatica dry Leaves | 90% w/w |
| Echinacea purpurea dry Flowers | 10% w/w |

Coextracted in water. 5 different batches used (manufactured with different stocks of starting raw materials and/or with separate production processes of the matrix and of the product, following the same protocol)
□ Batch 20B1955
□ Batch 20I1279
□ Batch 20J1770
□ Batch 20B0596
□ Batch 21E1640

### Product B (figures 7-8 and 23)

| | |
|---|---|
| Melissa officinalis leaves dry extract | 2.5% w/w |
| Royal jelly lyophilised | 2.5% w/w |
| Blueberry dry extract | 0.29% w/w |
| Concentrated blueberry juice | 5% w/w |
| Cynara scolymus L. leaves dry extract | 0.05% w/w |
| Curcuma longa L. roots dry extract | 0.16% w/w |
| Medicago sativa seeds dry extract | 1.6% w/w |
| Panax ginseng roots dry extract | 1.6% w/w |
| Honeycomb dry extract | 1.6% w/w |
| Concentrated apple juice | 44.35% w/w |
| Clarified lemon juice | 0.5% w/w |
| Honey | 30% w/w |
| Deionised water | 6.45% w/w |
| Malpighia emarginata juice | 1% w/w |
| Sambucus nigrum juice | 2% w/w |

### Product C (figures 10-11)

| | |
|---|---|
| Coral calcium powder | 32% w/w |
| Egg shell calcium powder | 30.2% w/w |
| Coral calcium citrate powder | 13% w/w |
| Agaricus bisporus powder | 4.65% w/w |
| Equisetum arvense flowering tops dry extract | 2% w/w |
| Malpighia punicifolia carried by inulin dry extract | 1.50% w/w |
| Cetraria islandica powder | 2% w/w |
| Agave sisalana leaves powder | 12% w/w |
| Acacia senegal powder | 2.15% w/w |

### Product D (figure 11)

36.05% in weight of freeze-dried coextract 1
63.06% in weight of freeze-dried coextract 2
0.89% in weight of freeze-dried Agave sisalana leaves freeze-dried extract.

### Coextract 1

Laurus nobilis leaves 25% w/w
Whitania somnifera roots 25% w/w
Filipendula vulgaris leaves and flowers 25% w/w
Brassica oleracea L. botrytis cymosa seeds 25% w/w
Coextracted in water

### Coextract 2

Cynara scolymus L. leaves 14.30% w/w
Curcuma longa L. roots 42.85% w/w
Tanacetum parthenium L. flowers 42.85% w/w
Coextracted in water

### 2 EXAMPLES OF IN VITRO CELL-BASED ASSAY AND HALLMARKS DEFINITIONS

### 2.1 Cell-based assay representative of Osteoarthritis

An in vitro cellular model capable of recapitulating features of osteoarthritis [1-3] was established by exposing primary human chondrocytes (HC, Cell Application INC 402K-05) to IL1B [5 ng/ml] for 6 hours followed by 24 hours of exposure to five different batches of "Arté-GX" [1.4 mg/ml]:
□ Batch 20B1955
□ Batch 20I1279
□ Batch 20J1770
□ Batch 20B0596
□ Batch 21E1640

Each time one of the batch solutions was added, fresh IL1B [5 ng/ml] was also added to the medium.

### 2.1.1. Time schedule on chondrocyte experiment

The time schedule used for experimental setting is as follows:

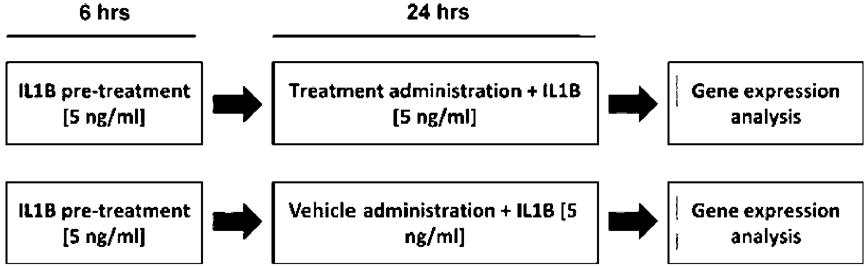

### 2.1.2. Gene expression analysis

At the end of described treatment periods, cells were washed with 100 µl PBS and lysed and collected in RLT buffer (Qiagen, 1053393) added with β-mercaptoethanol (Sigma, M3148) and DX reagent (Qiagen, 19088) for gene expression analysis experiments. Total RNA was extracted from cells lysates using an QIAsymphony RNA Kit (Qiagen,) with the QIAsymphony SP instrument (Qiagen).

The quality and quantity of RNA was determined by A230, A260, A280 and A320 measurements on Varioskan^{™} LUX multimode microplate reader (Thermo Scientific^{™}). Integrity of RNA was checked using a 2100 expert_Eukaryote Total RNA Nano Kit (Agilent). Whole transcriptome expression profile was evaluated using a Human Clariom^{™} S Pico Assay HT (Applied Biosystems, ThermoFisher Scientific) on a GeneTitan MC Instrument (Applied Biosystems, ThermoFisher Scientific), following the manufacturer's instructions. Briefly, 6 ng of total RNA was used to generate cDNA, then fragmented and labelled cDNA was hybridized to a Human Clariom S 96-array plate for 17 h at 45°C. Arrays were washed, stained and then scanned using the GeneTitan MC Instrument (Applied Biosystems, ThermoFisher Scientific) and CEL Intensity files were generated by Affymetrix GeneChip Command Console Software (AGCC, ThermoFisher Scientific).

### 2.1.3. Transcriptomics data analysis

Data analysis was performed using Transcriptomic Analysis Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package, p-value≤0.05).

### 2.1.4. Bioinformatic modelling of experimentally observed transcriptomics data

Ingenuity Pathways Analysis (IPA) (QIAGEN \Inc., https://www.qiagenbioinformatics.com/products/ingenuitypathway-analysis) was used, for each investigated batch to evaluate modulations of gene expression relevant for effects of interest.

IPA is an aggregator of scientific references that allows to search for information on genes/proteins and the construction of networks that predict the behaviour of biological systems according to their gene expression status.

The pathophysiological features of state-of-the-art "Osteoarthritis condition" were considered with particular attention to the following areas involved:

| **Keywords used to interrogate IPA** | **Chosen biological function** |
|---|---|
| Osteoarthritis [osteoarthrosis] | Non-traumatic arthropathy;Osteoarthritis |
| Arthralgia/arthritis | |
| arthropathy | |
| Damage of cartilage tissue | Formation of cartilage tissue |
| cartilage tissue | |
| Inflammation of joint | Inflammation of joint |
| Pain of joint | |

This knowledge was used to interrogate IPA via the "IPA Bioprofiler" tool, using the following key words: osteoarthritis, arthropathy, formation of cartilage tissue, destruction of cartilage tissue, damage of cartilage, connective tissue disorder, inflammation of joint, immune cell trafficking and oxidative stress.

The use of "IPA Bioprofiler" allowed to identify clusters of expressed genes causally linked to each of the identified one or more biological activities and the specific molecular pathway underpinning them. Information concerning the measured gene expression data (Fold change value cut-off ≤-2 and ≥ +2 and p-value≤0.05) induced by each batch was then superimposed on the networks obtained, to define influenced genes and modulation of the connected biofunction.

Modulation of expressed genes were shown in different intensities of blue (signifying down-modulation) or red (signifying up-modulation). The resulting expected calculated impact, based on the literature, on the related biofunctions was determined by "IPA Molecule Activity Predictor" tool (MAP) and resumed in a heatmap visualization.

The colour and intensity thereof were transformed into numerical values.

### 2.1.5. Results

The results of these tests yielded a comparative study of the performance and of the mechanism of action of five different batches of Arté GX. The analysis revealed that while all batches could return reproducible biological effects, it was also possible to identify batch-specific fluctuations in the induced transcriptional pattern. Evidently the induction of slightly different transcriptional patterns still results in the same desirable regulation of one or more biological activities. This is due to a functional resilience caused by redundancy in the interactions between the components of the product and the body, whereby, by virtue of the multifocal mechanism of action, different batches differing in their quali-quantitative composition elicit the same effect (Figs.3 and 4). The analysed batches are therefore considered as having equivalent biological outputs since the induction and repression patterns are conserved.

The different transcriptional patterns and relative biological effects of different batches is to be intended as the hallmark of the inherent variability present in a preparation constituted of biological material. From the results summarised in figure 4 it is evident that the observed transcriptional patterns of the different batches elicit a very reproducible biological (functional resilience) effect leading to a general modification of the pathological processes and of the overall pathological state equivalence for all the batches reported in Figure 4.

### 3. PROTOCOLS

All the protocols used are summarised in the table below.

| | **PRODUCT A** | **PRODUCT B** | **PRODUCT C** | | **PRODUCT D** | |
|---|---|---|---|---|---|---|
| **GENE EXPRESSION ANALYSIS** | *2.1.2. Gene expression analysis* | At the end of described treatment periods, cells were washed with 100 µl PBS and lysed and collected in RLT buffer (Qiagen, 1053393) added with β-mercaptoetha nol (Sigma, M3148) and DX reagent (Qiagen, 19088) for gene expression analysis experiments. Total RNA was extracted from cells lysates using an QIAsymphony RNA Kit (Qiagen,) with the | *2.1.2. Gene expression analysis* | | *2.1.2. Gene expression analysis* | |
| | At the end of described treatment periods, cells were washed with 100 µl PBS and lysed and collected in RLT buffer (Qiagen, 1053393) added with β-mercaptoethano l (Sigma, M3148) and DX reagent (Qiagen, 19088) for gene expression analysis experiments. Total RNA was extracted from cells lysates using an QIAsymphony RNA Kit (Qiagen,) with the QIAsymphony | | At the end of described treatment periods, cells were washed with 100 µl PBS and lysed and collected in RLT buffer (Qiagen, 1053393) added with β-mercaptoethanol (Sigma, M3148) and DX reagent (Qiagen, 19088) for gene expression analysis experiments. Total RNA was extracted from cells lysates using an QIAsymphony RNA Kit (Qiagen,) with the QIAsymphony SP instrument (Qiagen). | | At the end of described treatment periods, tumour fragments were homogenized using Lysing Matrix Tubes J (MP Biomedicals) in RLT buffer (Qiagen, 1053393) added with β-mercaptoethanol (Sigma, M3148) and DX reagent (Qiagen, 19088) for gene expression analysis experiments. Total RNA was extracted from cells lysates using an QIAsymphony RNA Kit (Qiagen,) with the QIAsymphony SP instrument (Qiagen). | |
| | | | The quality and quantity of RNA was determined by A230, A260, A280 and A320 measurements on Varioskan^{™} LUX multimode microplate reader (Thermo Scientific^{™}). Integrity of RNA was checked using a 2100 expert_Eukaryote Total RNA Nano Kit (Agilent). Whole transcriptome expression profile was evaluated using a Human Clariom^{™} S Pico Assay HT (Applied Biosystems, ThermoFisher Scientific) on a GeneTitan MC Instrument (Applied Biosystems, ThermoFisher Scientific), | | The quality and quantity of RNA | |
| | SP instrument (Qiagen). | QIAsymphony SP instrument (Qiagen). | following the manufacturer's instructions. Briefly, 6 ng of total RNA was used to generate cDNA, then fragmented and labelled cDNA was hybridized to a Human Clariom S 96-array plate for 17 h at 45°C. Arrays were washed, stained and then scanned using the GeneTitan MC Instrument (Applied Biosystems, ThermoFisher Scientific) and CEL Intensity files were generated by Affymetrix GeneChip Command Console Software (AGCC, ThermoFisher Scientific). | | was determined by A230, A260, A280 and A320 measurements on Varioskan^{™} LUX multimode microplate reader (Thermo Scientific^{™}). | |
| | The quality and quantity of RNA was determined by A230, A260, A280 and A320 measurements on Varioskan^{™} LUX multimode microplate reader (Thermo Scientific^{™}). Integrity of RNA was checked using a 2100 expert_Eukaryot e Total RNA Nano Kit (Agilent). Whole transcriptome expression profile was evaluated using a Human Clariom^{™} S Pico Assay HT (Applied Biosystems, ThermoFisher Scientific) on a GeneTitan MC Instrument (Applied Biosystems, ThermoFisher Scientific), following the manufacturer's instructions. Briefly, 6 ng of total RNA was used to generate cDNA, then fragmented and labelled cDNA was hybridized to a Human Clariom S 96-array plate for 17 h at 45°C. Arrays were washed, stained and then scanned using the GeneTitan MC Instrument (Applied Biosystems, ThermoFisher | The quality and quantity of RNA was determined by A230, A260, A280 and A320 measurement s on Varioskan^{™} LUX multimode microplate reader (Thermo Scientific^{™}). | | | Integrity of RNA was checked using a 2100 expert_Eukaryote Total RNA Nano Kit (Agilent). Whole transcriptome expression profile was evaluated using a Human Clariom^{™} S Pico Assay HT (Applied Biosystems, ThermoFisher Scientific) on a GeneTitan MC Instrument (Applied Biosystems, ThermoFisher Scientific), following the manufacturer's instructions. Briefly, 6 ng of total RNA was used to generate cDNA, then fragmented and labelled cDNA was hybridized to a Human Clariom S 96-array plate for 17 h at 45°C. Arrays were washed, stained and then scanned using the GeneTitan MC Instrument (Applied Biosystems, ThermoFisher Scientific) and CEL Intensity files were generated by Affymetrix GeneChip Command Console Software (AGCC, ThermoFisher Scientific). | |
| | | Integrity of RNA was checked using a 2100 expert_Eukar yote Total RNA Nano Kit (Agilent). Whole transcriptome expression profile was evaluated using RNA-Seq data obtained with Illumina NextSeq, sequenced in paired-end mode. | | | | |
| | Scientific) and CEL Intensity files were generated by Affymetrix GeneChip Command Console Software (AGCC, ThermoFisher Scientific). | | | | | |

| | **PRODUCT A** | **PRODUCT B** | **PRODUCT C** | | **PRODUCT D** | |
|---|---|---|---|---|---|---|
| **TRANSCRIPTOM ICS DATA ANALYSIS** | *2.1.3. Transcri ptomics data analysis* | Sample was mapped on reference genome using the bioinformatic s tool STAR (version 2.7.0f), with the standard parameters for paired reads. The reference track was the assembly Human obtained from GenCode (HG38). The quantification of transcripts expressed for each sequenced samples was performed using featureCount algorithm. R was used to create a matrix of all genes expressed in all samples with the correspondin g read-counts and the Bioconductor package DESeq2 was used to | *2.1.3. Transcriptomics data analysis* | | *2.1.3. Transcript omics data analysis* | |
| | Data analysis was performed using Transcriptomic Analysis Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package, p-value≤0.05). | | Data analysis was performed using Transcriptomic Analysis Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package, p-value≤0.05). | | Data analysis was performed using Transcriptomic Analysis Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package, p-value≤0.05). | |
| | | normalize the data, using the median of ratio, to perform the differential expression analysis. | | | | |
| | | Quality control check such as Euclidean distances (Heatmap Distances) and Principal component analysis (PCA) were performed among all samples in each condition considered. | | | | |

| | **PRODUCT A** | **PRODUCT B** | **PRODUCT C** | | **PRODUCT D** | |
|---|---|---|---|---|---|---|
| **CELL BASED ASSAY** | *2. In vitro cell assay representative of Osteoarthritis* | Human SH-SY5Y (ATCC^{®} No. CRL-2266^{™}) neuroblastom a cells are a commonly used as a neuronal model, as they maintain several neuron markers [Barbosa, D.J.; Capela, J.P.; de Lourdes Bastos, M.; Carvalho, F. In vitro models for neurotoxicolo gy research. [Tox icol. Res. 2015, 4, 801-842]. The cells were exposed for 24 hours to product B at 1.07 mg/ml in | Human, adipocyte-derived, mesenchymal stem cell lines (hADMSC), capable of differentiating into osteoblasts and mineralize the extracellular matrix (ECM) were used in this study. These cells were obtained during general surgery from three different patients (PA42, PA59 and PA69) (Romagnoli et al, "In Vitro Behaviour of Human Adipose Tissue-Derived Stem Cells on Poly(ε-caprolactone) Film for Bone Tissue Engineering Applications", BioMed Research International, vol. 2015, Article ID 323571, 12 pages, 2015. https://doi.org/10.1155/2 015/323571). These cell lines have been characterized with respect to the main stemness markers of mesenchymal stem cells (CD44, CD105, and STRO1) and by studying their multipotency toward osteogenic phenotypes at the Department of Surgery and Translational medicine of the University of Florence. | | In this study, FaDu cells, a squamous cell carcinoma line (obtained from the American Type Culture Collection-ATCC HTB-43), capable of tumorigenesis in nude mice, were utilized. After inoculation subcutaneously into animals and generating masses, the biopsies were explanted and immediately frozen in liquid nitrogen and then transferred to - 80°C. Upon thawing in Eagle's Minimum Essential Medium (EMEM) (ATCC, 30-2003), they were sectioned into approximately 40 mg pieces and cultured for either | |
| | An in vitro cellular model capable of recapitulating features of osteoarthritis [1-3] was established by exposing primary human chondrocytes (HC, Cell Application INC 402K-05) to IL1B [5 ng/ml] for 6 hours. The cells where hence exposed for 24 hours to five different batches of product A [1.4 mg/ml]: Batch 20B1955 | | hADMSCs were cultured in a growth medium (GM) and grown to 70-80% confluence. | | | |
| | (refernce batch or gold standard) | | | | | |
| | Batch 2011279 Batch 20J1770 Batch 21E1640 Batch 20B0596 Batch 21E1640 Dest Each time one of the batch solutions was added, fresh IL1B [5 ng/ml] was also added to the medium. | the appropriate cell culture medium. | Afterwards, the cells were seeded in 24-well plate at a concentration of 1 × 10⁵ cells/well. After a week, the GM was replaced with osteogenic medium (OM) containing the fluorophore calcein 1 µg/mL and incubated for 28 days with or without product C. The medium with or without the product C was refreshed twice a week. | | 6 hours (for gene expression analysis) or 72 hours (for cytofluorimetric analysis) in a humidified incubator at 37°C, with an atmosphere enriched with 5% CO2, in the absence or presence of PRODUCT D at 50 mg/ml or Cisplatin at 666 µM. | |
| | | | | | For gene expression analysis, at the end of the 6-hour treatment period, tumor fragments were homogenized using Lysing Matrix Tubes J in RLT buffer and followed the previously described process. | |
| | | | | | For cytofluorimetric analysis, at the end of the 72-hour treatment period, the masses were collected and fragmented using a scalpel to create smaller pieces. These fragments underwent further processing to obtain single-cell suspensions using the gentleMACS Octo Dissociator with Heaters (Miltenyi Biotec, 130-096-427) in combination with a tissue dissociation kit (Miltenyi Biotec, 130-095-0929), following the manufacturer's instructions. | |
| | | | | | Cellular debris was **removed using the** MACS Miltenyi Biotec Debris Removal Solution (Miltenyi Biotec, 130-109-398), and red blood cells were eliminated using Red Blood Cell Lysis Solution (10×) (Miltenyi Biotec, 130-095-0929) according to the manufacturer's protocol. | |
| | | | | | Following dissociation, cells were resuspended and stained with CD45 Monoclonal Antibody (30-F11), PE-Cyanine7, eBioscience^{™} (Invitrogen, 25-0451-82) at a concentration of 0.125 µg/test for 30 minutes on ice with occasional shaking. Cells were then washed in cold DPBS (ThermoFisher, 14190-094) at 1200 rpm for 5 minutes. CD45 Monoclonal Antibody was utilized to exclude all hematopoietic cells except mature erythrocytes and platelets. After removing the supernatant, cells were stained with Alexa Fluor^{®} 488 Annexin V/Dead Cell Apoptosis Kit (ThermoFisher, V13241, A13201). | |
| | | | | | Subsequently, the cells were resuspended in 500µl 1X annexin-binding buffer and analyzed using an Attune NxT flow cytometer (Life Technologies). | |
| **DEFINITION OF THE PATHOPHYSIOL OGICAL HALLMARKS OF THE DISEASE WITH WHICH TO INTERROGATE IPA** | The pathophysiologi cal features of state-of-the-art "Osteoarthritis condition" were considered with particular attention to the following areas involved: | The alteration of healthy physiological state features of state-of-the-art "Mild Cognitive Impairment" were considered with particular attention to the following areas involved: | The alteration of healthy physiological state features of state-of-the-art "Osteoporosis" were considered with particular attention to the following areas involved: | | The alteration of healthy physiological state features of state-of-the-art "Cancer" were considered with particular attention to the following areas involved: | |
| | | | | - Remodelling of bone | | |
| | | | | - Osteopenia | | |
| | | | | - Differentiation of osteoblasts | | |
| | | | | - Mineralization | | |
| | -inflammatory process, | | | - Reduction of | - Celldamage | |
| | | | | inflammation | - Energetic metabolism and insulin sensitivity | |
| | -cellular proliferation, | | | - Reduction of adipose tissue | | |
| | -anatomical damage, | | This knowledge was used to interrogate IPA via the "IPA Bioprofiler" tool, using the following keywords: osteoporosis, postmenopausal osteoporosis, calcification of bone, osteoblast and osteoclast differentiation, bone mineral density. | | | |
| | | -Cognition | | | - Epithelial-mesenchym al transition | |
| | -oxidative stress This knowledge was used to interrogate IPA via the "IPA Bioprofiler" tool, using the following keywords: osteoarthritis, arthropathy, formation of cartilage tissue, destruction of cartilage tissue, damage of cartilage, connective tissue disorder, inflammation of joint, immune cell trafficking. | -Activation and viability | | | | |
| | | -Myelination and branching | | | - Growth factor | |
| | | -Reduction of inflammation | | | - Inflammatio n | |
| | | -Skeletal and muscular system function This knowledge was used to interrogate IPA via the "IPA Bioprofiler" tool, using the following keywords: neurodegene ration, cognitive deficit, memory deficit, function of muscle, oxidative stress and inflammatory process. | | | | - Regulation of mitosis/proli feration |
| | | | | | | - Stemness |
| | | | | | This knowledge was used to select and classify IPA core analysis outputs (ie Canonical pathways). | |

### 4. ISOTOPIC ABUNDANCE

The analysis of isotopic abundance was carried out on the batches 20B0596, 20B1955 were prepared from different batches of starting materials with respect to batches 20I1279, 20J1770, 21E1640.

The samples were sent to the Chelab (Tentamus Company) laboratory and tested for stable isotopes as follows:
- δ18O: Method IRMS, UNIT ‰ V-SMOW.
- δ13C: Method QMA-M-01, EA-IRMS, UNIT ‰ V-PDB.

C14- activity was also tested:
- 14C-activity: Method ISO-16620-2;2015 (AMS), UNIT % modern carbon (pMC).

The results were as follows.

Δ ratio of the main isotopes of the co-extract centella-echinacea (product A).

| **Product A Batch** | **δ18O** | **δ13C** | **14C-activity (% modern carbon Pmc)** |
|---|---|---|---|
| | | | |
| **20B0596** | 22,6 | -25.54 | 102,35 |
| **20I1279** | 20,3 | -28,64 | 102,53 |
| **20B1955** | 22,3 | -28,83 | 102,28 |
| **20J1770** | 20,6 | -28,54 | 103,09 |
| **21E1640** | 20,8 | -28,51 | 102,56 |

### (The values in the table includes the percent error according to the official method used)

The measured values for the C14 activity of the samples of Pmc correspond to those for substances from purely bio-based carbon. There is no evidence of a synthetic source in the analysed material. The values δ18O and δ13C are overlaps among batches showing as it is not affected by processing but only by the biological variability of the starting materials, thus identifying high reproducibility of the production process according to conservation of this parameter.

On two batches of co-extract (20J1770 e 21E1640) a study of the isotopic abundance during different steps of the manufacturing process was conducted.

The results are reported in the table below depicting the δ ratio of the main isotopes of the coarse plant raw plant parts of the co-extract centella-echinacea.

| **Description** | **δ¹⁸O/¹⁶O** | **δ¹³C/¹²C** |
|---|---|---|
| **Centella leaves, Batch 20H1282** | +20.8 | -28.57 |
| **Echinacea roots,** | +22.7 | -28.17 |
| **Batch 20F0840** | | |
| **Mixture Centella leaves + Echinacea roots (batches above)** | +20.9 | -28.86 |
| **Batch 21E1640** | +20.8 | -28.51 |
| **Batch 20J1770** | +20.6 | -28.54 |

The assessment of the isotopic abundance of the materials along the production process shows that the production process does not alter the abundance ratios, thus substantiating the fact that the process conserves the native biophysical characteristics of the starting materials.

Analysis for the batches under study showed substantial similarity of values and maintenance of ratios during the manufacturing process.

### 5. miRNAs DETECTION

5.1 evaluation in production intermediate of product A (before ultrafiltration) Biophysical characterization of biological vegetal material includes the evaluation of biological material in production intermediates.

"RIC199EL0, extract_BLEND CENT_ECH EL, batch R20I4716" which corresponds to a production intermediate of product A, i.e., the Centella asiatica and Echinacea water coextract in the proportions depicted in example 1, before ultrafiltration. The presence of RNA has been evaluated both quantitatively and qualitatively. RNA was extracted using a plant matrix specific kit (Rneasy PowerPlant kit) after homogenization with the use of QIAshredder columns before proceeding with the kit extraction protocol. The dimensional distribution of the RNA obtained was performed using Bioanalyzer 2100 with RNA 6000 Nano, RNA 6000 Pico and small RNA kits.

A size distribution of the total RNA between 4 and 150 nt was detected.

Then, to quantitatively assess the total RNA extracted from the sample, a nucleic acid digestion was performed using the New England Biolabs nucleoside digestion Mix kit. The RNA concentration was expressed as total nucleosides by UHPLC-qToF analysis. The Table below reports RNA expressed as total nucleosides (obtained with method N of table above).

| | **BLEND CENT_ECHEL** | |
|---|---|---|
| | **µg/g** | **%** |
| **Guanosine** | 9,20 | 41,39 |
| **Adenosine** | 3,51 | 15,79 |
| **Uridine** | 2,06 | 9,27 |
| **Cytidine** | 7,46 | 33,56 |
| **Total** | 22,23 | 100 |

These observations, in addition to providing a method for validating the biological origin of a matrix, identify an additional degree of both structural and functional complexity of the matrix itself.

### 6. DETECTION OF SUPRAMOLECULAR STRUCTURES IN NATURAL MATRICES

### 6.1 Supramolecular aggregates, passage into vapor phase

Estragole in a natural matrix obtained from fennel shows a reduced tendency to pass into vapor phase with respect to that of the pure standard solution volatility of estragole was investigated by HS-GC-QqQ in bitter fennel seeds alcoholic extract.

Results were generated with two different methods described below:
- Method 1, a screening method optimized for total quantification of the analyte, is based on the use of experimental conditions that force passage into vapor phase of the molecule from the tested solution as it is performed at high temperatures and adding salts (90°C, NaCl added).
- Method 2, is designed to highlight the possible presence of a different rate of passage into vapor phase of the analyte when these originate from different tested solutions. This method is based on the use of lower extraction temperature (30°C) and absence of NaCl. With respect to purified synthetic estragole, quantification of the isolated molecule estragole with the two different methods (methods 1 and 2) shows comparable results

| **Purified synthetic estragole** | **Purified synthetic estragole_Calculated concentration (%)** | **Recovery %** |
|---|---|---|
| **Theoretical concentration (%)** | | |
| **0.02016** | 0.0224 | 111 |

| | | |
|---|---|---|
| Purified synthetic estragole recovery determined by Method 2. | | |

The concentration of pure estragole measured matched indeed with the high level of the calibration curves in both conditions, thus suggesting that the vapour phase transition of the isolated synthetic estragole is not affected by variables independent of method conditions favouring or not the occurrence of such phenomenon. Regarding naturally occurring estragole embedded in bitter fennel extract, the results obtained by applying the two different methods (Method 1 and Method 2) are not equivalent but display a deviation of 49,4% (Method 2) from the theorical result (recapitulated instead by Method 1) (Table 3).

| **(%)** | **SL1_003246_EALC_01 (Method 2)** | **SL1_003246_EALC_01 (Method 1)** | **Deviation (Method 1-Method 2) %** |
|---|---|---|---|
| | **Bitter fennel seeds Alcoholic extract** | **Bitter fennel seeds Alcoholic extract** | |
| **Estragole** | 0.0129 | 0.0261 | 49.4 % |

| | | | |
|---|---|---|---|
| Estragole content in bitter fennel extract measured with Method 2, compared with Method 1 and percentage deviation. | | | |

Estragole embedded in the fennel matrix shows a reduced tendency to pass into vapor phase with respect to that of the pure standard solution, when the less harsh conditions of Method 2 are applied.

Such a reduction, and the conditions in which it is observed, suggests that estragole embedded in the fennel extract may be complexed with other less volatile components of the extract, thus impairing its tendency to pass to the vapor phase.

The data here reported thus support the presence of matrix effect in fennel extracts as they indicate that the physicochemical behaviour of estragole is indeed different when the molecules are isolated (synthetic purified standards) or in the context of the fennel extracts.

6.2 Supramolecular aggregates, Diffusion NMR experiments on natural matrices from bitter and sweet fennel (bitter fennel and sweet fennel extracts) containing estragole.

As first experiment, the presence of estragole in the bitter fennel extracts (Lot 3246) and sweet fennel extract (mixture, Lot 3250) was analysed using 1H NMR spectra with triple signal suppression (figure14) and comparison with the 1H NMR spectra of the reference standard in the extraction solvent mixture (estragole 300 ppm in 50:50 water/bioethanol). Inspection of the spectra reveals two specific peaks in the reference standard (doublets, measured shifts 6.48 ppm and 6.71 ppm; Figure14) that are characteristic of estragole's aromatic protons and present in both the extracts of bitter fennel (Lot 3246) and sweet fennel (mixture, Lot 3250). As a result, presence of estragole was confirmed in both extracts.

Diffusion NMR experiments were performed Ten mono-dimensional spectra were recorded varying the strength of the pulsed- field gradient (G) along the z axis. For each sample, the logarithmic ratio of the resonance intensity (I) at the given strength of the pulsed-field gradient (G) over the initial resonance intensity (I0), recorded in absence of G, was plotted versus G2 as reported in Figure 15 (a, b, c). A similar experimental procedure was also applied to a sample of ethanol as calibration reference. Parameters of the linear regression model are reported in the table below. The inspection of the table below shows almost identical parameters for the calibration reference sample, suggesting no change in viscosity of the solution and the high reliability and reproducibility of the three in dependent experiments over the reference standard and samples 3250, 3246.

| **Sample** | **Intercept (c)** | **Slope (a)** | **Regression Coefficient (R²)** |
|---|---|---|---|
| Reference Standard (exp. # 1) | 0,04 ± 0,01 | -52,2 ± 0,7 | 0,998 |
| Ethanol (exp.#1) | 0,03 ± 0,01 | -77,23 ± 0,06 | 1,0 |
| Sample 3250 (exp.#2) | 0,01 ± 0,01 | -29,1 ± 0,8 | 0,993 |
| Ethanol (exp.#2) | 0,0289 ± 0,0006 | -76,18 ± 0,03 | 1,0 |
| Sample 3246 (exp.#3) | 0,00 ± 0,01 | -36,1 ± 0,4 | 0,999 |
| Ethanol (exp.#3) | 0,033 ± 0,004 | -76,3 ± 0,1 | 1,0 |

| | | | |
|---|---|---|---|
| Parameters of the linear regression model [y = a(x) +c]. | | | |

The slope (coefficient a) of the obtained straight lines is directly proportional to the translational self- diffusion coefficient (D). The calibration with a substance (ethanol) of which the translational self-diffusion coefficient (D) or van der Waals radius (RvdW (Ethanol) = 2,130 Å) is known, enables the determination of the proportionality constant between D or RvdW and the slope (coefficient a), according to equation 2. It should be noticed that it is the hydrodynamic radius (RH) to be determined from equation 2 and not the van der Waals radius since the actual radius of the diffusion particle (estragole) is extended by the solvent shell (RH = RvdW + ΔRshell). Hence, the hydrodynamic radius (RH) and hydrodynamic volume (VH) of estragole was determined for each sample of the study. These values are reported the table below and are indicative of the aggregation states of estragole in the three samples.

| **Sample** | **Estragole R_{H} (Å)** | **Estragole V_{H} (Å³)** | *** Estragole *R_{vdddd}* (Å)** | *** Estragole *V_{vdddd}* (Å³)** |
|---|---|---|---|---|
| Reference Standard (exp.#1) | 3,57 | 19 | 3,484 | 177 |
| | 9 | 2 | | |
| Sample 3250 (exp.#2) | 6,30 | 105 | 3,484 | 177 |
| | 6 | 0 | | |
| Sample 3246 (exp.#3) | 5,17 | 58 | 3,484 | 177 |
| | 4 | 0 | | |

| | | | | |
|---|---|---|---|---|
| Hydrodynamic radius (R_{H}) and hydrodynamic volume (V_{H}) of estragole in the three samples. *Theorical vanbder Waals volume *(V_{vdddd}Å)* and radius *(R_{vdddd}Å)* of estragole were determined by quantum mechanical calculations. | | | | |

Although the shape of estragole in the global minimum conformation is a non-spheric particle as shown in Figure 16, the closeness of the theorical van der Waals radius (R_{v*d*W}) of estragole to the experimentally determined hydrodynamic radius (R_{H}) of the reference standard suggests a good approximation of the NMR diffusion study to yield qualitatively reasonable results. Moreover, the similarity of values between the hydrodynamic volume (V_{H} = 192 Å³) of estragole and the volume of a theoretical spheric-like particle (V_{vdW} = 177 Å³) defined by the van der Waals radius of estragole suggests the lack of self-aggregation state in the reference standard. In comparison with the hydrodynamic volume of estragole in the reference standard (estragole 300 ppm; V_{H} = 192 Å³), the hydrodynamic volume of estragole in the extract of bitter fennel (sample 3246, estragole 300 ppm; V_{H} = 580 Å³) is significantly larger, suggesting the presence of an aggregation state containing this molecule in a range from 1 to 3 potential units. In the case of the extract of sweet fennel (sample 3250, estragole 30 ppm; V_{H} = 1050 Å³), the hydrodynamic volume of estragole is much larger, suggesting the presence of a distinct aggregation state with respect to the bitter fennel in size, shape and content that seems to be independent from or inversely related to the concentration of estragole which is ten times lower herein than in sample 3246.

This study shows that the extracts of bitter fennel (sample 3246) and sweet fennel (sample 3250) have different aggregation states containing estragole that seem to be independent or inversely related to its concentration (sample 3246, estragole 300 ppm; sample 3250, estragole 30 ppm). Although estragole is not able to self-aggregate as shown by the similarity of values between the hydrodynamic volume (V_{H} = 192 Å³) of estragole in the reference standard and the volume of a theoretical spheric- like particle (V_{vdW} = 177 Å³) defined by the van der Waals radius of estragole, additional and yet undefined contents in the extracts of bitter fennel and sweet fennel promote distinct aggregation states that can be associated to different chemical, physical, biophysical and biological properties of the two extracts as supramolecular complex entities.

### 6.3 Detection of supramolecular structures in a natural matrix

### DLS description:

The method of dynamic light scattering (DLS) is the most common measurement technique for particle size analysis in the nanometre range. DLS measures the hydrodynamic size of particles, by the mechanism of light scattering from a laser that passes through solution and analyses modulation of the intensity of scattered light as a function of time. Brownian motion of particles correlates with their hydrodynamic diameter. The smaller the particle, the faster it will diffuse than a larger one and the DLS instrument will generate a correlation function that is mathematically linked with particle size and its time-dependent light scattering capacity.

DLS has been used to measure the particle size of dispersing colloidal samples, to study the stability of formulations, and to detect the presence of aggregation or agglomeration. This method is also most suitable for analysing the size distribution of already isolated exosomes and micro vesicles.

### Sample preparation:

Freeze-dried extract of sweet fennel (FIN0SE#AQEC batch 23G0989) was dispersed at a concentration of 4 mg/ml in 0.22 µm filtered demineralized water.

After dispersion sample was vortexed for 2 minutes to provide complete dispersion. Sample was analysed at three different conditions:
- Unfiltered
- After filtration with 0.45 µm Nylon syringe filter
- After filtration with 0.1 µm Nylon syringe filter

0.1 µm filtration was performed on 0.45 µm filtered sample.

Before each filtration step, sample dispersions were vortexed for 30 seconds. Filtered dispersions were left at rest at room temperature for about 15 minutes and then analysed after a gentle manual stirring.

### Results:

Correlogram evaluation provides data quality information about DLS results. The graph shows the progress of a correlation function (y-axis) during time (x-axis) that should have a sigmoid shape. The intercept value on y-axis is linked to signal/noise ratio, i.e., how much scattering signal from the sample is reaching detectors and is successfully separated from background noise. The closer intercept is to 1, the better is the signal to noise ratio.

The position (on the x-axis, time) of the inflection point of the correlogram is related to the size of the particles: the longer the decay time of the correlation, the larger the particle size. The slope of this section of the curve is related to the polydispersity index (PdI) and thus to the dispersion of the sample size population: the steeper the decay, the less dispersed the particle size and the lower the PdI value. Finally, the section of the curve following the inflection point is related to the presence of large particles. In general, the absence of large particles and aggregates is evidenced by a curve tending to zero in this area.

Figure 13 reports the average particle size distributions (Fig 13a) and the average correlation functions, Fig. 13b (obtained from the 3 measurements). Averaged results of Z-Average, PdI and Peaks results obtained from 3 repeated measurements are reported in the figure 12c. Z-Average is the intensity weighted mean diameter, PdI is the polydispersity index.

Water dispersion provided the formation of large particles over 1 µm. The noisy right part of correlogram of unfiltered water dispersion suggests the presence of not characterized larger particles. Filtration caused a clear shift in the correlogram and size distribution. Both filtered water dispersion samples provided bi-modal distribution with small particles/structures of about and under 100 nm. Data quality of filtered samples is good.

### 6.4 Detection of exosomes in intermediate of product A (before ultrafiltration) Preparation of ultracentrifuged samples

The starting sample from which the ultra centrifugate is prepared was weighed and resuspended in a volume of VIB or vesicles isolation buffer (20 mM MES; 2 mM CaCl2; 100 mM NaCl, pH 6.0) maintaining the ratio of 5mL of buffer per 500 mg of sample. The sample was incubated under stirring at room temperature for 20-24h to promote solubilization. After incubation, several centrifuges were performed all at 4°C and at increasing speeds to isolate particles between 30-500 nm in size. The T-1250 rotor (Thermo Fisher Scientific, 11718-5) and the Thermo Scientific^{™} Sorvall^{™} WX+ ultracentrifuge (Thermo Fisher Scientific^{™} 75000080, no. 15342177) were used for the ultracentrifugation. The sample was centrifuged at 700 x g for 20 minutes; the pellet was discarded while the supernatant was filtered through a 0.45 µm filter and centrifuged at 10000 x g for 30 minutes. The supernatant was then moved to ultracentrifugation tubes and centrifuged at 40000 x g for 70 minutes. The supernatant was discarded while the pellet was resuspended in VIB and centrifuged again at 40000 x g for 70 minutes. The pellet was finally resuspended in 600 µL of 25 mM Trehalose in PBS and stored at 4°C if to be used within 24 h or at -30°C for long-term storage. Extracellular vesicles staining and flow cytometry analysis

Extracellular vesicles from samples were stained with CellMask^{™} Green Plasma Membrane Stain (ThermoFisher Scientific #C37608) according to the manufacturer's instructions and quantified on a Attune NxT flow cytometer. Briefly, 27 µL of exosomes were added to 3 µL of CellMask^{™} Green Plasma Membrane Stain (10x) per sample for 30 minutes at 37°. Then, added 170 µL of PBS (double filtered at 0.22 µm) to each sample and read them. To remove any non-specific events in the flow cytometry analysis, PBS stained with CellMask^{™} Green Plasma Membrane Stain was used as a negative control and Fluorescent Exosome Standards (Novus Biologicals #NBP3-11691) was used as a positive control. Moreover, each sample was analyzed without staining to exclude any autofluorescence.

### Analysis of micro vesicle size and concentration by NanoSight

The Malvern NanoSight NS300 uses the technology of Nanoparticle Tracking Analysis (NTA). This unique technology utilizes the properties of both light scattering and Brownian motion to obtain the size distribution and concentration measurement of particles in liquid suspension. A laser beam is passed through the sample chamber, and the particles in suspension in the path of this beam scatter light in such a manner that they can easily be visualized via 20x magnification microscope onto which is mounted a camera. The same protocol was carried out on samples of product C.

### RESULTS

The samples, for example "RIC199EL0, extract_BLEND CENT_ECH EL, batch R20I4716" which corresponds to a production intermediate of product A, i.e., the Centella asiatica and Echinacea water coextract in the proportions depicted in example 1, before ultrafiltration, were analyzed using Flow Cytometry as follows: the values of PBS marked by CellMask^{™} Green Plasma Membrane Stain were excluded from the area of interest because they were considered as blank samples. In the Fluorescent Exosome Standards, a large number of elements were identified in the same area being considered positive.

The samples then analyzed considering the number of elements present in the same area. The number of extracellular vesicles identified in the "RIC199EL0, extract_BLEND CENT_ECH EL, batch R20I4716" sample was 5.43 x10⁵. The number of extracellular vesicles identified in the product C sample was 2.7 x10⁶.

### 7. BIODEGRADABILITY TEST ACCORDING TO OECD 310: 2014

Biodegradation is the process by which organic substances are decomposed by microorganisms into the simplest natural building blocks (e.g., CO₂, H₂O, and NH₃) that can be integrated into natural biogeochemical cycles.

The evaluation of the biodegradability of chemicals is one of the main issues in environmental risk assessment. Biodegradability tests are designed to evaluate, under batch conditions, a chemical substance as the sole carbon source for the survival of microfauna. The ready biodegradation tests (RBT) are the basis of the integrated testing strategy on pure substance biodegradation. They are a series of tests (from no. 301A to 301F and no. 310) proposed by the Organization for Economic Co-operation and Development (OECD). Microorganisms and the tested substance are usually incubated in a buffered pH 7 medium containing N, P, and a trace element (named the "mineral medium"). The kinetics of biodegradation is monitored during at least 28 days by the evaluation of metabolic parameters such as oxygen consumption, carbon dioxide production, or dissolved organic carbon consumption. The RBT measures ultimate biodegradability, or complete biodegradation and a chemical can be classified as readily biodegradable if has passed one of the RBTs.

The term primary biodegradation indicates the structural modification of a substance caused by a biological event, which results in the loss of a specific property of that substance. It can be calculated from supplemental chemical analysis for parent compounds made at the beginning and end of the tests (OECD 301, 310).

On the test item, "Product A batch 20B1955", the analysis of inorganic carbon for the evaluation in an aqueous medium of the aerobic biodegradability has been performed following screening method described in OECD 310: 2014.

For this purpose, the amount of developed inorganic carbon has been measured and reported in comparison to the blank.

The test has been done using the test item with a fixed concentration of organic carbon Samples have been kept at the temperature of 20 + 1 °C for the whole period of the test (28 days).

50 mL of the three replications of the blank and the solutions contained the test item, at the beginning and at the end of the test, have been send to the Sponsor, as requested by him.

On the basis of results obtained, interpreted in accordance with OECD 310:2014, the tested batch was considered readily biodegradable in aerobic conditions.

### 7.1 Assay execution

For assay execution the following preparations have been done using deionized water. Unused have been eliminated at the end of the test.

### Test Sample

The elemental analysis of the test item has been determined by REDOX SnC using CHN analyser.

The sample has been used with the measured initial concentration of 22.79 mg/L of organic carbon (TOC).

### Reference substance

Considering Sodium benzoate as reference substance using its molecular formula, a starting nominal concentration of 24.07 mg/L of organic carbon (TOC) has been used.

### Blank

As blank has been used water containing culture Medium added with the inoculum of the muds.

### Assay sample + Reference substance preparation

Test sample and reference substance have been put together to have a final concentration given by the sum of organic carbon (TOC) of the 2 substances (see attachment N. 2 for results).

### Abiotic Check

Test item has been put together with 50 mg/L HgClz to check a possible abiotic degradation.

### Conditions of the Assay

The inoculum has been added in all bottles (see bottle preparation), closed and incubation continued under stirring in darkness at 20 + 1 °C for 28 days. Data recording is performed continuously by a validated informatic monitoring system (Labguard Evisense).

At least 1 hour before every determination of inorganic carbon (TIC) the reaction in one bottle of blank, one of reference substance and one of the test item has been stopped with the addition of 6 mL of sodium hydroxide (1 M).

Triplicate bottles have been analysed to detect inorganic carbon (IC) at each check point, except at the end of the test where five bottles have been analysed.

### 7.2 Results

The calculation of biodegradation is done at each sampling time for the reference substance, test sample and blank.

Total biodegradation has been calculated using the equation: $Dt = \left(ICt-ICb/TOCi\right) \times 100$

Where:
Dt = total biodegradation calculated with reference to values of blank, expressed in percentage.
ICt = concentration of inorganic carbon, in mg, produced by biodegradation of the test item.
ICb = concentration of inorganic carbon, in mg, produced by biodegradation in blank.
TOCi= concentration of organic carbon of the test item, in mg, added at the beginning. The result of biodegradability is expressed as the percentage ratio between the value of inorganic carbon measured and the value of organic carbon added at the beginning. The value of inorganic carbon is the highest value recorded during 28 days of experimental period.

### Interpretation of the results

A substance is considered readily biodegradable when its level of biodegradability is at least 60%, within 10d window during the test.

If, at day 28, the biodegradation in the bottle contained both the test sample and the reference substance referred to biodegradation in the bottle with reference substance result < 25%, it may be assumed that the test substance doesn't inhibit the activity of the inoculum.

If there is a significant increase (>10%) in the inorganic carbon content of the abiotic bottles over the test period, it may be concluded that abiotic degradation of the test substance has occurred, and this must be considered in the calculation of the percentage of biodegradation of the test item.

### Validity Criteria

The test is considered valid when:
- the percentage of average degradation of the reference substance is more than 60% after 14 days of incubation.
- the mean amount of total inorganic carbon (TIC) present in the blank at the end of the test is < 3 mg C/L.

### Results

Validity criteria of the test are satisfied.

The mean amount of TIC present in the blank controls at the end of the test is < 3 mg C/L.

Abiotic degradation didn't occur because amount of TIC during the test in the abiotic bottles has been lower than 10% (see table below).

| TIC T=1 day (ppm) | TIC T=28 day (ppm) | % INCREASE (<10%) |
|---|---|---|
| 2.762 | 2-478 | <10% |

The biodegradation percentage of the test item during the test is detailed in attachment N. 2 and summarized in the following table.

The trend of the inorganic carbon and related biodegradation percentages (see table below - column % of biodegradation Reference substance + Test item) in the bottle containing both the test sample and the reference substance confirms the absence of an inhibitory effect of the test sample on inoculum at the concentration at which it was applied in the test (5%).

| CHECK POINT (days) | % Biodegradation Ref. substance | % Biodegradation Test item | % Biodegradation Ref. substance + test item |
|---|---|---|---|
| 0 | 0 | 0 | 0 |
| 1 | 31 | 5 | 29 |
| 2 | 52 | 30 | - |
| 12 | 78 | 65 | - |
| 21 | 80 | 72 | - |
| 28 | 77 | 80 | 81 |

The trend of the degradation of the test item and the reference substance is reported in Figures 17 and 18.

On the basis of results obtained, interpreted in accordance with OECD 310:2014, the test item Product A is considered readily biodegradable in aerobic conditions.

### 8. DETAILED ANALYSIS OF PRODUCT A BY TARGETED METABOLOMICS

To appreciate whether the final matrix constituting product A is characterized by the matrix effect, a series of analyses to grasp the product's features on different aspects was carried out on the batches reported above. A targeted metabolomics analysis capable of identifying most of such molecular components, was carried out on different batches of the product (see figure 5) together with the other analysis reported herein. The product, as described above, consist of two vegetal matrices assembled and resulting in a final new vegetal matrix. Several analytical techniques have been used to identify and quantify compounds belonging to the main classes present in plants. Although metabolomic analysis does not allow to appreciate the dynamic changes within the component of the matrix, it allows a "picture" of the composition in the moment the analysis is carried out.

In the following analysis each individual component (plant metabolite) is specifically researched, for this reason the analysis is called "targeted metabolomics". This analysis allows to capture a frame on the qualitative data, by determining the chemical compounds present in the material, and quantitative data, by defining the concentrations of each compound in the material.

For product A, a qualitative and quantitative characterization of as many primary and secondary metabolites as possible was carried out using an "omic" approach, the targeted metabolomics analysis, based on the use of multiple analytical methodologies. The analytical methods used for the chemical characterization of each batch are described below. The most appropriate analytical techniques have been adopted based on the chemical nature of the classes of compounds present. The analysis with chromatographic methods combined with different detection techniques (e.g., GC and LC each combined with a suitable detector), made it possible to identify and quantify, as appropriate, the organic compounds. The inductively coupled plasma analysis using a single quadrupole mass spectrometer (ICP-MS) or an optical emission spectrometer (ICP-OES) made it possible to establish the levels of elements present, while the anions were determined by ion chromatography and conductivity detector. Other gravimetric methods were used for the determination of classes of substances non-quantifiable by means of chromatographic methods.

The table below summarizes all the methods used.

| **Class of Compounds** | **Characteristics** | **Type of Method** | **Short description** |
|---|---|---|---|
| **Fibres** | Insoluble Fibres | Gravimetric | Method AOAC 991.42 **(A1)** |
| | Soluble fibres | Gravimetric | Method AOAC 993.19 **(A2)** |
| | FOS, Fructans | HPAEC-PAD | Extraction of FOS and Fructans followed by enzymatic digestion; analysis by ion chromatograph equipped with a pulsed amperometric detector. **(A3)** |
| **Polysaccharides** | Polysaccharides ≥ 20.000 Dalton | HPLC-RID | Extraction of water-soluble polysaccharides and analysis by HPLC equipped with molecular exclusion column and refractive index detector.(B) |
| **Water** | Loss on drying | Gravimetric | Method ISTISAN 1996/34 (pag 7-10). **(C)** |
| **Phenols** | Phenols polar | UHPLC-qToF | Sample extraction and reverse phase chromatography analysis by UHPLC coupled to a quadrupole-time-of-flight mass spectrometer. **(D1)** |
| | | HPLC-UV | Sample extraction and reverse phase chromatography analysis by HPLC coupled to UV-VIS detector. **(D2)** |
| **Terpenes** | Terpenes polar | UHPLC-qToF | Sample extraction and reverse phase chromatography analysis by UHPLC coupled to a quadrupole-time-of-flight mass spectrometer. **(E1)** |
| | | HPLC-UV | Sample extraction and reverse phase chromatography analysis by HPLC coupled to UV-VIS detector. **(E2)** |
| | Terpenes apolar | GC-TQ | Headspace analysis of the sample by gas chromatograph coupled to a triple quadrupole mass spectrometer. **(E3)** |
| | Sterols | GC-FID | Analysis of the sample by gas chromatograph coupled to a flame ionization detector, after derivatization and transformation into trimethylsilyl ethers. **(E4)** |
| **Organic acids** | Organic acids mono-, di-, tri-carboxylic | HPLC-UV | Sample extraction and analysis by HPLC coupled to UV detector. **(F)** |
| **NITROGEN Compounds** | Nitrogen-containing substances, total | Kjeldahl | Method AOAC 920.53. **(G)** |
| **Lipids** | Fats, total | Gravimetric | Method ISTISAN 1996/34 (pag 39-40). **(H1)** |
| | Fatty acids derivatives | GC-TQ | Headspace analysis of the sample by gas chromatograph coupled to a triple quadrupole mass spectrometer. **(H2)** |
| | Fatty acids | HPLC-UV | Sample extraction and analysis by HPLC coupled to UV detector. **(H3)** |
| | Fatty acids | GC-FID | Analysis of the sample by gas chromatograph coupled to a flame ionization detector, after derivatization. **(H4)** |
| **Sugars and Derivatives** | Monosaccharides | IC-PAD | Sample extraction and analysis by ion chromatograph coupled to pulsed amperometric detector. **(I)** |
| | Disaccharides | IC-PAD | Sample extraction and analysis by ion chromatograph coupled to pulsed amperometric detector. **(I)** |
| **Other Organic Compounds** | Other organic compounds, apolar | GC-TQ | Headspace analysis of the sample by gas chromatograph coupled to a triple quadrupole mass spectrometer. **(L)** |
| **Inorganic Compounds** | Elements | ICP-MS, ICP-OES | Acid mineralization of the sample in a microwave oven and analysis of the conductively induced plasma by means of a single quadrupole mass spectrometer, or optical emission spectrometer. **(M1)** |
| | | IC-CD | Sample extraction and analysis by ion chromatograph coupled to conductometric detector. **(M2)** |
| | Anions | IC-CD | Sample extraction and analysis by ion chromatograph coupled to conductometric detector. **(M2)** |
| **Total RNA** | Nucleosides | UHPLC-qToF | After extraction and digestion of RNA from the sample a reverse phase chromatography analysis was applied by UHPLC coupled to a quadrupole-time-of-flight mass spectrometer. **(N)** |

The results, that are summarised in the table below, show an appreciable composition variability of each batch and underline the impossibility to recapitulate the properties of the matrix as the sum of its single components. The work performed and reported herein (see cell-based assay results) together with the data below, demonstrates that the biological effect elicited by product cannot be recapitulated by the sum of the effects elicited by the single molecular components but is the result of interconnections and interactions among the components: the matrix effect. This translates into the impossibility to formally define a structure-activity relationship (SAR) according to the principles canonically applied to APIs.

| **Method** | **COMPOUNDS** | **21E1640 (%)** | **20B1955 (%)** | **20I1279 (%)** | **20J1770 (%)** | **20B0596 (%)** | ***21E1640 (d%)*** | ***20I1279 (d%)*** | ***20J1770 (d%)*** | ***20B0596 (d%)*** |
|---|---|---|---|---|---|---|---|---|---|---|
| **G** | **NITROGEN-CONTAINING SUBSTANCES, Total** | 5,1 | 5,1 | 5 | 4,7 | 0,76 | 0,00 | 1,96 | 7,84 | 85,10 |
| | **FIBERS, Total** | 5,4 | 2,3 | 1,5 | 1,7 | 1,8 | 134,78 | 34,78 | 26,09 | 22,26 |
| A2 | **SOLUBLE FIBERS, Total** | 5,4 | 2,3 | 1,5 | 1,1 | 1,5 | 134,78 | 34,78 | 52,17 | 34,78 |
| A3 | **FRUCTOLIGOSAC CHARIDES, Total** | <LdQ | <LdQ | <LdQ | 0,6 | 0,2880 | / | / | / | / |
| A1 | **INSOLUBLE FIBERS, Total** | <LdQ | <LdQ | <LdQ | <LdQ | <LdQ | / | / | / | / |
| B | **MACROMOLECUL ES > 20000 (POLYSACCHARID ES), Total** | <LdQ | <LdQ | <LdQ | <LdQ | <LdQ | / | / | / | / |
| C | **WATER, Total** | 3,4 | 5,4 | 4 | 4,10 | 4,50 | 37,04 | 25,93 | 24,07 | 16,67 |
| | **PHENOLS, Total** | 4,57 | 3,45 | 3,20 | 3,33 | 2,97 | 32,30 | 7,37 | 3,48 | 13,95 |
| | **FLAVONOIDS, Total** | 1,80 | 1,49 | 1,49 | 1,74 | 1,18 | 20,48 | 0,47 | 16,39 | 20,81 |
| D1 | Rottlerin | <LdQ | <LdQ | <LdQ | <LdQ | / | / | / | / | / |
| D1 | Phloridzin | nq | nq | nq | / | / | / | / | / | / |
| | **FLAVANONES, Total** | 0,01 | 0,0052 | 0,0096 | nq | nq | 92,31 | 84,62 | / | / |
| D1 | Flavanomarein | 0,01 | 0,0052 | 0,0096 | nq | nq | 92,31 | 84,62 | / | / |
| | **FLAVONES, Total** | 0,0258 | 0,02 | 0,0297 | 0,023858 | 0,0134 | 29,00 | 48,50 | 19,29 | 33,00 |
| D1 | 3',4',5,5',6,7-Hexamethoxyflav one | <LdQ | 0,0002 | 0,0002 | 0,000119 | 0,0002 | / | 0,00 | 40,55 | 5,52 |
| D1 | Diosmin | nd | <LdQ | <LdQ | <LdQ | / | / | / | / | / |
| D1 | Homoorientin | 0,009 | 0,0063 | 0,0128 | 0,003715 | 0,0037 | 42,86 | 103,17 | 41,03 | 41,65 |
| D1 | Isoschaftoside | 0,0008 | 0,001 | 0,0009 | 0,004852 | 0,0022 | 20,00 | 10,00 | 385,20 | 124,34 |
| D1 | Isovitexin | 0,0012 | 0,001 | 0,0011 | 0,000779 | 0,0006 | 20,00 | 10,00 | 22,10 | 40,89 |
| D1 | Linarin | nd | nd | nd | <LdQ | / | / | / | / | / |
| D1 | Luteolin-7-O-beta-D-glucoside (Cynaroside) | nd | nd | nd | <LdQ | / | / | / | / | / |
| D1 | Luteolin-7-O-glucuronide | nd | nd | nd | <LdQ | <LdQ | / | / | / | / |
| D1 | Nobiletin | <LdQ | <LdQ | <LdQ | <LdQ | <LdQ | / | / | / | / |
| D1 | Orientin | 0,0025 | 0,0026 | 0,0018 | nq | nq | 3,85 | 30,77 | / | / |
| D1 | Schaftoside | 0,0067 | 0,0044 | 0,0081 | 0,008796 | 0,0036 | 52,27 | 84,09 | 99,91 | 19,06 |
| D1 | Vicenin-2 | 0,0038 | 0,0032 | 0,0029 | 0,004069 | 0,0021 | 18,75 | 9,38 | 27,16 | 33,32 |
| D1 | Vitexin | 0,0018 | 0,0013 | 0,0019 | 0,001528 | 0,0010 | 38,46 | 46,15 | 17,55 | 25,67 |
| | **FLAVONOLS, Total** | 1,7619 | 1,4669 | 1,4458 | 1,707743 | 1,1682 | 20,11 | 1,44 | 16,42 | 20,36 |
| D1 | Isorhamnetin-3-O-glucoside | 0,0022 | 0,0018 | 0,002 | 0,001514 | 0,0016 | 22,22 | 11,11 | 15,92 | 11,28 |
| D1 | Isorhamnetin-3-O-rutinoside | <LdQ | <LdQ | <LdQ | <LdQ | / | / | / | / | / |
| D1 | Kaempferol | 0,0062 | 0,0136 | 0,0104 | 0,002918 | 0,0030 | 54,41 | 23,53 | 78,54 | 77,76 |
| D1 | Kaempferol-3-O-glucoside | 0,0342 | 0,1187 | 0,0743 | 0,032471 | 0,0729 | 71,19 | 37,41 | 72,64 | 38,56 |
| D1 | Kaempferol-3-O-glucuronide | 0,3409 | 0,2751 | 0,2855 | 0,618309 | 0,5180 | 23,92 | 3,78 | 124,76 | 88,31 |
| D1 | Kaempferol-3-O-rutinoside | 0,0076 | 0,0119 | 0,0092 | 0,00195 | 0,0035 | 36,13 | 22,69 | 83,61 | 70,69 |
| D1 | Quercetin | 0,0201 | 0,0352 | 0,0159 | 0,012575 | 0,0192 | 42,90 | 54,83 | 64,27 | 45,36 |
| D1 | Quercetin-3-O-glucopyranoside (Isoquercetin) | 0,0694 | 0,0871 | 0,0694 | 0,025885 | 0,0598 | 20,32 | 20,32 | 70,28 | 31,36 |
| D1 | Quercetin-3-O-glucuronide | 1,2456 | 0,8651 | 0,9198 | 0,985214 | 0,4558 | 43,98 | 6,32 | 13,88 | 47,31 |
| D1 | Rutin | 0,0357 | 0,0584 | 0,0593 | 0,026907 | 0,0313 | 38,87 | 1,54 | 53,93 | 46,34 |
| | **ISOFLAVONES, Total** | <LdQ | <LdQ | <LdQ | <LdQ | <LdQ | / | / | / | / |
| D1 | Genistin | <LdQ | <LdQ | <LdQ | <LdQ | <LdQ | / | / | / | / |
| | **PHENOLIC ACIDS, Total** | 0,0745 | 0,0707 | 0,0923 | 0,077983 | 0,1091 | 5,37 | 30,55 | 10,30 | 54,35 |
| D1 | Protocatechuic acid | 0,0745 | 0,0707 | 0,0923 | 0,077983 | 0,1091 | 5,37 | 30,55 | 10,30 | 54,35 |
| D1 | Vanillic acid | nd | nd | nd | <LdQ | / | / | / | / | / |
| | **PHENYLPROPAN OIDS, Total** | 2,682 | 1,872 | 1,606 | 1,490576 | 0,929 | 43,25 | 14,23 | 20,39 | 50,38 |
| | **COUMARINS, Total** | 0,0149 | 0,0168 | 0,0164 | 0,007515 | 0,0022 | 11,31 | 2,38 | 55,27 | 86,90 |
| D1 | Aesculin | 0,0051 | 0,0043 | 0,0112 | 0,006231 | 0,0047 | 18,60 | 160,47 | 44,90 | 8,63 |
| D1 | Esculetin | 0,0098 | 0,0125 | 0,0052 | 0,001284 | 0,0053 | 21,60 | 58,40 | 89,73 | 57,65 |
| D1 | Fraxin | <LdQ | <LdQ | <LdQ | / | <LdQ | / | / | / | / |
| | **HYDROXYCINNA MIC ACIDS, Total** | 2,667 | 1,856 | 1,590 | 1,442046 | 0,9068 | 43,75 | 14,33 | 22,29 | 51,13 |
| D1 | 3,5-Dicaffeoylquinic acid | 1,043 | 0,5573 | 0,516 | 0,4833 | 0,2254 | 87,15 | 7,41 | 13,28 | 59,56 |
| D1 | 4,5-Dicaffeoylquinic acid | 0,1822 | 0,1601 | 0,1568 | 0,058033 | 0,0547 | 13,80 | 2,06 | 63,75 | 65,85 |
| D1 | Caffeic acid | 0,0219 | 0,0168 | 0,0274 | 0,008865 | 0,0089 | 30,36 | 63,10 | 47,23 | 47,04 |
| D2 | Caftaric acid | 0,2338 | 0,2825 | 0,2005 | 0,1949 | 0,2198 | 17,24 | 29,03 | 31,01 | 22,19 |
| D2 | Chicoric acid | 0,282 | 0,2948 | 0,17 | 0,1748 | 0,2142 | 4,34 | 42,33 | 40,71 | 27,34 |
| D2 | Chlorogenic acid | 0,8213 | 0,4702 | 0,4644 | 0,4276 | 0,2931 | 74,67 | 1,23 | 9,06 | 37,66 |
| D2 | Echinacoside | <LdQ | <LdQ | <LdQ | / | / | / | / | / | / |
| D1 | Ferulic acid | 0,0081 | 0,0047 | 0,0055 | 0,003602 | nq | 72,34 | 17,02 | 23,36 | / |
| D1 | Neochlorogenic acid | 0,0626 | 0,0658 | 0,0445 | 0,090946 | 0,0598 | 4,86 | 32,37 | 38,22 | 9,18 |
| D1 | Rosmarinic acid | 0,0082 | 0,0034 | 0,0026 | nq | / | 141,18 | 23,53 | / | / |
| D1 | Verbascoside | 0,0043 | <LdQ | 0,0019 | / | nq | / | / | / | / |
| | **PHLOROGLUCIN OLS, Total** | nq | nq | nq | <LdQ | / | / | / | / | / |
| D1 | Phloroglucinol carboxylic acid | nq | nq | nq | <LdQ | / | / | / | / | / |
| | **SALICYLATES, Total** | 0,0118 | 0,0162 | 0,0136 | 0,0145 | 0,006522 | 27,16 | 16,05 | 10,50 | 59,74 |
| D1 | Salicylic acid | 0,0118 | 0,0162 | 0,0136 | 0,0145 | 0,006522 | 27,16 | 16,05 | 10,50 | 59,74 |
| | **TERPENES, Total** | 13,63 | 15,80 | 16,59 | 14,92 | 14,93 | 13,74 | 5,02 | 5,57 | 5,49 |
| | **MONOTERPENES, Total** | 0,000408 | 0,000329 | 0,00042 | 0,000305 | 0,0008 | 23,96 | 29,09 | 7,15 | 143,18 |
| | **MONOTERPENE ALCOHOLS, Total** | 0,000355 | 0,00028 | 0,00037 | 0,000278 | 0,000309 | 26,59 | 32,76 | 0,95 | 10,17 |
| E3 | alpha-Terpineol | <LdQ | <LdQ | <LdQ | <LdQ | <LdQ | / | / | / | / |
| E3 | Carvacrol | 4,49E-05 | 5,08E-05 | 5,1E-05 | 2,61E-05 | 9,34E-05 | 11,67 | 0,85 | 48,61 | 83,83 |
| E3 | Eucalyptol | <LdQ | nd | nd | <LdQ | <LdQ | / | / | / | / |
| E3 | Linalool | 0,00031 | 0,00023 | 0,00032 | 0,000252 | 0,000216 | 35,06 | 39,82 | 9,59 | 6,13 |
| | **MONOTERPENE HYDROCARBONS, Total** | 5,27E-05 | 4,85E-05 | 5,2E-05 | 2,77E-05 | <LdQ | 8,70 | 7,90 | 42,97 | / |
| E3 | 4-Cymene | 5,27E-05 | 4,85E-05 | 5,2E-05 | 2,77E-05 | <LdQ | 8,70 | 7,90 | 42,97 | / |
| E3 | alpha-Pinene | nd | nd | <LdQ | <LdQ | <LdQ | / | / | / | / |
| E3 | alpha-Terpinene | <LdQ | <LdQ | <LdQ | <LdQ | <LdQ | / | / | / | / |
| E3 | beta-Pinene | <LdQ | <LdQ | <LdQ | <LdQ | <LdQ | / | / | / | / |
| E3 | Camphene | <LdQ | <LdQ | <LdQ | <LdQ | <LdQ | / | / | / | / |
| E3 | gamma-Terpinene | <LdQ | <LdQ | <LdQ | <LdQ | <LdQ | / | / | / | / |
| E3 | Myrcene | <LdQ | <LdQ | <LdQ | <LdQ | <LdQ | / | / | / | / |
| E3 | Sabinene | <LdQ | nd | <LdQ | <LdQ | <LdQ | / | / | / | / |
| E3 | Terpinolene | <LdQ | <LdQ | <LdQ | <LdQ | <LdQ | / | / | / | / |
| | **TRITERPENES, Total** | 13,627 | 15,799 | 16,592 | 14,919 | 14,9314 | 13,74 | 5,02 | 5,57 | 5,49 |
| | **PHYTOSTEROLS, Total** | 0,001141 | 0,001289 | <LdQ | / | / | 11,45 | / | / | / |
| E4 | 24-Methylenecholesterol | 0,000056 | <LdQ | <LdQ | / | / | / | / | / | / |
| E4 | 7-Dehydrocholesterol | <LdQ | <LdQ | <LdQ | / | / | / | / | / | / |
| E4 | beta-Sitosterol | 0,000435 | 0,000578 | <LdQ | / | / | 24,65 | / | / | / |
| E4 | Brassicasterol | <LdQ | <LdQ | <LdQ | / | / | / | / | / | / |
| E4 | Campestanol | 0,000181 | 6,19E-05 | <LdQ | / | / | 192,73 | / | / | / |
| E4 | Campesterol | 0,000149 | 0,000125 | <LdQ | / | / | 19,35 | / | / | / |
| E4 | Cholesterol | 8,78E-05 | 0,000075 | <LdQ | / | / | 17,32 | / | / | / |
| E4 | Clerosterol | <LdQ | <LdQ | <LdQ | / | / | / | / | / | / |
| E4 | delta-5-Avenasterol | <LdQ | 7,1E-05 | <LdQ | / | / | / | / | / | / |
| E4 | delta-5,23-Stigmastadienol | <LdQ | <LdQ | <LdQ | / | / | / | / | / | / |
| E4 | delta-5,24-Stigmastadienol | <LdQ | <LdQ | <LdQ | / | / | / | / | / | / |
| E4 | delta-7-Avenasterol | <LdQ | <LdQ | <LdQ | / | / | / | / | / | / |
| E4 | delta-7-Campesterol | <LdQ | <LdQ | <LdQ | / | / | / | / | / | / |
| E4 | delta-7-Stigmastenol | 0,000074 | 0,000107 | <LdQ | / | / | 30,79 | / | / | / |
| E4 | delta-7,9(11)-Stigmastadienol | <LdQ | <LdQ | <LdQ | / | / | / | / | / | / |
| E4 | Sitostanol | 0,000041 | 9,55E-05 | <LdQ | / | / | 57,01 | / | / | / |
| E4 | Stigmasterol | 0,000116 | 0,000175 | <LdQ | / | / | 33,72 | / | / | / |
| | **SAPOGENINS, Total** | 0,0091 | 0,0154 | 0,0176 | <LdQ | 0,0054 | 40,91 | 14,29 | / | 64,94 |
| E1 | Asiatic acid | 0,001 | 0,0033 | 0,0024 | <LdQ | <LdQ | 69,70 | 27,27 | / | / |
| E1 | Madecassic acid | 0,0081 | 0,0121 | 0,0152 | <LdQ | 0,005364 | 33,06 | 25,62 | / | 55,67 |
| | **SAPONINS, Total** | 13,617 | 15,782 | 16,574 | 14,919 | 14,926 | 13,72 | 5,02 | 5,47 | 5,42 |
| E2 | Asiaticoside | 5,526 | 7,121 | 6,935 | 6,143 | 6,801 | 22,40 | 2,61 | 13,73 | 4,49 |
| E2 | Madecassoside | 8,091 | 8,661 | 9,639 | 8,776 | 8,125 | 6,58 | 11,29 | 1,33 | 6,19 |
| | **SESQUITERPENES , Total** | <LdQ | nd | <LdQ | <LdQ | nd | / | / | / | / |
| E3 | Farnesol | <LdQ | nd | <LdQ | / | / | / | / | / | / |
| | **ORGANIC ACIDS, Total** | 10,37 | 14,48 | 9,94 | 9,03 | 15,07 | 28,38 | 31,31 | 37,66 | 4,07 |
| | **MONOCARBOXYL IC ACIDS, Total** | <LdQ | 2,03 | <LdQ | 1,431 | 1,627 | / | / | 29,51 | 19,85 |
| F | Acetic acid | <LdQ | 1,07 | <LdQ | 0,151 | 0,177 | / | / | 85,89 | 83,46 |
| F | Formic acid | <LdQ | <LdQ | <LdQ | <LdQ | <LoQ | / | / | / | / |
| F | Lactic acid | <LdQ | 0,96 | <LdQ | 1,28 | 1,45 | / | / | 33,33 | 51,04 |
| | **DICARBOXYLIC ACIDS, Total** | 8,2088 | 10,4075 | 7,6748 | 4,7048 | 10,05 | 21,13 | 26,26 | 54,79 | 3,44 |
| F | Fumaric acid | 0,067 | 0,0446 | 0,067 | 0,13 | 0,136 | 50,22 | 50,22 | 191,48 | 204,93 |
| F | Malic acid | 8,08 | 2,2 | 7,54 | 4,55 | 5,75 | 267,27 | 242,73 | 106,82 | 161,36 |
| F | Succinic acid | <LdQ | 8,14 | <LdQ | <LdQ | 4,13 | / | / | / | 49,26 |
| F | Tartaric acid | 0,0618 | 0,0229 | 0,0678 | 0,0248 | 0,034 | 169,87 | 196,07 | 8,30 | 48,47 |
| | **TRICARBOXYLIC ACIDS, Total** | 2,16 | 2,04 | 2,27 | 2,89 | 3,39 | 5,88 | 11,27 | 41,67 | 66,18 |
| F | Citric acid | 2,16 | 2,04 | 2,27 | 2,89 | 3,39 | 5,88 | 11,27 | 41,67 | 66,18 |
| | **SUGARS AND DERIVATIVES, Total** | 11,42 | 1,85 | 10,78 | 13,34 | 7,46 | 516,13 | 481,66 | 619,61 | 302,37 |
| | **MONOSACCHARI DES, Total** | 11,423 | 1,854 | 10,784 | 10,0247 | 7,16 | 516,13 | 481,66 | 440,71 | 286,19 |
| I | Fructose | 5,707 | 1,854 | 5,38 | 6,20591 | 4,5300 | 207,82 | 190,18 | 234,73 | 144,34 |
| I | Galactose | 1,369 | <LdQ | 1,315 | 1,30558 | 1,1000 | / | / | / | / |
| I | Glucose | 4,347 | <LdQ | 4,089 | 2,51321 | 1,2900 | / | / | / | / |
| | **DISACCHARIDES, Total** | <Ldq | <Ldq | <Ldq | 3,31694 | 0,30 | / | / | / | / |
| I | Lactose | <Ldq | <Ldq | <Ldq | <LdQ | / | / | / | / | / |
| I | Maltose | <Ldq | <Ldq | <Ldq | 1,54 | / | / | / | / | / |
| I | Sucrose | nq | nq | nq | 1,77694 | 0,30 | / | / | / | / |
| | **LIPIDS, Total** | 0,200 | 0,170 | 0,190 | 4,03E-05 | 5,88E-05 | 17,64 | 11,76 | 99,98 | 99,97 |
| | **FATTY ACIDS, Total** | 0,2 | 0,17 | 0,19 | <LdQ | 5,88E-05 | 17,65 | 11,76 | / | 99,97 |
| H4 | Decanoic acid | nd | nd | 0,01 | / | / | / | / | / | / |
| H4 | Dodecanoic acid | 0,02 | nd | nd | / | / | / | / | / | / |
| H4 | Linoleic acid | nd | nd | nd | / | / | / | / | / | / |
| H4 | Linolenic acid | nd | nd | nd | / | / | / | / | / | / |
| H4 | Myristic acid | nd | nd | nd | / | / | / | / | / | / |
| H4 | Octanoic acid | nd | nd | 0,01 | / | / | / | / | / | / |
| H4 | Oleic acid | nd | nd | nd | / | / | / | / | / | / |
| H4 | Palmitic acid | 0,1 | 0,1 | 0,09 | / | / | 0,00 | 10,00 | / | / |
| H3 | Propionic acid | <LdQ | <LdQ | <LdQ | / | / | / | / | / | / |
| H4 | Stearic acid | 0,08 | 0,07 | 0,08 | / | / | 14,29 | 14,29 | / | / |
| | **FATTY ACID DERIVATIVES, Total** | 9,38E-05 | 9,37E-05 | 9,5E-05 | 4,03E-05 | 5,88E-05 | 0,09 | 1,20 | 57,05 | 37,24 |
| | **FATTY ACID ESTERS, Total** | 9,38E-05 | 9,37E-05 | 9,5E-05 | 4,03E-05 | 5,88E-05 | 0,09 | 1,20 | 57,05 | 37,24 |
| H2 | Ethyl palmitate | 9,38E-05 | 9,37E-05 | 9,5E-05 | 4,03E-05 | 5,88E-05 | 0,09 | 1,20 | 57,05 | 37,24 |
| | **OTHER ORGANIC COMPOUNDS, Total** | 0,00069 | 0,00048 | 0,00069 | 0,00064 | 0,000361 | 44,12 | 44,78 | 33,41 | 24,64 |
| | **AROMATIC COMPOUNDS, Total** | 0,000691 | 0,00048 | 0,00069 | 0,00064 | 0,000361 | 44,12 | 44,78 | 33,41 | 24,64 |
| | **AROMATIC ALCOHOLS, Total** | 0,000665 | / | 0,00067 | 0,000622 | 0,000361 | / | / | / | / |
| L | para,alpha,alpha-Trimethylbenzyl alcohol | 0,000665 | 0,000466 | 0,00067 | 0,000622 | 0,000361 | 42,67 | 44,25 | -33,35 | 22,46 |
| L | Benzaldehyde | <LdQ | <LdQ | <LdQ | <LdQ | <LdQ | / | / | / | / |
| | **STYRENES, Total** | 2,62E-05 | 1,35E-05 | 2,2E-05 | 1,82E-05 | <LdQ | 94,07 | 62,94 | 35,28 | / |
| L | Dimethylstyrene | 2,62E-05 | 1,35E-05 | 2,2E-05 | 1,82E-05 | <LdQ | 94,07 | 62,94 | 35,28 | / |
| | **INORGANIC COMPOUNDS, Total** | 21,85 | 26,21 | 21,45 | 22,98 | 27,44 | 16,63 | 18,18 | 12,34 | 4,68 |
| | **ANIONS, Total** | 4,6313 | 6,34 | 4,4114 | 5,463733 | 5,53 | 26,95 | 30,42 | 13,82 | 12,78 |
| M2 | Nitrate | 0,0013 | <LdQ | 0,0014 | 0,092379 | <IdQ | / | / | / | / |
| M2 | Nitrite | <LdQ | <LdQ | <LdQ | <LdQ | <IdQ | / | / | / | / |
| M2 | Phosphate | 1,63 | 1,74 | 1,55 | 1,529767 | 1,4700 | 6,32 | 10,92 | 12,08 | 15,52 |
| M2 | Sulfate | 3 | 4,6 | 2,86 | 3,841587 | 4,0600 | 34,78 | 37,83 | 16,49 | 11,74 |
| | **MACROELEMENT S, Total** | 17,05 | 19,66 | 16,87 | 17,32551 | 21,9985 | 13,28 | 14,18 | 11,88 | 11,89 |
| M1 | Calcium | 1,745388 | 2,544027 | 1,75523 | 1,847967 | 2,8998 | 31,39 | 31,01 | 27,36 | 13,98 |
| M2 | Chloride | 4,67 | 5,32 | 4,36 | 5,496178 | 4,8700 | 12,22 | 18,05 | 3,31 | 8,46 |
| M1 | Magnesium | 1,270564 | 1,533499 | 1,25715 | 1,233837 | 1,6827 | 17,15 | 18,02 | 19,54 | 9,73 |
| M1 | Phosphorus | 0,624007 | 0,619255 | 0,60815 | 0,579524 | 0,7223 | 0,77 | 1,79 | 6,42 | 16,64 |
| M1 | Potassium | 5,728856 | 6,134733 | 5,80766 | 5,657116 | 7,6743 | 6,62 | 5,33 | 7,79 | 25,10 |
| M1 | Sodium | 2,326488 | 2,741546 | 2,40561 | 2,510884 | 3,8194 | 15,14 | 12,25 | 8,41 | 39,31 |
| M1 | Sulfur | 0,683772 | 0,767168 | 0,6784 | 1,56 | <LdQ | 10,87 | 11,57 | 103,35 | / |
| | **MICROELEMENTS** , **Total** | 0,137432 | 0,165006 | 0,12715 | 0,134181 | 0,1918 | 16,71 | 22,94 | 18,68 | 16,24 |
| M1 | Chromium | 3,09E-05 | 0,000102 | 0,00011 | 8,77E-05 | 0,0007 | 69,77 | 9,86 | 14,21 | 567,60 |
| M1 | Cobalt | 0,000127 | 0,000122 | 0,00013 | 0,000149 | 0,0001 | 4,08 | 8,87 | 22,19 | 7,45 |
| M1 | Copper | 0,000718 | 0,000226 | 0,00091 | 0,000945 | 0,0009 | 217,47 | 303,07 | 317,91 | 287,93 |
| M2 | Fluoride | NQ | NQ | NQ | <Ldq | nq | / | / | / | / |
| M1 | Iron | 0,000372 | 0,000777 | 0,00326 | 0,002209 | 0,0027 | 52,15 | 319,33 | 184,10 | 243,53 |
| M1 | Manganese | 0,128456 | 0,156247 | 0,11479 | 0,121585 | 0,1722 | 17,79 | 26,53 | 22,18 | 10,20 |
| M1 | Molybdenum | <LdQ | <LdQ | <LdQ | <LdQ | <LdQ | / | / | / | / |
| M1 | Nickel | 0,000873 | 0,000727 | 0,00106 | 0,00111 | 0,0008 | 20,11 | 45,89 | 52,65 | 3,32 |
| M1 | Selenium | <LdQ | <LdQ | <LdQ | <LdQ | <LdQ | / | / | / | / |
| M1 | Tin | <LdQ | <LdQ | <LdQ | <LdQ | <LdQ | / | / | / | / |
| M1 | Vanadium | <LdQ | <LdQ | <LdQ | <LdQ | <LdQ | / | / | / | / |
| M1 | Zinc | 0,006855 | 0,006804 | 0,00688 | 0,008096 | 0,0145 | 0,75 | 1,09 | 18,99 | 112,71 |
| | **OTHER ELEMENTS, Total** | 0,035826 | 0,049164 | 0,03758 | 0,056604 | 0,0508 | 27,13 | 23,57 | 15,13 | 3,33 |
| M1 | Aluminum | 0,00085 | 0,001453 | 0,00176 | <LdQ | 0,0021 | 41,48 | 20,92 | / | 45,49 |
| M1 | Antimony | <LdQ | <LdQ | <LdQ | <LdQ | <LdQ | / | / | / | / |
| M1 | Arsenic | <LdQ | <LdQ | <LdQ | <LdQ | <LdQ | / | / | / | / |
| M1 | Barium | 0,00455 | 0,005308 | 0,00493 | 0,005361 | 0,0055 | 14,29 | 7,16 | 0,98 | 4,28 |
| M1 | Boron | 0,002366 | 0,002735 | 0,00244 | 0,002466 | 0,0028 | 13,49 | 10,73 | 9,84 | 3,87 |
| M3 | Bromide | 0,00019 | 0,00027 | 0,00018 | 0,016442 | / | 29,63 | 33,33 | 5989,72 | / |
| M1 | Cadmium | <LdQ | <LdQ | <LdQ | <LdQ | <LdQ | / | / | / | / |
| M1 | Gadolinium | <LdQ | <LdQ | <LdQ | <LdQ | <LdQ | / | / | / | / |
| M1 | Gallium | <LdQ | <LdQ | <LdQ | <LdQ | <LdQ | / | / | / | / |
| M1 | Gold | <LdQ | <LdQ | <LdQ | <LdQ | <LdQ | / | / | / | / |
| M1 | Iridium | <LdQ | <LdQ | <LdQ | <LdQ | <LdQ | / | / | / | / |
| M1 | Lead | <LdQ | <LdQ | <LdQ | <LdQ | nd | / | / | / | / |
| M1 | Lithium | 2,79E-05 | 4,26E-05 | 3,6E-05 | 3,27E-05 | 0,0000 | 34,59 | 16,02 | 23,32 | 12,05 |
| M1 | Lutetium | <LdQ | <LdQ | <LdQ | <LdQ | <LdQ | / | / | / | / |
| M1 | Mercury | <LdQ | <LdQ | <LdQ | <LdQ | <LdQ | / | / | / | / |
| M1 | Rubidium | 0,016844 | 0,022168 | 0,01703 | 0,019779 | 0,0230 | 24,02 | 23,20 | 10,78 | 3,70 |
| M1 | Silver | <LdQ | <LdQ | <LdQ | <LdQ | <LdQ | / | / | / | / |
| M1 | Strontium | 0,008375 | 0,013771 | 0,00873 | 0,01 | 0,0125 | 39,19 | 36,62 | 27,38 | 9,23 |
| M1 | Tellurium | <LdQ | <LdQ | <LdQ | <LdQ | <LdQ | / | / | / | / |
| M1 | Thallium | <LdQ | <LdQ | <LdQ | <LdQ | <LdQ | / | / | / | / |
| M1 | Thorium | <LdQ | <LdQ | <LdQ | <LdQ | <LdQ | / | / | / | / |
| M1 | Titanium | 0,002622 | 0,003414 | 0,00248 | 0,002523 | 0,0047 | 23,20 | 27,35 | 26,09 | 39,02 |
| M1 | Tungsten | <LdQ | <LdQ | <LdQ | <LdQ | <LdQ | / | / | / | / |
| M1 | Uranium | <LdQ | <LdQ | <LdQ | <LdQ | <LdQ | / | / | / | / |
| M1 | Ytterbium | <LdQ | <LdQ | <LdQ | <LdQ | <LdQ | / | / | / | / |

| | | | | | | | | | | |
|---|---|---|---|---|---|---|---|---|---|---|
| Note 1. Gray boxes indicate chemical macro classes. Note 2. %= compound concentration expressed as percentage of the whole matrix. Note 3. (d%)= percentage deviation: (\|Gold (%)-Test(%)/*(Gold {%)) x 100).* Note 4. The term "Total" refers to the sum of the value of the various compounds which forms the corresponding group. Note 5. <LdQ= below the limit of quantification. Note 6. nd = compound not detected Note 7. nq= compound not quantifiable. Note 8. /*= compound not reported.* | | | | | | | | | | |

The results show that there are more or less marked quantitative fluctuations of the individual chemical classes in the five batches of co-extract. These fluctuations, if taken as the reference parameter, would lead to an *a priori* expectation for these batches to have a different therapeutic action. The analysis here reported demonstrates that, despite the biological activity is maintained across all the different batches assessed, none of the single molecular components identified would respect the criteria set for a single API, thus demonstrating that the matrix cannot be considered as a compilation of APIs. As seen above, the same therapeutic action is preserved in every batch (functional resilience).

The product, through a physiological mechanism of action is capable to evoke, in biological systems, the same reaction relevant for the intended use.

This also highlights the fact that there are both structural and functional redundancy mechanisms maintained in products comprising or consisting of natural matrices, of a functional resilience (reaching the same result notwithstanding the individual differences among individuals of a same species) that is typical of the living matter. The compliance with the functional resilience requirement was assessed in the same way for all the products tested (A, B, C and D).

### 9. NETWORK ANALYSIS

A network analysis of the pathologies treated by products A-D was performed, the data obtained show how the natural matrices-based products tested are able to influence the body on a systemic scale.

### Specifically, relative to:

### Product A

- the condition in a pathological state (Figure 19 Panel A): body adipose tissue has been shown to be related to the development and progression of knee OA. Systemic adipose tissue is significantly and negatively associated with the presence and progression of gonarthrosis. By hindering the gonarthrosis process, motor activity is increased and consequently a decrease in systemic fat (responsible for biomechanical damage and the release of pro-inflammatory factors that contribute to gonarthrosis itself). [Chang J, Liao Z, Lu M, Meng T, Han W, Ding C. Systemic and local adipose tissue in knee osteoarthritis. Osteoarthritis Cartilage. 2018].
- the condition when treated with drug reference (Figure 19 Panel B): the beneficial effect is only partial as only the inflammatory and pain-relieving component of the pathology is influenced.
- the state when treated with product A (Figure 19 Panel C): the beneficial effect is the result of a harmonization of the anti-inflammatory and stimulating effect of cell proliferation, all to counteract tissue damage as well as pain.

### Product B

- the condition in a pathological state (Figure 20 Panel A): Mild Cognitive Impairment (MCI) is defined as a clinical condition characterized by mild cognitive deficits, with little impact on the functional state. It has been widely demonstrated that an increase in the levels of inflammation mediators and in the number of activated glial cells is considered to be a critical risk factor responsible for impaired neurogenesis, synaptic plasticity and cognitive deficits. An increased susceptibility to the long-term effects of inflammation would contribute to the decrease in cognitive activities and motor performance observed in both aging and neurodegenerative diseases, as MCI (Di Benedetto S, Müller L, Wenger E, Düzel S, Pawelec G. Contribution of neuroinflammation and immunity to brain aging and the mitigating effects of physical and cognitive interventions. Neuroscience & Biobehavioral Reviews. 2017; 75:114-128. Doi: 10.1016/j.neubiorev.2017.01.044.)
- the condition when treated with drug reference (Figure 20 Panel B): drug reference, a well-known acetylcholine esterase (AChE) inhibitor, shows to exert neuroprotective effects, to improve synaptic plasticity and to ameliorate functionality of CNS but it does not lead to any systemic action.
- the state when treated with product B (Figure 20 Panel C): the product B is capable of acting at systemic level, improving a neuroprotective effect, restoring synaptic plasticity, mediating improvement of cognitive activities and physical performance and also reducing chronic inflammation.

### Product C

- the condition in a pathological state (Figure 21 Panel A): dysfunctional and inflamed adipose tissue leads to an imbalance in bone homeostasis, negatively affecting the competition for mesenchymal stem cell reserves to induce osteoblast, osteoclast or adipocyte differentiation, and unphysiologically shifting this phenomenon towards osteoclast differentiation. This leads to a reduction in the number of mature osteoblasts, resulting in an inability to ensure proper mineralisation of the cellular matrix with loss of bone activity. In conditions of dysregulation of lipid metabolism and inflammation of the adipose tissue, a dysfunctional loop is established between the adipose tissue and the bone, leading to the accumulation of adipocytes and osteoclasts, disadvantaging the osteoblastic component and aggravating bone fragility. Bone is also an organ with endocrine activities and can therefore influence events in other tissues on a systemic level, for example through the secretion of osteocalcin (OCN), which promotes insulin secretion by the pancreas, insulin sensitivity in peripheral organs, such as muscle, and regulation of overall energy expenditure (Fukumoto S, Martin TJ. Bone as an endocrine organ. Trends Endocrinol Metab. 2009 Jul;20(5):230-6. Doi: 10.1016/j.tem.2009.02.001. Epub 2009 Jun 21. PMID: 19546009).
- the condition when treated with drug reference (Figure 21 Panel B): drug reference is only able to reduce the quantity of the adipocytes.
- the state when treated with product C (Figure 21 Panel C): product C, by interacting with the mesenchymal stem pool in bone and adipose tissue, is able to recapitulate all the necessary elements to restore the correct bone turnover, which is beneficial for the formation of a solid and functional bone structure, and, at a systemic level, to restore the balance of metabolic dysregulation and reduce inflammation.

### Product D

- the condition in a pathological state (Figure 22 Panel A): Systemic inflammation is closely associated with the clinical symptoms of the tumour, indicating its presence and progression. Cytokines, inflammatory proteins and immune cells are present and easily detectable in systemic circulation [Dolan RD, Lim J, McSorley ST, Horgan PG, McMillan DC. The role of the systemic inflammatory response in predicting outcomes in patients with operable cancer: Systematic review and meta-analysis. Sci Rep. 2017; Dolan RD, McMillan DC. The prevalence of cancer associated systemic inflammation: Implications of prognostic studies using the Glasgow Prognostic Score. Crit Rev Oncol Hematol. 2020; Roxburgh CS, McMillan DC. Cancer and systemic inflammation: treat the tumour and treat the host. Br J Cancer. 2014].
- the condition when treated with drug reference (Figure 22 Panel B): drug reference is not able to counteract the systemic inflammation.
- the state when treated with product D (Figure 22 Panel C): product D is able to modulate specific biological activities capable of beneficially influencing systemic inflammation.

Graphs were designed using Gephi, an open-source network visualization platform (Bastian M., Heymann S., Jacomy M. (2009). Gephi: an open source software for exploring and manipulating networks. International AAAI Conference on Weblogs and Social Media). The layout Yifan Hu was chosen for the "Pathology" network, the conformation of the graph was saved and applied in the construction of the "Natural Product" and the "Reference Drug" graphs.

The direction of the modulation is represented by the black filling of the nodes for up-regulation and empty nodes for down-regulation. Nodes in grey do not present a modulation in a specific direction. The size of the nodes represents the Z-scores, which were previously scaled based on all the values used in the a specific panel. In Gephi, size of the nodes were controlled by using the "Ranking" parameter with a size range from 15 to 60.

The "HUBs" of the networks represent central biological processes of interest for the specific Pathology. They are represented in grey colour, labelled in capital letters and their expected modulation is illustrated by arrows going up or down, according to literature (for Pathology networks) or based on the modulation of the diseases and Biofunctions/ biological parameters (for "Natural Product" and "Reference Drug" networks). (figure 19).

### 10. EXAMPLE OF THE METHOD OF THE INVENTION

The method of the invention was performed on a product for the treatment of osteoarthrosis.

The selected product was product A which comprises as natural matrix the coextract of Centella asiatica leaves and Echinacea purpurea flowers as described in example 1. Five different validated batches of the product were selected (see example 1).

A set of genes whose modulation resulted in the modulation of the one or more biological activities underlying osteoarthrosis was identified as exemplified in example 2 above and the in vitro cell assay described in example 1 and in the annexed table was performed.

The quali-quantitative modulation values for each one or more biological activities were calculated as described above in terms of z-scores, and the values were compared as reported in figures 1-4.

Figures 2 to 4 show that the conditions of (c) of the method of the invention are fulfilled, i.e.,
- at least 50% of the one or more biological activities identified for each hallmark are modulated by each batch according to the modulation trend representative of the healthy state and
the quali-quantitative modulation values calculated for each of said at least 50% of one or more biological activities in a) above, differ of at least 15% from the respective quali-quantitative modulation values calculated for said pathological state control.

In fact, figures 2-4 show that all the one or more biological activities identified for each hallmark are modulated according to the modulation trend concurring to the healthy physiological state (healthy state) represented in figure 1, and the same figure shows that the quali-quantitative modulation values calculated for each one or more biological activities differs of at least 15% from the respective modulation values calculated for the pathophysiological state control.

The same can be observed also for the other 3 tested products (B, C, and D in the related figures).

Product A hence regulates a state. Figure 3 also shows that triamcinolone, which is a drug commonly used for treating osteoarthrosis, consistently with what is known in the state of the art, is not capable or f regulating a state as it does not comply with the requirements above as for one of the selected hallmarks the identified activity is not modulated according to the modulation trend concurring to the healthy state. Indeed, for the biofunction related to the formation of cartilage tissue, the reference drug analysed shows a worsening of the pathological regulation rather than the desired up-regulation to reach the healthy physiological state.

Product A hence, comprises a natural matrix and regulates a state.

Furthermore, as clear from figure 4, the different batches of the product show a functional resilience, i.e., notwithstanding their different quali-quantitative chemical composition (see figure 5 and example 8), the quali-quantitative modulation value calculated each batch for a given activity in each group, differs of maximum 20% from the average of said value calculated on all groups.

By way of example, the one or more biological activities "formation of cartilage tissue" average modulation value is 0.15 and each modulation value for said activity for each batch was 0.15; the one or more biological activities "inflammation of joint" average modulation value is -0.29, and the measured values span from -0.24 to -0.33, the difference from the average value being of about ±8%; the differences for the other two examined activities being even narrower.

Therefore, notwithstanding the evident quali-quantitative variability in the components of each batch, the overall therapeutic result provided by each batch is substantially superimposable i.e., show functional resilience.

As stated above, the parameters selected for product A were genes and their expression was analysed through conventional transcriptomic analysis.

A summary of the involved relevant genes for the selected one or more biological activities shows that, for each activity, the set of regulated genes is different for every batch, nevertheless, the final modulation of each analysed activity is extremely similar, and the calculated modulation value differs between different batches, of maximum ±8% with respect to the average modulation value obtained for said activity.

This result can only be explained by the preservation, in the therapeutic product, of a physiological mechanism of action.

The genes symbols used in the table below are the official gene symbols used in the NIH genbank.

| **Hallmarks of the pathology** | **Biological functions** | **TREATMENTS PRODUCT A** | | | | | | | | |
|---|---|---|---|---|---|---|---|---|---|---|
| | | **Batch 20B1955** | | | **Batch 20B0596** | | | **Batch 2011279** | | |
| | | **Genes** | **Fold change** | **z-score value** | **Genes** | **Fold change** | **z-score value** | **Genes** | **Fold change** | **Z-score value** |
| **Induction of proliferation** | **Formation of cartilage tissue** | DKK1 | -27,03 | **0,29** | DKK1 | -6,49 | **0,29** | DKK1 | -5,83 | **0,29** |
| | | BCL2L11 | 2,38 | | BCL2L11 | 2,52 | | BMX | 3,06 | |
| | | BDKRB2 | 2,11 | | BHLHE40 | 2,03 | | CD70 | -4,44 | |
| | | BHLHE40 | 2,37 | | CD70 | -4 | | CFD | -2,43 | |
| | | BMX | 2 | | CR2 | 2 | | CXCL16 | 2,57 | |
| | | BTC | 2,13 | | CXCL16 | 2,83 | | ERRFI1 | 2,85 | |
| | | CD70 | -5,42 | | ERRFI1 | 3,04 | | HES1 | -2,26 | |
| | | COL9A1 | 2,01 | | HES1 | -2,84 | | HMMR | -3,48 | |
| | | CSF3 | -2,2 | | HMMR | -3,16 | | IFNB1 | 9,13 | |
| | | CXCL16 | 3,44 | | IL23A | -2,46 | | IRF1 | 2,01 | |
| | | CYP7B1 | -2,26 | | JAG1 | -3,19 | | JAG1 | -2,73 | |
| | | ERRFI1 | 3,5 | | LDLR | 2,43 | | LDLR | 2,29 | |
| | | GDFS | 3,04 | | LTBP3 | 3,31 | | LTBP3 | 5,99 | |
| | | HMMR | -4,71 | | MACIR | -2,5 | | MMP16 | -2,02 | |
| **Reduction of inflammation** | **Inflammation of joint** | IFNGR1 | -2,06 | **-0,43** | MERTK | -2,45 | **-0,53** | NFATC1 | 2,03 | **-0,52** |
| | | IL23A | -2,36 | | NFATC2 | 2,16 | | NFATC2 | 2,1 | |
| | | JAG1 | -3,02 | | NQ01 | 2,62 | | NQO1 | 2,6 | |
| | | LDLR | 2,81 | | PARP1 | -3,04 | | PARP1 | -2,35 | |
| | | LGALS9B | 2,16 | | PGF | -2,18 | | PGF | -2,4 | |
| | | LTBP3 | 5,64 | | PLAU | -2,12 | | PLAU | -2,17 | |
| | | NQ01 | 2,52 | | PTGER4 | 3,17 | | PRKCQ | 2,85 | |
| | | PARP1 | -2,46 | | RORA | 3,75 | | PTGER4 | 2,57 | |
| | | PGLYRP2 | 2,46 | | SOD3 | 2 | | RAG1 | 2,99 | |
| | | PLA2G4A | 2,18 | | TMEM178A | 3,48 | | RORA | 3,1 | |
| | | PLAU | -2,33 | | | | | SLAMF9 | 2,32 | |
| | | PTGER4 | 2,68 | | | | | TNFSF10 | 3,28 | |
| | | RORA | 3,83 | | | | | ZFP36 | 2,01 | |
| | | SPHK1 | -2,38 | | | | | | | |
| | | TRPV1 | 2,43 | | | | | | | |
| | | BHLHE40 | 2,37 | | BHLHE40 | 2,03 | | CFD | -2,43 | |
| | | COL9A1 | 2,01 | | ERRFI1 | 3,04 | | ERRFI1 | 2,85 | |
| | | CSF3 | -2,2 | | HES1 | -2,84 | | HES1 | -2,26 | |
| | | CYP7B1 | -2,26 | | LTBP3 | 3,31 | | IFNB1 | 9,13 | |
| | | ERRFI1 | 3,5 | | NFATC2 | 2,16 | | LTBP3 | 5,99 | |
| | | GDFS | 3,04 | | PARP1 | -3,04 | | NFATC1 | 2,03 | |
| | | LTBP3 | 5,64 | | PTGER4 | 3,17 | | NFATC2 | 2,1 | |
| | **Non-traumatic arthropathy** | PARP1 | -2,46 | **-0,52** | RORA | 3,75 | **-0,49** | PARP1 | -2,35 | **-0,53** |
| | | PTGER4 | 2,68 | | | | | PTGER4 | 2,57 | |
| | | RORA | 3,83 | | | | | RAG1 | 2,99 | |
| | | | | | | | | RORA | 3,1 | |
| **Protection from anatomical damage** | | | | | | | | SLAMF9 | 2,32 | |
| | **Osteoarthritis** | BHLHE40 | 2,37 | **-0,51** | BHLHE40 | 2,03 | **-0,49** | ERRFI1 | 2,85 | **-0,51** |
| | | COL9A1 | 2,01 | | ERRFI1 | 3,04 | | HES1 | -2,26 | |
| | | CYP7B1 | -2,26 | | HES1 | -2,84 | | LTBP3 | 5,99 | |
| | | ERRFI1 | 3,5 | | LTBP3 | 3,31 | | NFATC1 | 2,03 | |
| | | GDFS | 3,04 | | NFATC2 | 2,16 | | NFATC2 | 2,1 | |
| | | LTBP3 | 5,64 | | RORA | 3,75 | | RORA | 3,1 | |
| | | RORA | 3,83 | | | | | SLAMF9 | 2,32 | |

| | | **Batch 21E1640** | | | | | **Batch 2011770** | | | |
|---|---|---|---|---|---|---|---|---|---|---|
| **Genes** | | **Fold change** | | **Z-score value** | **Genes** | | **Fold change** | **Z-score value** | | |
| **Induction of proliferation** | **Formation of cartilage tissue** | DKK1 | | -5,39 | | **0,29** | DKK1 | | -4,19 | **0,29** |
| | | BCL2L11 | | 2,12 | | | AREG | | 2,11 | |
| | | C5AR1 | | 2,3 | | | CCL3L3 | | 2,47 | |
| | | CD274 | | -2,39 | | | CD70 | | -6,48 | |
| | | CD70 | | -4,7 | | | CXCL16 | | 2,88 | |
| | | CXCL16 | | 2,47 | | | CYP7B1 | | -2,39 | |
| | | CYP7B1 | | -2,11 | | | ERRFI1 | | 2,94 | |
| | | ERRFI1 | | 2,85 | | | FGFBP1 | | -2,17 | |
| | | GDFS | | 2,32 | | | GDFS | | 2,01 | |
| | | HMMR | | -9,97 | | | HMMR | | -4,64 | |
| | | ICOSLG | | -2,87 | | | IFNB1 | | 2,14 | |
| | | ILIA | | -2,19 | | | IL12A | | -2,48 | |
| | | IL1B | | -3,49 | | | IL23A | | -2,99 | |
| **Reduction of inflammation** | **Inflammation of joint** | IL23A | | -5,45 | | **-0,50** | JAG1 | | -2,33 | **-0,52** |
| | | LTBP3 | | 5,7 | | | LDLR | | 2,15 | |
| | | MACIR | | -2,82 | | | NFATC1 | | 2,52 | |
| | | MMD | | -2,08 | | | NFATC2 | | 2,22 | |
| | | MSTN | | 2,36 | | | NQO1 | | 2,33 | |
| | | NFATC2 | | 2,82 | | | PARP1 | | -2,12 | |
| | | NFKB1 | | -2,09 | | | PGF | | -2,5 | |
| | | NLRP3 | | 2,7 | | | PLAU | | -2,07 | |
| | | NQ01 | | 3,17 | | | RORA | | 3,26 | |
| | | PLAU | | -2,56 | | | SH2D1A | | -2,22 | |
| | | PTGS1 | | 2,17 | | | SPHK1 | | -2,02 | |
| | | RORA | | 3,01 | | | TMEM178A | | 2,98 | |
| | | TNFSF12 | | 2,53 | | | UNK | | 2,18 | |
| | | TSLP | | -6,91 | | | ZFP36 | | 2,5 | |
| | | ZFP36 | | 3,39 | | | | | | |
| | | | | | | | | | | |
| | | CSAR1 | | 2,3 | | | CYP7B1 | | -2,39 | |
| | | CYP7B1 | | -2,11 | | | ERRFI1 | | 2,94 | |
| | | ERRFI1 | | 2,85 | | | FGFBP1 | | -2,17 | |
| | | GDFS | | 2,32 | | | GDFS | | 2,01 | |
| | | IL1B | | -3,49 | | | IFNB1 | | 2,14 | |
| | | LTBP3 | | 5,7 | | | NFATC1 | | 2,52 | |
| | | NFATC2 | | 2,82 | | | NFATC2 | | 2,22 | |
| | **Non-traumatic arthropathy** | RORA | | 3,01 | | **-0,44** | PARP1 | | -2,12 | **-0,51** |
| | | | | | | | RORA | | 3,26 | |
| | | | | | | | UNK | | 2,18 | |
| | | | | | | | | | | |
| **Protection from anatomical damage** | | | | | | | | | | |
| | **Osteoarthritis** | CYP7B1 | | -2,11 | | **-0,49** | CYP7B1 | | -2,39 | **-0,47** |
| | | ERRFI1 | | 2,85 | | | ERRFI1 | | 2,94 | |
| | | GDFS | | 2,32 | | | FGFBP1 | | -2,17 | |
| | | LTBP3 | | 5,7 | | | GDFS | | 2,01 | |
| | | NFATC2 | | 2,82 | | | NFATC1 | | 2,52 | |
| | | RORA | | 3,01 | | | NFATC2 | | 2,22 | |
| | | | | | | | RORA | | 3,26 | |

The data summarised in the table above, where only the genes regulated underlying a particular biological activity, are indicated for each batch, shows that each batch modulates a different set of genes (up modulation positive numbers, down-modulation negative numbers) but that the resulting modulation of the one or more biological activities under examination is batch-to-batch superimposable.

Therefore, the product has an inter-batch variable quali-quantitative composition, each batch modulates the one or more biological activities of interest regulating different set of genes (some in common and some not) but nevertheless, the modulation of the one or more biological activities examined is retained batch-to-batch in terms of direction and magnitude. The data hence demonstrate the functional resilience of the batches and show that this resilience is the result "redundancy" in gene regulation, in which the matrices networks interact with the living biological target network through different pathways and genes triggering the same final answer from the biological system. This mode of action is the result of two entities substantially "speaking the same language", i.e., the physiological language as they both belong of the domain of the everything that is living, in other words, natural.

### EXAMPLE 11 CLINICAL VALIDATION OF THE PHYSIOLOGICAL MECHANISM OF ACTION

In the context of the "Randomized controlled trial to evaluate the efficacy of a food supplement in subjects with mild cognitive impairment" study, ClinicalTrials.gov Identifier: NCT03581929, in addition to data on the achievement of endpoints classically derived from the geriatric field and relating to the cognitive and physical performance of the patients enrolled in the study, it was possible to analyse a set of biological data ('multiomics' parameters such as transcriptomics, metabolomics and microbiomics) by submitting to biological assays samples collected from the groups of patients corresponding to (i) patients treated with therapeutic or beneficial product exerting its therapeutic or beneficial effect through a physiological mechanism of action at different time moments, and (ii) patients treated with a therapeutic or beneficial reference product (comparator) at the same different time moments and/or (iii) patients treated with a placebo at the same different time moments (or different combinations thereof e.g., initial treatment with placebo followed by treatment with product of interest), determining whether said product exert its therapeutic or beneficial action by regulating a network of biological activities that underly the desired therapeutic or beneficial effect. The study was a 12-month single-center randomized controlled study (including 6 months double-blind versus placebo and 6 months open-label). 50 subjects were enrolled (V1) in the study after eligibility evaluation. The purpose of the study was to evaluate whether treatment with Product B could improve cognitive performance in subjects with MCI.

Primary objectives:
1) to evaluate the effect of Product B on the overall cognitive performance in patients with MCI after 6 months (V2), compared to placebo, measured by Free and Cued Selective Reminding Test (FCSRT) and Addenbrooke's Cognitive Examination-Revised (ACE-R) tests.
2) to evaluate the effect of Product B on the overall cognitive performance in the entire population studied in an open-label extension phase lasting 6 further months (V3), measured by the FCSRT and ACE-R tests.

### Secondary objectives:

1) to evaluate the potential variations of the main parameters of
   - cognitive performance measured with additional tests that explored multiple cognitive functions with respect to assessments as primary objectives
   - physical performance
   - modification of biochemical parameters of inflammation
   - modification of parameters related to oxidative stress conditions
   - changes in the microbiome
   after 6 (V2) and 12 (V3) months of treatment compared to baseline (V1) and compared to placebo.
2) to evaluate the safety and tolerability of Product B through the analysis of Adverse Events (AEs) and Serious Adverse Events (SAEs).

The clinical tests carried out of on the patients to validate the therapeutic/beneficial effect of the product tested included Cognitive performances were evaluated by ACE-R (Pigliautile et al., 2015), FCSRT (Frasson et al., 2011), Mini-Mental State Examination (MMSE) (Magni et al., 1996), Digit Span forward and backward (Monaco et al. 2013), Trail Making Test (TMT) (Amodio et al., 2002), Babcock Story Recall Test (BSRT) (Spinnler and Tognoni, 1987), Rey Auditory Verbal Learning Test (AVLT) (Carlesimo et al., 1995; 1996), Raven test (Carlesimo et al., 1995; 1996), Token test (Spinnler and Tognoni, 1987), Verbal Fluency Test (VFT letter: Carlesimo et al., 1995; 1996. VFT category: Spinnler and Tognoni, 1987), Copy of drawings (Spinnler and Tognoni, 1987) and Verbal judgement (Spinnler and Tognoni, 1987). Physical Performance assessment were done through Bioelectrical Impedance Analysis (BIA), Handgrip, Timed Up and Go test (TUG), Short Physical Performance Battery (SPPB), Frailty Index, Activities of Daily Living (ADL), Instrumental Activities of Daily Living (IADL). The overall results of these exams confirmed the desired therapeutic/beneficial effect of the product.

Additionally, to determine whether said product exerts its therapeutic or beneficial action by regulating a network of said biological activities underlying a pathophysiological or altered physiological state related to said condition, a set of biological assays were carried out from samples collected by said patients at different time points of the treatment.

In particular, omics analyses were performed on faecal samples using NGS sequencing to determine the composition of the faecal microbiota and to predict the bacterial metabolites produced and systemically absorbed in the gut. Furthermore, gene expression analysis by RNA-seq, metabolomics analysis by GC-MS (Gas Chromatography and Mass Spectrometry), determination of levels of a panel of circulating cytokines, levels of oxidative stress and an extensive battery of markers typical of clinical biochemistry were performed on peripheral blood samples.

### Materials and methods

### Microbiome Analysis

Each patient provided 3 stool samples during the study (visits 1, 2, 3), according to the instructions provided by the clinical center and using the sample collection kit provided. The collected samples were stored at room temperature and small aliquots of the samples were transferred into 2 ml tubes and stored at - 80°C. The samples were sent on dry ice to the external laboratory (Edmund Mach Foundation, San Michele all'Adige - Trento) in charge of the analysis of the intestinal microbiota [bacterial Deoxyribonucleic Acid (DNA) analysis]. Samples were processed for DNA extraction, PCR, libraries preparation and sequencing. An average concentration of 6600 ± 6100 ng of DNA was isolated from 100mg of feces.

130 faecal samples and 8 control samples (5 DNA extraction buffers and 3 PCR negative controls) were sent to be sequenced. Sequencing produced 47 549 492 total reads, 42,828,220 PF Reads (i.e. total number of clusters passing filter), 74% Reads Identified (PF) (i.e. percentage of clusters passing filter that are assigned to an index), corresponding to 15,841,323.4 forward and reverse identified reads, with 0,27 CV (i.e. coefficient of variation for the number of counts across all indexes) and with 121822,9 ± 24605,26 (mean±SD) raw reads per sample (excluding controls, which produced 1237,3 ± 836,02 reads per sample, mean±SD). After merging forward and reverse reads, de-noising and chimera removal 10,844,273 sequences per samples were obtained, with 83414,4 ± 18174,1 (mean ± SD) mean number of sequences per samples (excluding controls), corresponding to 36,035 features in total. Sequences had 434.31 ± 11.63 bp mean length.

Relative abundance (%) and absolute abundance of taxa summary analysis at phylum has been performed.

Further microbiome analyses were performed to identify which bacteria are enriched in patients after treatment and which metabolic activities are restored.

Two different analysis approaches were used. Once the gene sequences of the variable region V3-V4 of the 16S gene were obtained, the QIIME2 software was used for the taxonomic identification of the bacteria present in the faecal samples and the PICRUSt2 software was used to simulate the metagenomic content, in other words the metabolic pathways expressed in the identified bacteria whose abundance was enriched or depleted in response to treatment.

### Clinical Laboratory exams:

Ematochemical analyses and urinalyses as well as ECG changes from baseline were evaluated. Safety laboratory tests were performed in the clinical site facility (Servizio di Patologia Clinica ed Ematologia - Azienda Ospedaliera di Perugia) and ECGs were performed locally, too. Blood and urine analyses were assessed at baseline, after 6 and after 12 months of regular consumption of the study products. The analyses included: glucose, nitrogen, Gamma GT, total cholesterol, potassium, calcium, vitamin B12, folic acid, CRP. Results are expressed as the ratio between the average modulation values of the groups (see tables below). ù

### Plasma Inflammatory Cytokines and Plasma Markers of Oxidative Stress:

Samples were collected at each visit. Samples were stored at -80°C and then analysed at the Research Laboratory of the Department of Gerontology and Geriatrics of the Perugia University.

Plasma inflammatory cytokines: Different chemokines and cytokines were evaluated in the plasma of all subjects with the Multiplex method, to evaluate the inflammatory response. This method allows the simultaneous measurement of: EGF, GM-CSF, IFNα2, IFNγ, IL-10, , IL-13, IL-17, IL-1RA, IL-1B, IL-2, IL-4, IL-5, IL-6, TNFα, , VEGF. Results are expressed as the ratio between the average modulation values of the groups (see tables below).

### Plasma Markers of oxidative stress:

The concentration or enzymatic activity of Superoxide Dismutase (SOD) were evaluated on plasma. Results are expressed as the ratio between the average modulation values of the groups.

### Transcriptomic analysis

Samples were collected at each visit. Samples were stored at -20°C for 24 h then at - 80°C. RNA profiles from patient's whole blood will be evaluated using The PAXgene^{™} Blood RNA System (PreAnalytiX, QIAGEN), that includes the PAXgene^{™} Blood RNA Tube, a vacutainer for RNA collection and stabilization, and the QIAsymphony PAXgene^{™} Blood RNA Kit for automated RNA purification on the QIAsymphony SP instrument. The gene expression profiles was evaluated by using RNA-Seq data obtained with Illumina NextSeq, sequenced in paired-end mode. Sample was mapped on reference genome using the bioinformatics tool STAR (version 2.7.5c), with the standard parameters for paired reads. The reference track was the assembly Homo Sapiens HG38 obtained from GenCode (Release 35 (GRCh38.p13)). The quantification of transcripts expressed for each sequenced sample was performed using featureCount algorithm. Bioconductor package DESeq2 was used to normalize the data, using the median of ratio, to perform the differential expression analysis. Quality control check such as Euclidean distances (Heatmap Distances) and Principal component analysis (PCA) were performed among all samples in each condition considered.

### Metabolomic analysis:

Samples were collected at each visit. Samples were centrifuged at 3000g for five minutes to separate the palsma within two hours of collection and stored at -80°C.

The extraction, purification, and derivatization processes for the metabolome were carried out using the MetaboPrep kit (Theoreo, Montecorvino Pugliano, SA), following the manufacturer's guidelines.

A 2 µL aliquot of the derivatized solution was introduced into the GC-MS System, consisting of a GC-2010 Plus gas chromatograph coupled to a 2010 Plus single quadrupole mass spectrometer, both from Shimadzu Corp. in Kyoto, Japan. Chromatographic separation was accomplished utilizing a CP-Sil 8 CB fused silica capillary column (Agilent, J&W), boasting a 1.00 µm film thickness, a length of 30 meters, and an outer diameter of 0.25 mm, with helium serving as the carrier gas. The initial temperature was established at 100 °C and maintained for 1 minute, after which it was incrementally raised to 320 °C at a rate of 6 °C per minute and held at that final temperature for an additional 2.33 minutes. The gas flow was regulated to maintain a consistent linear velocity of 39 cm/s, and the split flow was configured at a ratio of 1:5. The mass spectrometer operated with electron impact ionization at 70 electronvolts (eV) in full scan mode, scanning within the range of 35-600 m/z (mass-to-charge ratio) at a rate of 3333 atomic mass units per second (amu/sec). A solvent cutoff time of 6.0 minutes was applied. The entire gas chromatography (GC) program had a total duration of 40 minutes. To facilitate the analysis, the samples were grouped, with each group typically containing 10 samples (or fewer). Each group was monitored with 3 controls: a run without injection, an injection of a mix of standards, and duplicate analysis of a randomly selected sample in that group (Figure 3). The linear retention index was also determined at the commencement and conclusion of the analytical run and after every 50 samples analyzed. This determination was made employing a mixture of alkanes with an even number of carbon atoms, as proposed by Kovats et al. to enhance the precision of structural annotation. For the blank injection, 2 µL of hexane was employed. In contrast, the standard solution contained a mixture of 16 diverse molecules, encompassing organic acids, sugars, amino acids, sterols, fatty acids, and vitamins. The second injection involved a randomly selected sample from within the same group. Each sample (including replicate) was analyzed in triplicate. Each analysis group was considered valid if the blank did not generate any peaks, if the ratio of the areas underlying the peak of the standards (normalized to the area of the internal standard) remained within 90-110% of the expected value, and if the variance of the ratio of areas (normalized to the area of the internal standard) of the 100 major peaks of the repeat injection was below 15% from the original sample. Given the absence of baseline resolution in the obtained chromatograms, a spectral deconvolution process was applied. This procedure involved the utilization of the primary fragments discerned in the mass spectra associated with each metabolite, provided that they conformed to the specified criteria, which were as follows: Area > 10,000; Slope > 100 per minute; Width > 1 second. Single Ion Monitoring (SIM) chromatograms were employed for the quantification of metabolite areas. As part of this process, 2-isopropyl malic acid was employed as the internal standard, with a SIM value set at 147.

A targeted quantitative metabolomics approach was applied to analyze the samples using a reverse-phase LC-MS/MS custom assay. This custom assay, was based on an Orbitrap Exploris^{™} 120 Mass Spectrometer (Thermo Fischer, USA)

The method combines the derivatization and extraction of analytes, and the selective mass-spectrometric detection using exact mass extraction signals and a data-dependent approach. Isotope-labeled internal standards and other internal standards are used for metabolite quantification. The custom assay contains a 96 deep-well plate with a filter plate attached with sealing tape, and reagents and solvents used to prepare the plate assay. First 14 wells are used for one blank, three zero samples, seven standards and three quality control samples. For all metabolites except organic acid, samples will be thawed on ice and vortexed and centrifuged at 13,000x g. Sample will be loaded onto the center of the filter on the upper 96-well plate and dried in a stream of nitrogen. Subsequently, phenyl-isothiocyanate was added for derivatization. After incubation, the filter spots will be dried again using an evaporator. Extraction of the metabolites will then be achieved by adding 300 µL of extraction solvent. The extracts will be obtained by centrifugation into the lower 96-deep well plate, followed by a dilution step with MS running solvent.

For organic acid analysis, 150 µL of ice-cold methanol and 10 µL of isotope-labeled internal standard mixture was added to the samples for overnight protein precipitation. Then it was centrifuged at 13000x g for 20 min. 50 µL of supernatant will be loaded into the center of wells of a 96-deep well plate, followed by the addition of 3-nitrophenylhydrazine (NPH) reagent. After incubation for 2h, BHT stabilizer and water was added before LC-MS injection.

Mass spectrometry instrument was equipped with a Vanquish UHPLC system (Thermo Scientific, USA). The samples were delivered to the mass spectrometer by a LC method followed an ESI (Electrospray ionization) method. Data analysis was done using Compound Discovery 3.3 and Xcalibur^{™} Software.

### Partial Least Square Discriminant Analysis (PLS-DA) for the identification of metabolytes of interest:

Multivariate Partial Least Squares Discriminant Analysis (PLS-DA) was conducted on chromatograms that had been normalized to the peak area of the internal standard. This analysis was carried out utilizing the R software, developed by the Foundation for Statistical Computing in Vienna, Austria. The separation of distinct classes was achieved through the application of a PLS-DA algorithm, a supervised method employing multivariate regression techniques. This technique leverages linear combinations of the original variables (X) to extract information capable of predicting class membership (Y). To pinpoint the variables that contributed most significantly to the separation of classes, Variable Importance in Projection (VIP) scores were computed for each metabolite. Subsequently, the constructed models underwent both permutation testing and cross-validation procedures, ensuring the mitigation of potential errors attributable to overfitting.

### Identification of metabolites of interest:

The application of "high-throughput technologies" in untargeted metabolomics analysis enables the concurrent measurement of numerous metabolites in a biological sample. Not all of this extensive information, however, pertains directly to the specific condition under investigation. It has been established that the judicious selection of metabolites plays a pivotal role in enhancing the predictive accuracy of various classifiers, as demonstrated in prior studies. Furthermore, classification models constructed from smaller subsets of the original metabolites tend to be more straightforward and easier to interpret than those based on the complete set of metabolites. Based on these considerations, we streamlined the analysis of the original metabolites by curating a simplified dataset. This curation process involved the use of a genetic algorithm (GA) to pinpoint the metabolites of paramount interest. Subsequently, these metabolites of interest underwent structural identification by comparison with a library of fragmentation spectra (NIST 2014), with the Kovats index serving to discern and exclude spurious occurrences. Additionally, metabolites that displayed either the highest VIP-scores (Top-10) or exhibited a positive outcome in the analysis of variance (ANOVA), wherever applicable, were subject to further scrutiny to establish their putative identification at level 1 in accordance with the criteria set forth by the Metabolomics Standard Initiative (MSI).

### RESULTS

The most significant evidence that was evaluated in clinical responses to assumption of Product B product was found in "Cognitive Performance".

### Cognitive Performance

### Patients treated with Product B:

Group A V1 first visit, no treatment, V2 6 months of treatment with product B from V1, V3 6 months of treatment with product B from V2.

Group B V1 first visit, no treatment, V2 6 months of treatment with placebo from V1, V3 6 months of treatment with product B from V2.

### Every single test submitted to patients at every visit/check was processed for both groups of patients.

Group A: improvements in the scores such as: the F-A-S Test (which evaluates executive functions), Free and Cued Selective Reminding Test, Immediate and Delayed recall (which is related to verbal long term memory), Token Test linked to language and Timed Up and Go (sensitive to risk of falls among older adults) were observed. All test results showhed an improvement after 6 months of treatment within the Product B group. The improvement on verbal long-term memory was present also in the V2-V3 comparison (Rey Auditory Verbal Learning Test Immediate and Delayed recall). After 12 months of Product B, positive effects were found in attention and executive functions measures (B-A score at the Trail Making Test, Trail Making Test B, Digit Span Forward, F-A-S test), verbal long term memory (Rey Auditory Verbal Learning Test Immediate and Delayed recall), mediated by short and long term memory abilities and language (Category Fluency Test).

Group B: No difference was found between V1-V2 evaluation within the group starting with placebo. However, we can note that improvement on global cognitive domain (Mini Mental State Examination), attention (B-A score at the Trail Making Test) and physical performance measures (Hand grip Test, Timed Up and Go Test (in males) were found in V2-V3. Moreover, in V1-V3 comparison an improvement on cognitive performance emerged for attention and executive function domain (Digit Span Backward), language (Category Fluency Test), constructional abilities (Copy Drawing Test) and physical performance measures linked to strength of the hand and forearm muscles (Hand grip Test, both in males and females), progress of balance (Timed Up and Go Test, both in males and females) and to lower extremity function and mobility (Short Physical Performance Battery).

Conversely, the scores of the Addenbrooke's Cognitive Examination Revised (used as comprehensive cognitive assessment involving orientation, memory, verbal fluency, language and visuospatial abilities) did not show changes in the two groups. Similarly, Trail Making Test A, used to explore cognitive processing speed, Babcock Story Recall which measure episodic verbal memory and verbal (Verbal Judgment Test) and non-verbal (Raven's Coloured Progressive Matrices) general intelligence measures, Geriatric Depression Scale (useful to detect depression symptoms) and Activity of Daily Living and Instrumental Activities of Daily Living (common measures of functional status) remained stable during the 12 months of the study.

In general, improved or stable conditions were observed following treatment with Product B, suggesting for non-progression of the disease, which instead is typically known to be degenerative. Based on this study results, an effect of the Product B treatment was particularly evident on the cognitive performance, especially when the treatment was prolonged up to 12 months. In particular, Product B improved attention, executive functions, memory, and language abilities. These functions resulted improved in Product B group, but also in the Placebo group since the switch to Product B. At the same time, Product B showed to improve physical performance with an action on strength and mobility. No effect was found on mood.

### Effects on gut microbiota

Samples collected from subjects enrolled in clinical trial, were analyzed to assess the changes in gut microbiota composition after 6 months (V2) and 12 months (V3) of Product B treatment (group A).

The Product B treatment identified a significant decrease of Actinobacteria and Firmicutes after 6 months of Product B treatment (V2) and a significant increase of Bacteroidetes after 6 and 12 months. These results indicate that the product modulates the gut microbiome composition both stimulating the growth of Bacteroidetes and reducing the Phylum of Firmicutes, resulting in reduction of the ratio of Firmicutes/Bacteroidetes from 12.9±33.7 to 6±1.2 after 6 months of treatment and to 1.8±0.8 after 12 months.

The absolute abundance results highlighted that a significant increase of Bacteroidetes was observed in gut microbiome of patients affected by MCI after 6 (V2) and 12 (V3) months of Product B and a significant increase is also clear between 6 and 12 months of treatment, indicating that the beneficial effects of treatment depend on treatment time. In addition, absolute abundance data have identified a significant increase of Firmicutes after 12 months of Product B and no significant effects on the absolute abundance of Proteobacteria and Actinobacteria.

At Family, Genus and Species level no significant differences were observed. However, significant differences were observed at the level of enrichments of bacterial metabolic pathways, i.e "pyruvate fermentation to butanoate" and "L-lysine fermentation to acetate and butanoate", resulting in an increase of SCFA producers and consequentially propionic acid, pentanoic acid, butyric acid, D-gluconic acid and acetoacetic acid. The beneficial effects of Product B in reshaping the gut microbiome composition in patients affected by MCI positively correlate with the improvement of cognitive and physical performances identified in the patients after 6 or 12 months of Product B treatment.

### Clinical laboratory exams, biochemical parameters of inflammation and oxidative stress:

Some clinical laboratory evaluations (Hematology, biochemistry and urine examinations) were analysed and reported as secondary efficacy and safety parameters.

| clinical laboratory evaluations V2vsV1 | Product B | Placebo |
|---|---|---|
| Glycemia | 1,08 | 1,03 |
| Azotemia | -1,03 | 1,03 |
| Gamma GT | 1,06 | 1,06 |
| Calcium | 1,01 | 1,01 |
| Potassium | 1,00 | -1,00 |
| Vitamin B12 | 1,01 | 1,12 |
| Folic acid | -1,06 | -1,11 |
| C-reactive protein | 2,94 | -1,02 |
| Total Cholesterol | -1,01 | -1,02 |

Furthermore, different chemokines and cytokines were dosed in the plasma of all subjects with the Multiplex method, to evaluate the inflammatory response. Oxidative stress conditions were evaluated through the enzymatic activity of Superoxide Dismutase (SOD), on plasma.

With regard to plasma inflammatory cytokines, parameters of inflammation, such as IL-10, IL-1b and IL-2, resulted significantly decreased at Visit 2 in Product B treated subjects compared to Placebo subjects.

| plasma inflammatory cytokines V2vsV1 | Product B | Placebo |
|---|---|---|
| EGF | -1,03 | -1,12 |
| GM-CSF | -1,02 | 1,16 |
| IFNa2 | 1,10 | 1,20 |
| IFN g | -1,06 | 1,10 |
| IL-10 | 1,03 | 1,14 |
| IL-13 | -1,11 | 1,00 |
| IL-17 | -1,04 | 1,18 |
| IL-1 RA | -1,10 | -1,02 |
| IL-1b | -1,06 | 1,09 |
| IL-2 | -1,17 | 1,04 |
| IL-4 | -1,31 | -1,27 |
| IL-5 | 1,05 | 1,17 |
| IL-6 | 1,02 | -1,02 |
| TNF-a | -1,07 | -1,01 |
| VEGF | -1,05 | 1,01 |

The elaboration of the data provided herein shows that these findings are in agreement with the observation that Product B improves attention, memory and general physical performance.

### Transcriptomic analysis

The tables below display the number of differentials expressed genes (DEG) identified with a FDR≤0.05 and -2≤FC≥2.

| | FDR≤0.05 | FC≥2 & FDR≤0.05 | FC≤2 & FDR≤0.05 |
|---|---|---|---|
| V2 vs V1 | 249 | 31 | 218 |

The software Ingenuity Pathway Analysis (IPA, QIAGEN Inc., https://www.qiagenbioinformatics.com/products/ingenuitypathway-analysis) via the "grow" function allows the identification of metabolites, to which the DEGs (Differentially Expressed Genes) identified via RNAseq were linked based on knowledge of their functional relationships. This information was filtered based on the type of biological material of interest, specifying blood and plasma/serum.

### Metabolomic analysis

The tables below display the metabolites of interest, delineating metabolic patterns or "fingerprinting" associated with 6 months of Product B treatment.

| Plasma Metabolites V2 vs V1 | FC |
|---|---|
| Gluconic acid | 2 |
| Pipecolic acid | 2 |
| 5-hydroxymethyluracil | 2 |
| Acetoacetic acid | 2 |

Bioinformatic modelling of experimentally observed multiomic parametersavailable from the clinical study.

According to the invention, the read out of the biological assay provides quali-quantitative data on the modulation or modulation pattern of each of said parameters on each of said samples or each of said group (pool) and related one or more biological activities thereto, associated to one or more hallmark of the pathological condition or of altered physiological state of interest.

When multiomic parameters such as gene expression, plasma metabolites, biochemical parameters, microbiome composition and bacterial metabolites are selected as markers, the modulation or modulation pattern of each of these parameters has been used to evaluate the modification of each biological activity causally linked to each selected hallmark of a pathological condition, this work can be facilitated by using ad hoc bioinformatics tools.

Ingenuity Pathways Analysis (IPA) (QIAGEN \Inc., https://www.qiagenbioinformatics.com/products/ingenuitypathway-analysis) was used, for each investigated group to evaluate modulations of multiomic parameter relevant for effects of interest.

IPA is an aggregator of scientific references that allows to search for information on genes/proteins/metabolites and the construction of networks that predict the behaviour of biological systems according to their gene/protein/metabolite expression status. This knowledge was used to interrogate IPA via the "IPA Bioprofiler" tool, using the following keywords: neuro, cognition, memory, learning, inflammation, function of muscle.

The use of "IPA Bioprofiler" allowed to identify clusters of molecules causally linked to each of the identified biological activities and the specific molecular pathway underpinning them. Information concerning the measured modulation or modulation pattern of each of said parameters induced by each group was then superimposed on the networks obtained, to define influenced parameters and modulation of the connected biofunction.

Modulation of each of said parameters were shown in different intensities of blue (signifying down-modulation) or red (signifying up-modulation).

To determine the expected calculated impact of such experimentally observed modulations of each of said omic parameters on biological activities, the "Overlay" and "Molecule Activity Predictor" tool (MAP) are employed. The "Prediction" function is activated within MAP tool to calculate the resulting expected modulation of biological activities. Colour coding is thus established:
- orange: increase in activity
- blue: decrease in activity
- white: not achievable/not predictable

As the "Overlay analysis" does not directly calculate the Z-score for each biological activity but provides results in terms of colours indicating the direction of the modification and intensity of the colour signal proportional to the magnitude of the modulation of interest, it is necessary to translate the intensity of the modulation signal (graphically represented in the "my pathway" tab) into a numerical value. This is achieved by converting the colour intensity obtained for each biological activity into a Z-score.

One of the possible tools that can be used for this purpose is "IPAmap_Parser" app, a web port app of Pipeline Pilot that aims to assign a score, called z-score, to genes and biofunctions based on their coloration within a biological pathway generated by QIAGEN's Ingenuity Pathway Analysis software. The key step of the algorithm is the conversion from the RGB colour model to the LAB model (https://www.xrite.com/it-it/blog/lab-color-space posted on October 2018 by Tim Mouw), a colorimetric coding that also allows the intensity of the colour to be recorded and not just the RGB composition. This conversion occurs within a Pipeline Pilot "component" that uses a procedure written using R software that is based on specific features of the colorspace package (https://cran.r-project.org/web/packages/colorspace/index.html, details can be found on Zeileis et al 2020journal of statistical software, doi:10.18637/jss.v096.i01). Such a model displays the functional connections within and among the different datasets and enables to highlight coherence, on a systemic scale, with the achievement of a clinically improved cognitive and physical performance.

The activation of these activities in response to Product B (V2 vs V1), is summarized in figures 23a and 23b. Product B is effective in modulating activities correlated to cognitive state and is therefore confirmed in a clinical setup as exerting its beneficial action through a physiological mechanism of action. These observations are confirmed by the results of a clustering analysis performed on the patterns of activation/repression of activities in response to different treatment and in relationship with the desirable profile for improvement of cognitive and physical parameters (desiderata). In particular it was possible to identify parameters of neuronal function, metabolic health, systemic inflammation, oxidative stress and microbiome composition responsive to Product B treatment.

The effectiveness of the treatment was confirmed by the clinical trials with above mentioned standard clinical tests and endpoints related thereto.

The results of the clinical validation of the therapeutic/beneficial effect through a physiological mechanism of action are summarised in figure 23. The figure shows that in patients treated with product B (V2 vs V1) the conditions set forth in the clinical validation method of the invention are fulfilled. In fact, at least 50% of the biological activities for each hallmark retrieved for the *in vitro* cell based assay according to the invention, are modulated in V2 with the modulation trend of the network concurring to the healthy state previously determined, and each of the calculated modulation values of V2 of said at least 50% biological activities differ from the ones of V1 of at least 0.15 (V1 being, in the example provided, the baseline with modulation value of 0).

## Claims

1. A method for measuring different batches of a therapeutic product comprising one or more natural matrices, to determine that said product exerts its therapeutic effect through a physiological mechanism of action, wherein said physiological mechanism of action is a non-pharmacological, -immunological, -metabolic (Ph.IM) mode of action, and wherein said physiological mechanism is **characterized by** the regulation of a network of biological activities that underly hallmarks defining a pathological state, and by the therapeutic functional resilience of said product among said different batches the method comprising:
a) selecting a therapeutic product comprising one or more natural matrices and providing different batches of said product;
b) providing a list of hallmarks representative of said pathological state;
c) identifying for each of said hallmarks one or more biological activities modifications underlying said pathological state thereby pinpointing a network of biological activities whose modulation concurs to said pathological state;
d) identifying one or more gene expression patterns whose modulation concurs to the modulation of said one or more biological activities underlying the therapeutic effect of the product tested and determining the modulation trend in terms of up or down modulation of said one or more biological activities, in said network, concurring to said pathological state or to a healthy state
e) performing, on each of said different batches, at least one *in vitro* cell-based assay wherein the read out of said cell-based assay is representative of the modulation of one or more biological activities underlying the desired therapeutic effect, on the following groups of cells
(e1) at least one control group and at least two test groups of cells having the diseased phenotype relevant to the intended use of the therapeutic product; or
(e2) at least one group of cells with a healthy physiological phenotype; and at least one control group and at least two test groups of said cells with a healthy physiological phenotype wherein the diseased phenotype relevant to the intended use of the therapeutic product is induced,
and treating each of said test groups of cells with one of said different batches of therapeutic product;
(f) determining the modulation or modulation pattern of each of said gene expression patterns on each of said groups of cells of step (e) and calculating the respective modulation values for each of said one or more biological activities;
(g) said therapeutic product is shown to exert its therapeutic effect through said physiological mechanism when, comparing said modulation values, the conditions (g1) and (g2) are met:
(g1) at least 50% of said one or more biological activities for each hallmark are modulated by each product batch with the modulation trend of the network concurring to the healthy state, and the modulation values determined in (f) of each of said at least 50% one or more biological activities for said test groups of cells of (e1) differ, respectively, from the ones of said control group of cells of (e1) of at least 0.15, or
at least 50% of said one or more biological activities for each hallmark are modulated by each product batch with the modulation trend of the network concurring to the healthy state, and the modulation values determined in (f) of each of said at least 50% one or more biological activities for said test groups of cells of (e2) differ, respectively, from the ones of said control group of cells of (e2) by at least 15%, and
(g2) functional resilience of the product is demonstrated by the modulation values for each one or more biological activities of each said test groups of cells differing by less than 20% from the average of said values;
wherein gene expression is assessed by transcriptomic analysis.

2. A method for measuring different batches of a beneficial product adjuvating homeostasis in an altered physiological state the product comprising one or more natural matrices, to determine that said product exerts its beneficial effect through a physiological mechanism of action wherein said physiological mechanism of action is a non-pharmacological, -immunological, -metabolic (Ph.IM) mode of action, and is **characterized by** the regulation of a network of biological activities that underly hallmarks defining a pathological state, and by the beneficial functional resilience of said product among said different batches the method comprising:
a) selecting a beneficial product comprising one or more natural matrices and providing different batches of said product;
b) providing a list of hallmarks representative of a pathological state that can stem from said altered physiological state;
c) identifying for each of said hallmarks one or more biological activities modifications underlying said pathological state thereby pinpointing a network of biological activities whose modulation concurs to said pathological state;
d) identifying one or more gene expression patterns whose modulation concurs to the modulation of said one or more biological activities underlying the beneficial effect of the product tested and determining the modulation trend in terms of up or down modulation of said one or more biological activities, in said network, concurring to said pathological state or to a healthy state
e) performing, on each of said different batches, at least one *in vitro* cell-based assay wherein the read out of said cell-based assay is representative of the modulation of one or more biological activities underlying the desired beneficial effect, on the following groups of cells:
at least one control group of cells having healthy phenotype or at least one control group of cells wherein the aberrantly modulated phenotype targeted by said beneficial product is suitably induced, and at least two test groups of cells taken from said control group
and treating, each of said test groups of cells, with one of said different batches of beneficial product;
(f) determining the modulation or modulation pattern of each of said gene expression patterns on each of said groups of cells of step (e) and calculating the respective modulation values for each of said one or more biological activities;
(g) said therapeutic product is shown to exert its beneficial effect through said physiological mechanism when comparing said modulation values the conditions (g1) and (g2) are met:
g1) at least 50% of said one or more biological activities for each hallmark are modulated by each product batch with the modulation trend of the network concurring to the healthy state and the modulation values determined in (f) of each of said at least 50% one or more biological activities for said test groups of cells differ, respectively, from the ones of said control group of cells of at least 0.15 and
g2) functional resilience of the product is demonstrated by the modulation values for each one or more biological activities of each said test groups of cells differing by less than 20% from the average of said values;
wherein gene expression is assessed by transcriptomic analysis.

3. The method according to any one of claims 1 or 2, wherein said therapeutic or beneficial product comprises or consists of one or more of: cut or pulverized plant parts, plant extracts, fractions of said extracts, microorganisms, honey, propolis, silk, wax, plant resins, plant gums, plant exudates, vegetable oils, vegetable essential oils, animal tissues lysates, or plant or animal fluids.

4. The method of any one of claims 1-3, wherein said product is a medical device as defined in EU Regulation 2017/745 Article 2 (1) indents 1-3; or a medicament or a food supplement.

5. The method of any one of claims 1-4, wherein said cell-based assay is designed to simulate conditions related to said pathological condition or to simulate conditions related to said altered physiological state.

6. The method of any one of claims 1-5, further comprising analysing said one or more batches of therapeutic or beneficial product for the presence of supramolecular structures, and/or for the presence of miRNAs, and/or in its isotopic abundance
wherein, when isotopic abundance is analysed, the isotopic abundance analysed is that of one or more of C, H, O, N atoms in said product and in the raw materials from which the one or more natural matrices contained therein are prepared.

7. The method of any one of claims 1-6, further comprising testing said therapeutic or beneficial product for ready biodegradability using an OECD 310: 2014 biodegradability test.

8. A method for validating in a clinical set-up the physiological mechanism of action of a product for treating a pathological state or adjuvating homeostasis in an altered physiological state previously determined with the method according to anyone of claims 1 to 7, the method comprising:
1) subjecting to one or more biological assays, at least two groups (i) of biological samples from patients treated with a therapeutic or beneficial product exerting its therapeutic or beneficial effect through a physiological mechanism of action as assessed with the method according to claims 1 to 7, said groups of samples having been collected at different time moments V1 and Vn, n being an integer number >1, and at least two groups (ii) of biological samples from patients treated with a therapeutic or beneficial comparator reference product and/or at least two groups (iii) of biological samples from patients treated with a placebo, said groups of samples having been collected at the same time moments of group (i), wherein the read-out of said one or more biological assays is representative of the modulation of one or more biological activities underlying the desired therapeutic or beneficial effect, and determining from said read out:
whether said product exert its therapeutic or beneficial action by regulating a network of said biological activities that underly said pathological state or altered physiological state related to said condition, and wherein,
said product is clinically confirmed to exert its therapeutic or beneficial action through a physiological mechanism of action when said product is shown to regulate a network of said biological activities that underly said pathophysiological or altered physiological state.

9. The method of claim 8 further comprising: (1) providing a list of hallmarks representative of said pathological state or of the pathological state that can stem from said altered physiological state; (2) identifying for each of said hallmarks one or more biological activities modifications underlying said pathological state thereby pinpointing a network of biological activities whose modulation concurs to said pathological state and (3) identifying one or more parameters whose modulation concurs to the modulation of said one or more biological activities underlying the therapeutic effect of the product tested and determining the modulation trend in terms of up or down modulation of said one or more biological activities, in said network, concurring to said pathological state or to a healthy state.

10. The method according to claim 9 wherein said hallmarks and biological activities correspond to the hallmarks and biological activities selected for the same product in the method according to of claims 1 or 2.

11. The method according to claim 10, comprising:
(a) performing at least one biological assays on each of said biological samples
(b) determining the modulation or modulation pattern of each of said parameters and calculating the respective modulation values for each of said one or more biological activities for each of said samples;
(c) comparing said modulation values for each of said one or more biological activities thereby providing an average modulation value for each of said activities for each of said groups of samples, or thereby providing the modulation value for each of said activities for each patient in each group of samples wherein:
the product is shown to exert its therapeutic or beneficial effect through a physiological mechanism of action when
at least 50% of said one or more average biological activities or patient biological activities for each hallmark are modulated in V2 with the modulation trend of the network concurring to the healthy state, and each of said modulation values of V2 determined in (b) of said at least 50% one or more average biological activities or patient biological activities differ, respectively, from the ones of V1 of at least 0.15.

## Patentansprüche

1. Verfahren zum Messen verschiedener Chargen eines therapeutischen Produktes, umfassend eine oder mehrere natürliche Matrizen, um zu bestimmen, dass das Produkt seine therapeutische Wirkung durch einen physiologischen Wirkmechanismus ausübt, wobei der physiologische Wirkmechanismus eine nicht-pharmakologische, -immunologische, -metabolische (Ph.IM) Wirkungsweise ist und wobei der physiologische Mechanismus **gekennzeichnet ist durch** die Regulierung eines Netzwerks biologischer Aktivitäten, welche Kennzeichen zugrunde liegen, die einen pathologischen Zustand definieren, und **durch** die therapeutische funktionelle Resilienz des Produktes zwischen den verschiedenen Chargen, wobei das Verfahren umfasst:
a) Auswählen eines therapeutischen Produktes, umfassend eine oder mehrere natürliche Matrizen, und Bereitstellen verschiedener Chargen des Produktes;
b) Bereitstellen einer Liste von Kennzeichen, welche für den pathologischen Zustand repräsentativ sind;
c) Identifizieren einer oder mehrerer Modifikationen biologischer Aktivitäten, die dem pathologischen Zustand zugrunde liegen, für jedes der Kennzeichen, wodurch ein Netzwerk biologischer Aktivitäten ermittelt wird, dessen Modulation zu dem pathologischen Zustand beiträgt;
d) Identifizieren eines oder mehrerer Genexpressionsmuster, deren Modulation zur Modulation der einen oder der mehreren biologischen Aktivitäten beiträgt, die der therapeutischen Wirkung des getesteten Produkts zugrunde liegen, und Bestimmen der Modulationsrichtung in Bezug auf eine Modulation nach oben oder nach unten der einen oder der mehreren biologischen Aktivitäten in dem Netzwerk, die zu dem pathologischen Zustand oder zu einem gesunden Zustand beitragen
e) Durchführen mindestens eines zellbasierten *In-vitro*-Assays an jeder der verschiedenen Chargen, wobei das Ausleseergebnis des zellbasierten Assays für die Modulation einer oder mehrerer biologischer Aktivitäten repräsentativ ist, die der gewünschten therapeutischen Wirkung zugrunde liegen, an den folgenden Gruppen von Zellen
(e1) mindestens einer Kontrollgruppe und mindestens zwei Testgruppen von Zellen mit dem Krankheitsphänotyp, der für die beabsichtigte Verwendung des therapeutischen Produkts relevant ist; oder
(e2) mindestens einer Gruppe von Zellen mit einem gesunden physiologischen Phänotyp; und mindestens einer Kontrollgruppe und mindestens zwei Testgruppen der Zellen mit einem gesunden physiologischen Phänotyp, wobei der Krankheitsphänotyp induziert wird, der für die beabsichtigte Verwendung des therapeutischen Produktes relevant ist,
und Behandeln jeder der Testgruppen von Zellen mit einer der verschiedenen Chargen des therapeutischen Produktes;
(f) Bestimmen der Modulation oder des Modulationsmusters jedes der Genexpressionsmuster in jeder der genannten Gruppen von Zellen von Schritt (e) und Berechnen der jeweiligen Modulationswerte für jede der einen oder der mehreren biologischen Aktivitäten;
(g) es wird gezeigt, dass das therapeutische Produkt seine therapeutische Wirkung durch den physiologischen Mechanismus ausübt, wenn beim Vergleich der Modulationswerte die Bedingungen (g1) und (g2) erfüllt sind:
(g1) durch jede Produktcharge werden mindestens 50 % der einen oder der mehreren biologischen Aktivitäten für ein jeweiliges Kennzeichen moduliert, wobei die Modulationsrichtung des Netzwerks zu dem gesunden Zustand beiträgt, und die in (f) bestimmten Modulationswerte jeder der mindestens 50 % der einen oder der mehreren biologischen Aktivitäten für die Testgruppen von Zellen aus (e1) unterscheiden sich jeweils um mindestens 0,15 von denjenigen der Kontrollgruppe von Zellen aus (e1), oder
durch jede Produktcharge werden mindestens 50 % der einen oder der mehreren biologischen Aktivitäten für ein jeweiliges Kennzeichen moduliert, wobei die Modulationsrichtung des Netzwerks zu dem gesunden Zustand beiträgt, und die in (f) bestimmten Modulationswerte jeder der mindestens 50 % der einen oder der mehreren biologischen Aktivitäten für die Testgruppen von Zellen aus (e2) unterscheiden sich jeweils um mindestens 15 % von denjenigen der Kontrollgruppe von Zellen aus (e2), und
(g2) die funktionelle Resilienz des Produktes wird dadurch nachgewiesen, dass die Modulationswerte für jede der einen oder der mehreren biologischen Aktivitäten jeder der Testgruppen von Zellen um weniger als 20 % vom Mittelwert der Werte abweichen;
wobei die Genexpression durch transkriptomische Analyse bewertet wird.

2. Verfahren zum Messen verschiedener Chargen eines vorteilhaften Produktes, welches Homöostase bei einem veränderten physiologischen Zustand unterstützt, wobei das Produkt eine oder mehrere natürliche Matrizen umfasst, um zu bestimmen, dass das Produkt seine vorteilhafte Wirkung durch einen physiologischen Wirkmechanismus ausübt, wobei der physiologische Wirkmechanismus eine nicht-pharmakologische, -immunologische, -metabolische (Ph.IM) Wirkungsweise ist und **gekennzeichnet ist durch** die Regulierung eines Netzwerks biologischer Aktivitäten, welche Kennzeichen zugrunde liegen, die einen pathologischen Zustand definieren, und **durch** die vorteilhafte funktionelle Resilienz des Produktes zwischen den verschiedenen Chargen, wobei das Verfahren umfasst:
a) Auswählen eines vorteilhaften Produktes, umfassend eine oder mehrere natürliche Matrizen, und Bereitstellen verschiedener Chargen des Produktes;
b) Bereitstellen einer Liste von Kennzeichen, welche für einen pathologischen Zustand repräsentativ sind, der aus dem veränderten physiologischen Zustand resultieren kann;
c) Identifizieren einer oder mehrerer Modifikationen biologischer Aktivitäten, die dem pathologischen Zustand zugrunde liegen, für jedes der Kennzeichen, wodurch ein Netzwerk biologischer Aktivitäten ermittelt wird, dessen Modulation zu dem pathologischen Zustand beiträgt;
d) Identifizieren eines oder mehrerer Genexpressionsmuster, deren Modulation zur Modulation der einen oder der mehreren biologischen Aktivitäten beiträgt, die der vorteilhaften Wirkung des getesteten Produkts zugrunde liegen, und Bestimmen der Modulationsrichtung in Bezug auf eine Modulation nach oben oder nach unten der einen oder der mehreren biologischen Aktivitäten in dem Netzwerk, die zu dem pathologischen Zustand oder zu einem gesunden Zustand beitragen
e) Durchführen mindestens eines zellbasierten *In-vitro*-Assays an jeder der verschiedenen Chargen, wobei das Ausleseergebnis des zellbasierten Assays für die Modulation einer oder mehrerer biologischer Aktivitäten repräsentativ ist, die der gewünschten vorteilhaften Wirkung zugrunde liegen, an den folgenden Gruppen von Zellen:
mindestens einer Kontrollgruppe von Zellen mit gesundem Phänotyp oder mindestens einer Kontrollgruppe von Zellen, wobei der aberrant modulierte Phänotyp, auf den das vorteilhafte Produkt gerichtet ist, in geeigneter Weise induziert wird, und mindestens zwei Testgruppen von Zellen, die aus der Kontrollgruppe entnommen sind,
und Behandeln jeder der Testgruppen von Zellen mit einer der verschiedenen Chargen des vorteilhaften Produktes;
(f) Bestimmen der Modulation oder des Modulationsmusters jedes der Genexpressionsmuster in jeder der genannten Gruppen von Zellen von Schritt (e) und Berechnen der jeweiligen Modulationswerte für jede der einen oder der mehreren biologischen Aktivitäten;
(g) es wird gezeigt, dass das therapeutische Produkt seine vorteilhafte Wirkung durch den physiologischen Mechanismus ausübt, wenn beim Vergleich der Modulationswerte die Bedingungen (g1) und (g2) erfüllt sind:
g1) durch jede Produktcharge werden mindestens 50 % der einen oder der mehreren biologischen Aktivitäten für ein jeweiliges Kennzeichen moduliert, wobei die Modulationsrichtung des Netzwerks zu dem gesunden Zustand beiträgt, und die in (f) bestimmten Modulationswerte jeder der mindestens 50 % der einen oder der mehreren biologischen Aktivitäten für die Testgruppen von Zellen unterscheiden sich jeweils um mindestens 0,15 von denjenigen der Kontrollgruppe von Zellen, und
g2) die funktionelle Resilienz des Produktes wird dadurch nachgewiesen, dass die Modulationswerte für jede der einen oder der mehreren biologischen Aktivitäten jeder der Testgruppen von Zellen um weniger als 20 % vom Mittelwert der Werte abweichen;
wobei die Genexpression durch transkriptomische Analyse bewertet wird.

3. Verfahren nach einem der Ansprüche 1 oder 2, wobei das therapeutische oder vorteilhafte Produkt eines oder mehrere aus geschnittenen oder pulverisierten Pflanzenteilen, Pflanzenextrakten, Fraktionen der Extrakte, Mikroorganismen, Honig, Propolis, Seide, Wachs, Pflanzenharzen, Pflanzengummis, Pflanzenexsudaten, pflanzlichen Ölen, pflanzlichen ätherischen Ölen, tierischen Gewebelysaten oder pflanzlichen oder tierischen Fluiden umfasst oder daraus besteht.

4. Verfahren nach einem der Ansprüche 1 bis 3, wobei das Produkt ein Medizinprodukt, wie in der EU-Verordnung 2017/745 Artikel 2 (1) Spiegelstriche 1 bis 3 definiert, oder ein Arzneimittel oder ein Nahrungsergänzungsmittel ist.

5. Verfahren nach einem der Ansprüche 1 bis 4, wobei der zellbasierte Assay dazu ausgelegt ist, Bedingungen zu simulieren, die sich auf den pathologischen Zustand oder den veränderten physiologischen Zustand beziehen.

6. Verfahren nach einem der Ansprüche 1 bis 5, ferner umfassend Analysieren der einen oder der mehreren Chargen des therapeutischen oder vorteilhaften Produktes auf das Vorhandensein von supramolekularen Strukturen und/oder auf das Vorhandensein von miRNAs und/oder in seiner Isotopenhäufigkeit,
wobei, wenn die Isotopenhäufigkeit analysiert wird, die analysierte Isotopenhäufigkeit diejenige von einem oder mehreren der Atome C, H, O, N in dem Produkt und in den Rohmaterialien ist, aus denen die eine oder mehreren darin enthaltenen natürlichen Matrizen hergestellt werden.

7. Verfahren nach einem der Ansprüche 1 bis 6, ferner umfassend Testen des therapeutischen oder vorteilhaften Produktes auf leichte biologische Abbaubarkeit unter Verwendung eines OECD 310: 2014-Tests der biologischen Abbaubarkeit.

8. Verfahren zum Validieren des physiologischen Wirkmechanismus eines Produktes zum Behandeln eines pathologischen Zustands oder zum Unterstützen von Homöostase bei einem veränderten physiologischen Zustand, der zuvor mit dem Verfahren nach einem der Ansprüche 1 bis 7 bestimmt wurde, in einem klinischen Umfeld, wobei das Verfahren umfasst:
1) Unterziehen mindestens zweier Gruppen (i) von biologischen Proben von Patienten, die mit einem therapeutischen oder vorteilhaften Produkt behandelt wurden, das seine therapeutische oder vorteilhafte Wirkung durch einen physiologischen Wirkmechanismus ausübt, bewertet mit dem Verfahren nach den Ansprüchen 1 bis 7, wobei die Gruppen von Proben zu unterschiedlichen Zeitpunkten V1 und Vn entnommen wurden, wobei n eine ganze Zahl >1 ist, und mindestens zweier Gruppen (ii) von biologischen Proben von Patienten, die mit einem therapeutischen oder vorteilhaften Komparator-Referenzprodukt behandelt wurden, und/oder mindestens zweier Gruppen (iii) von biologischen Proben von Patienten, die mit einem Placebo behandelt wurden, wobei die Gruppen von Proben zu denselben Zeitpunkten wie Gruppe (i) entnommen wurden, einem oder mehreren biologischen Assays, wobei das Ausleseergebnis des einen oder der mehreren biologischen Assays für die Modulation einer oder mehrerer biologischer Aktivitäten repräsentativ ist, die der gewünschten therapeutischen oder vorteilhaften Wirkung zugrunde liegen, und Bestimmen aus dem Ausleseergebnis:
ob das Produkt seine therapeutische oder vorteilhafte Wirkung durch Regulieren eines Netzwerks der biologischen Aktivitäten ausübt, die dem pathologischen Zustand oder dem veränderten physiologischen Zustand zugrunde liegen, der mit dem Leiden verbunden ist, und wobei
klinisch bestätigt wird, dass das Produkt seine therapeutische oder vorteilhafte Wirkung durch einen physiologischen Wirkmechanismus ausübt, wenn gezeigt wird, dass das Produkt ein Netzwerk der biologischen Aktivitäten reguliert, die dem pathophysiologischen oder dem veränderten physiologischen Zustand zugrunde liegen.

9. Verfahren nach Anspruch 8, ferner umfassend: (1) Bereitstellen einer Liste von Kennzeichen, die für den pathologischen Zustand oder für den pathologischen Zustand, der aus dem veränderten physiologischen Zustand resultieren kann, repräsentativ sind; (2) Identifizieren einer oder mehrerer Modifikationen biologischer Aktivitäten, die dem pathologischen Zustand zugrunde liegen, für jedes der Kennzeichen, wodurch ein Netzwerk biologischer Aktivitäten ermittelt wird, dessen Modulation zu dem pathologischen Zustand beiträgt, und (3) Identifizieren eines oder mehrerer Parameter, deren Modulation zu der Modulation der einen oder der mehreren biologischen Aktivitäten, die der therapeutischen Wirkung des getesteten Produkts zugrunde liegen, beiträgt, und Bestimmen der Modulationsrichtung in Bezug auf eine Modulation nach oben oder nach unten der einen oder der mehreren biologischen Aktivitäten in dem Netzwerk, die zu dem pathologischen Zustand oder zu einem gesunden Zustand beitragen.

10. Verfahren nach Anspruch 9, wobei die Kennzeichen und biologischen Aktivitäten den Kennzeichen und biologischen Aktivitäten entsprechen, die für dasselbe Produkt in dem Verfahren nach Anspruch 1 oder 2 ausgewählt werden.

11. Verfahren nach Anspruch 10, umfassend:
(a) Durchführen mindestens eines biologischen Assays an jeder der biologischen Proben
(b) Bestimmen der Modulation oder des Modulationsmusters jedes der Parameter und Berechnen der jeweiligen Modulationswerte für jede der einen oder der mehreren biologischen Aktivitäten für jede der Proben;
(c) Vergleichen der Modulationswerte für jede der einen oder der mehreren biologischen Aktivitäten, wodurch ein mittlerer Modulationswert für jede der Aktivitäten für jede der Probengruppen bereitgestellt wird oder wodurch der Modulationswert für jede der Aktivitäten für jeden Patienten in jeder Probengruppe bereitgestellt wird, wobei:
gezeigt wird, dass das Produkt seine therapeutische oder vorteilhafte Wirkung durch einen physiologischen Wirkmechanismus ausübt, wenn
mindestens 50 % der einen oder der mehreren mittleren biologischen Aktivitäten oder biologischen Patientenaktivitäten für ein jeweiliges Kennzeichen in V2 moduliert sind, wobei die Modulationsrichtung des Netzwerks zu dem gesunden Zustand beiträgt, und sich jeder der in (b) bestimmten Modulationswerte von V2 der mindestens 50 % der einen oder mehreren mittleren biologischen Aktivitäten oder biologischen Patientenaktivitäten jeweils von denjenigen von V1 um mindestens 0,15 unterscheidet.

## Revendications

1. Procédé destiné à mesurer différents lots d'un produit thérapeutique comprenant une ou plusieurs matrices naturelles, pour déterminer que ledit produit exerce son effet thérapeutique par le biais d'un mécanisme d'action physiologique, dans lequel ledit mécanisme d'action physiologique est un mode d'action non pharmacologique, non immunologique, non métabolique (Ph.IM), et dans lequel ledit mécanisme physiologique est **caractérisé par** la régulation d'un réseau d'activités biologiques qui sous-tendent des signes distinctifs définissant un état pathologique, et par la résilience fonctionnelle thérapeutique dudit produit parmi lesdits différents lots le procédé comprenant :
a) la sélection d'un produit thérapeutique comprenant une ou plusieurs matrices naturelles et la fourniture de différents lots dudit produit ;
b) la fourniture d'une liste de signes distinctifs représentatifs dudit état pathologique ;
c) l'identification pour chacun desdits signes distinctifs d'une ou plusieurs modifications d'activités biologiques qui sous-tendent ledit état pathologique en mettant de ce fait en évidence un réseau d'activités biologiques dont une modulation concourt audit état pathologique ;
d) l'identification d'un ou plusieurs profils d'expression génique dont la modulation concourt à la modulation de ladite ou desdites activités biologiques qui sous-tendent l'effet thérapeutique du produit testé et la détermination de la tendance de modulation en termes de modulation à la hausse ou à la baisse de ladite ou desdites activités biologiques, dans ledit réseau, concourant audit état pathologique ou à un état de bonne santé
e) la mise en œuvre, sur chacun desdits différents lots, d'au moins un dosage à base de cellules *in vitro* dans lequel le résultat dudit dosage à base de cellules est représentatif de la modulation d'une ou plusieurs activités biologiques qui sous-tendent l'effet thérapeutique souhaité, sur les groupes de cellules suivants
(e1) au moins un groupe témoin et au moins deux groupes de test de cellules ayant le phénotype pathologique en rapport avec l'utilisation prévue du produit thérapeutique ; ou
(e2) au moins un groupe de cellules avec un phénotype physiologique sain ; et au moins un groupe témoin et au moins deux groupes de test desdites cellules avec un phénotype physiologique sain dans lequel le phénotype pathologique en rapport avec l'utilisation prévue du produit thérapeutique est induit,
et le traitement de chacun desdits groupes de test de cellules avec l'un desdits différents lots de produit thérapeutique ;
(f) la détermination de la modulation ou du profil de modulation de chacun desdits profils d'expression génique sur chacun desdits groupes de cellules de l'étape (e) et le calcul des valeurs de modulation respectives pour chacune de ladite ou desdites activités biologiques ;
(g) ledit produit thérapeutique est montré exercer son effet thérapeutique par le biais dudit mécanisme physiologique lorsque, en comparant lesdites valeurs de modulation, les conditions (g1) et (g2) sont respectées :
(g1) au moins 50 % de ladite ou desdites activités biologiques pour chaque signe distinctif sont modulées par chaque lot de produit avec la tendance de modulation du réseau concourant à l'état de bonne santé, et les valeurs de modulation déterminées en (f) de chacune desdites au moins 50 % d'une ou plusieurs activités biologiques pour lesdits groupes de test de cellules de (e1) diffèrent, respectivement, de celles provenant dudit groupe témoin de cellules de (e1) d'au moins 0,15, ou
au moins 50 % de ladite ou desdites activités biologiques pour chaque signe distinctif sont modulées par chaque lot de produit avec la tendance de modulation du réseau concourant à l'état de bonne santé, et les valeurs de modulation déterminées en (f) de chacune desdites au moins 50 % d'une ou plusieurs activités biologiques pour lesdits groupes de test de cellules de (e2) diffèrent, respectivement, de celles provenant dudit groupe témoin de cellules de (e2) d'au moins 15 %, et
(g2) la résilience fonctionnelle du produit est démontrée par les valeurs de modulation pour chacune de la ou des activités biologiques de chacun desdits groupes de test de cellules différant de moins de 20 % de la moyenne desdites valeurs ;
dans lequel l'expression génique est évaluée par analyse transcriptomique.

2. Procédé destiné à mesurer différents lots d'un produit bénéfique favorisant l'homéostasie dans un état physiologique altéré le produit comprenant une ou plusieurs matrices naturelles, pour déterminer que ledit produit exerce son effet bénéfique par le biais d'un mécanisme d'action physiologique dans lequel ledit mécanisme d'action physiologique est un mode d'action non pharmacologique, non immunologique, non métabolique (Ph.IM), et est **caractérisé par** la régulation d'un réseau d'activités biologiques qui sous-tendent des signes distinctifs définissant un état pathologique, et par la résilience fonctionnelle bénéfique dudit produit parmi lesdits différents lots le procédé comprenant :
a) la sélection d'un produit bénéfique comprenant une ou plusieurs matrices naturelles et la fourniture de différents lots dudit produit ;
b) la fourniture d'une liste de signes distinctifs représentatifs d'un état pathologique qui peut résulter dudit état physiologique altéré ;
c) l'identification pour chacun desdits signes distinctifs d'une ou plusieurs modifications d'activités biologiques qui sous-tendent ledit état pathologique en mettant de ce fait en évidence un réseau d'activités biologiques dont une modulation concourt audit état pathologique ;
d) l'identification d'un ou plusieurs profils d'expression génique dont la modulation concourt à la modulation de ladite ou desdites activités biologiques qui sous-tendent l'effet bénéfique du produit testé et la détermination de la tendance de modulation en termes de modulation à la hausse ou à la baisse de ladite ou desdites activités biologiques, dans ledit réseau, concourant audit état pathologique ou à un état de bonne santé
e) la mise en œuvre, sur chacun desdits différents lots, d'au moins un dosage à base de cellules *in vitro* dans lequel le résultat dudit dosage à base de cellules est représentatif de la modulation d'une ou plusieurs activités biologiques qui sous-tendent l'effet bénéfique souhaité, sur les groupes de cellules suivants :
au moins un groupe témoin de cellules ayant un phénotype sain ou au moins un groupe témoin de cellules dans lequel le phénotype modulé de manière anormale, ciblé par ledit produit bénéfique est induit de manière appropriée, et au moins deux groupes de test de cellules prélevées auprès dudit groupe témoin
et le traitement de chacun desdits groupes de test de cellules avec l'un desdits différents lots de produit bénéfique ;
(f) la détermination de la modulation ou du profil de modulation de chacun desdits profils d'expression génique sur chacun desdits groupes de cellules de l'étape (e) et le calcul des valeurs de modulation respectives pour chacune de ladite ou desdites activités biologiques ;
(g) ledit produit thérapeutique est montré exercer son effet bénéfique par le biais dudit mécanisme physiologique lorsque, en comparant lesdites valeurs de modulation, les conditions (g1) et (g2) sont respectées :
g1) au moins 50 % de ladite ou desdites activités biologiques pour chaque signe distinctif sont modulées par chaque lot de produit avec la tendance de modulation du réseau concourant à l'état de bonne santé, et les valeurs de modulation déterminées en (f) de chacune desdites au moins 50 % d'une ou plusieurs activités biologiques pour lesdits groupes de test de cellules diffèrent, respectivement, de celles provenant dudit groupe témoin de cellules d'au moins 0,15 et
g2) la résilience fonctionnelle du produit est démontrée par les valeurs de modulation pour chacune de la ou des activités biologiques de chacun desdits groupes de test de cellules différant de moins de 20 % de la moyenne desdites valeurs ;
dans lequel l'expression génique est évaluée par analyse transcriptomique.

3. Procédé selon l'une quelconque des revendications 1 ou 2, dans lequel ledit produit thérapeutique ou bénéfique comprend ou est constitué d'un ou plusieurs parmi parties de plantes coupées ou pulvérisées, extraits végétaux, fractions desdits extraits, micro-organismes, miel, propolis, soie, cire, résines végétales, gommes végétales, exsudats végétaux, huiles végétales, huiles essentielles végétales, lysats de tissus animaux ou fluides végétaux ou animaux.

4. Procédé selon l'une quelconque des revendications 1 à 3, dans lequel ledit produit est un dispositif médical tel que défini à l'article 2 (1), tirets 1 à 3, du règlement UE 2017/745 ; ou un médicament ou un complément alimentaire.

5. Procédé selon l'une quelconque des revendications 1 à 4, dans lequel ledit dosage à base de cellules est conçu pour simuler des conditions se rapportant audit état pathologique ou pour simuler des conditions se rapportant audit état physiologique altéré.

6. Procédé selon l'une quelconque des revendications 1 à 5, comprenant en outre l'analyse dudit ou desdits lots de produit thérapeutique ou bénéfique pour déceler la présence de structures supramoléculaires, et/ou pour déceler la présence de miARN, et/ou dans son abondance isotopique
dans lequel, lorsque l'abondance isotopique est analysée, l'abondance isotopique analysée est celle d'un ou plusieurs des atomes de C, H, O, N dans ledit produit et dans les matières premières à partir desquelles la ou les matrices naturelles contenues dans celles-ci sont préparées.

7. Procédé selon l'une quelconque des revendications 1 à 6, comprenant en outre le fait de tester ledit produit thérapeutique ou bénéfique pour une biodégradabilité immédiate à l'aide d'un test de biodégradabilité OCDE 310 : 2014.

8. Procédé permettant de valider dans une configuration clinique le mécanisme d'action physiologique d'un produit permettant de traiter un état pathologique ou de favoriser l'homéostasie dans un état physiologique altéré précédemment déterminé avec le procédé selon l'une quelconque des revendications 1 à 7, le procédé comprenant :
1) le fait de soumettre à un ou plusieurs dosages biologiques, au moins deux groupes (i) d'échantillons biologiques provenant de patients traités avec un produit thérapeutique ou bénéfique exerçant son effet thérapeutique ou bénéfique par le biais d'un mécanisme d'action physiologique tel qu'évalué avec le procédé selon les revendications 1 à 7, lesdits groupes d'échantillons ayant été collectés à différents moments dans le temps V1 et Vn, n étant un nombre entier >1, et au moins deux groupes (ii) d'échantillons biologiques provenant de patients traités avec un produit de référence thérapeutique ou bénéfique servant de comparateur et/ou au moins deux groupes (iii) d'échantillons biologiques provenant de patients traités avec un placebo, lesdits groupes d'échantillons ayant été collectés aux mêmes moments dans le temps que le groupe (i), dans lequel le résultat dudit ou desdits dosages biologiques est représentatif de la modulation d'une ou plusieurs activités biologiques qui sous-tendent l'effet thérapeutique ou bénéfique souhaité, et le fait de déterminer à partir dudit résultat :
si ledit produit exerce son action thérapeutique ou bénéfique en régulant un réseau desdites activités biologiques qui sous-tendent ledit état pathologique ou ledit état physiologique altéré se rapportant à ladite condition, et dans lequel,
ledit produit est cliniquement confirmé pour exercer son action thérapeutique ou bénéfique par le biais d'un mécanisme d'action physiologique lorsque ledit produit est montré réguler un réseau desdites activités biologiques qui sous-tendent ledit état physiopathologique ou état physiologique altéré.

9. Procédé selon la revendication 8, comprenant en outre : (1) la fourniture d'une liste de signes distinctifs représentatifs dudit état pathologique ou de l'état pathologique qui peut résulter dudit état physiologique altéré ; (2) l'identification pour chacun desdits signes distinctifs d'une ou plusieurs modifications d'activités biologiques qui sous-tendent ledit état pathologique en mettant de ce fait en évidence un réseau d'activités biologiques dont la modulation concourt audit état pathologique et (3) l'identification d'un ou plusieurs paramètres dont la modulation concourt à la modulation de ladite ou desdites activités biologiques qui sous-tendent l'effet thérapeutique du produit testé et la détermination de la tendance de modulation en termes de modulation à la hausse ou à la baisse de ladite ou desdites activités biologiques, dans ledit réseau, concourant audit état pathologique ou à un état de bonne santé.

10. Procédé selon la revendication 9 dans lequel lesdits signes distinctifs et lesdites activités biologiques correspondent aux signes distinctifs et aux activités biologiques sélectionnés pour le même produit dans le procédé selon les revendications 1 ou 2.

11. Procédé selon la revendication 10, comprenant :
(a) la mise en œuvre d'au moins un dosage biologique sur chacun desdits échantillons biologiques
(b) la détermination de la modulation ou du profil de modulation de chacun desdits paramètres et le calcul des valeurs de modulation respectives pour chacune de ladite ou desdites activités biologiques pour chacun desdits échantillons ;
(c) la comparaison desdites valeurs de modulation pour chacune de ladite ou desdites activités biologiques en fournissant de ce fait une valeur moyenne de modulation pour chacune desdites activités pour chacun desdits groupes d'échantillons, ou en fournissant de ce fait la valeur de modulation pour chacune desdites activités pour chaque patient dans chaque groupe d'échantillons dans lequel :
le produit est montré exercer son effet thérapeutique ou bénéfique par le biais d'un mécanisme d'action physiologique lorsque
au moins 50 % de ladite ou desdites activités biologiques moyennes ou activités biologiques de patient pour chaque signe distinctif sont modulées en V2 avec la tendance de modulation du réseau concourant à l'état de bonne santé, et chacune desdites valeurs de modulation de V2 déterminées en (b) desdites au moins 50 % d'une ou plusieurs activités biologiques moyennes ou activités biologiques de patient diffère, respectivement, de celles de V1 d'au moins 0,15.
